# EUROPEAN PATENT APPLICATION

(11) **EP 1 236 739 A2**
(43) Date of publication of application: **04.09.2002**
(21) Application number: 02250691.9
(22) Date of filing: 01.02.2002
(51) Int. Cl.: C07K 14/47, C12N 15/12, C12N 15/63, C07K 16/18, G01N 33/50, G01N 33/53, C12Q 1/68

(54) **59914 and 59921 Choline transporters and uses therefore**

(30) Priority: 01.02.2001 US 267076 P
(71) Applicant: Millennium Pharmaceuticals, Inc., Cambridge, Massachusetts 02139 (US)
(72) Inventor: Curtis, Rory A. J., Massachusetts 01772 (US)
(74) Representative: Lunt, Mark George Francis

(57) **Abstract**

The invention provides isolated nucleic acids molecules, designated 59914 and 59921 nucleic acid molecules, which encode choline transporter family members. The invention also provides antisense nucleic acid molecules, recombinant expression vectors containing 59914 and 59921 nucleic acid molecules, host cells into which the expression vectors have been introduced, and nonhuman transgenic animals in which 59914 and 59921 genes have been introduced or disrupted. The invention still further provides isolated 59914 and 59921 proteins, fusion proteins, antigenic peptides and anti-59914 and 59921 antibodies. Diagnostic and therapeutic methods utilizing compositions of the invention are also provided.

SEQUENCE LISTING

<110> Millennium Pharmaceuticals, Inc

<120> 59914 and 59921, CHOLINE TRANSPORTERS
  AND USES THEREFOR

<130> P71893EP

<150> 60/267,076
<151> 2001-02-01

<160> 12

<170> FastSEQ for Windows Version 4.0

<210> 1
<211> 2473
<212> DNA
<213> Homo sapiens

<220>
<221> CDS
<222> (88)...(2238)

<221> 5'UTR
<222> (1)...(87)

<221> 3'UTR
<222> (2239)...(2473)

<400> 1
gaaatgatgg agtaagagac tcttttctaa gcaactcaag tttgcagtga ttcaggccta 60
cttctgaaga gacagccttt tatctca atg aat gac aca gaa aaa cca gca gat 114
                              Met Asn Asp Thr Glu Lys Pro Ala Asp
                               1               5

act ccc tct gag gaa gag gac ttt ggt gat cca agg aca tat gac cca    162
Thr Pro Ser Glu Glu Glu Asp Phe Gly Asp Pro Arg Thr Tyr Asp Pro
 10                  15                  20                  25

gat ttc aag ggg cct gtt gcc aac agg agt tgt aca gat gtt ctg tgc    210
Asp Phe Lys Gly Pro Val Ala Asn Arg Ser Cys Thr Asp Val Leu Cys
                 30                  35                  40

tgt atg atc ttc cta ctg tgt att att ggc tac att gtt tta gga ctt    258
Cys Met Ile Phe Leu Leu Cys Ile Ile Gly Tyr Ile Val Leu Gly Leu
             45                  50                  55

gtg gcc tgg gta cat ggg gac ccc aga aga gca gcc tat cct aca gac    306
Val Ala Trp Val His Gly Asp Pro Arg Arg Ala Ala Tyr Pro Thr Asp
         60                  65                  70

agc cag ggc cac ttt tgt ggc cag aag ggc act ccc aat gag aac aag    354
Ser Gln Gly His Phe Cys Gly Gln Lys Gly Thr Pro Asn Glu Asn Lys
     75                  80                  85

acc att ttg ttt tac ttt aac ctg tta cgc tgt acc agt ccc tcc gtg    402
Thr Ile Leu Phe Tyr Phe Asn Leu Leu Arg Cys Thr Ser Pro Ser Val
 90                  95                 100                 105

ttg cta aac cta cag tgc cct acc aca cag atc tgt gtc tcc aag tgc 450
Leu Leu Asn Leu Gln Cys Pro Thr Thr Gln Ile Cys Val Ser Lys Cys
                110                 115                 120

cca gaa aaa ttt tta acc tat gtg gaa atg caa ctt ttg tac aca aaa 498
Pro Glu Lys Phe Leu Thr Tyr Val Glu Met Gln Leu Leu Tyr Thr Lys
            125                 130                 135

gac aaa agc tac tgg gaa gac tac cgt cag ttc tgt aag acc act gct 546
Asp Lys Ser Tyr Trp Glu Asp Tyr Arg Gln Phe Cys Lys Thr Thr Ala
        140                 145                 150

aag cct gtg aag tct ctc aca cag ctt tta ctg gat gat gat tgt cca 594
Lys Pro Val Lys Ser Leu Thr Gln Leu Leu Leu Asp Asp Asp Cys Pro
    155                 160                 165

aca gcg att ttt ccc agc aaa cct ttt ctc cag aga tgt ttc cct gac 642
Thr Ala Ile Phe Pro Ser Lys Pro Phe Leu Gln Arg Cys Phe Pro Asp
170                 175                 180                 185

ttc tct acc aaa aat ggc act tta aca ata gga agt aag atg atg ttt 690
Phe Ser Thr Lys Asn Gly Thr Leu Thr Ile Gly Ser Lys Met Met Phe
                190                 195                 200

caa gat gga aat gga ggg aca aga agt gtt gta gaa ctc ggg att gct 738
Gln Asp Gly Asn Gly Gly Thr Arg Ser Val Val Glu Leu Gly Ile Ala
            205                 210                 215

gca aat ggt atc aat aaa ctt ctt gat gca aag tca ctt gga ttg aaa 786
Ala Asn Gly Ile Asn Lys Leu Leu Asp Ala Lys Ser Leu Gly Leu Lys
        220                 225                 230

gtg ttt gaa gac tat gca aga act tgg tat tgg att ctt att ggc ctg 834
Val Phe Glu Asp Tyr Ala Arg Thr Trp Tyr Trp Ile Leu Ile Gly Leu
    235                 240                 245

acg atc gcc atg gtc ctt agt tgg ata ttt ttg ata ctt ctg agg ttc 882
Thr Ile Ala Met Val Leu Ser Trp Ile Phe Leu Ile Leu Leu Arg Phe
250                 255                 260                 265

ata gct gga tgc ctc ttc tgg gtc ttc atg att ggt gtg att gga att 930
Ile Ala Gly Cys Leu Phe Trp Val Phe Met Ile Gly Val Ile Gly Ile
                270                 275                 280

ata ggt tat gga ata tgg cac tgt tac cag cag tac acc aat ctt cag 978
Ile Gly Tyr Gly Ile Trp His Cys Tyr Gln Gln Tyr Thr Asn Leu Gln
            285                 290                 295

gaa cgc cca agt tct gta tta act atc tat gac atc ggg att cag act 1026
Glu Arg Pro Ser Ser Val Leu Thr Ile Tyr Asp Ile Gly Ile Gln Thr
        300                 305                 310

aac ata agc atg tac ttt gaa ctg caa caa aca tgg ttc aca ttt atg 1074
Asn Ile Ser Met Tyr Phe Glu Leu Gln Gln Thr Trp Phe Thr Phe Met
    315                 320                 325

ata ata ctc tgc atc att gaa gtg att gtc atc ctc atg ctg atc ttc 1122
Ile Ile Leu Cys Ile Ile Glu Val Ile Val Ile Leu Met Leu Ile Phe
330                 335                 340                 345

ctc agg aat cga atc cga gtc gcc att atc ctg ctg aag gaa gga agc 1170

Leu Arg Asn Arg Ile Arg Val Ala Ile Ile Leu Leu Lys Glu Gly Ser
                350                 355                 360

aaa gcc att gga tat gtt cct agt aca tta gtc tat cca gct tta act     1218
Lys Ala Ile Gly Tyr Val Pro Ser Thr Leu Val Tyr Pro Ala Leu Thr
            365                 370                 375

ttc att ttg ctc tca atc tgc att tgc tac tgg gtc gtg aca gca gtt     1266
Phe Ile Leu Leu Ser Ile Cys Ile Cys Tyr Trp Val Val Thr Ala Val
        380                 385                 390

ttc ttg gcg aca tcg ggg gta cct gta tac aaa gtc ata gct cca ggg     1314
Phe Leu Ala Thr Ser Gly Val Pro Val Tyr Lys Val Ile Ala Pro Gly
    395                 400                 405

ggg cat tgt ata cat gaa aat caa acc tgt gac cca gag att ttt aat     1362
Gly His Cys Ile His Glu Asn Gln Thr Cys Asp Pro Glu Ile Phe Asn
410                 415                 420                 425

aca act gaa att gcc aaa gct tgc cct ggg gct ctg tgt aac ttt gct     1410
Thr Thr Glu Ile Ala Lys Ala Cys Pro Gly Ala Leu Cys Asn Phe Ala
                430                 435                 440

ttc tat ggt gga aag agc ttg tac cat cag tac atc cct acc ttc cat     1458
Phe Tyr Gly Gly Lys Ser Leu Tyr His Gln Tyr Ile Pro Thr Phe His
            445                 450                 455

gta tac aac tta ttt gtc ttt ctc tgg ctt ata aac ttc gtc att gca     1506
Val Tyr Asn Leu Phe Val Phe Leu Trp Leu Ile Asn Phe Val Ile Ala
        460                 465                 470

tta ggt cag tgc gcc ctt gct ggt gca ttc gct act tat tac tgg gcc     1554
Leu Gly Gln Cys Ala Leu Ala Gly Ala Phe Ala Thr Tyr Tyr Trp Ala
    475                 480                 485

atg aaa aaa cct gat gac atc cca cga tat cca ctt ttt act gca ttt     1602
Met Lys Lys Pro Asp Asp Ile Pro Arg Tyr Pro Leu Phe Thr Ala Phe
490                 495                 500                 505

gga cga gcc ata cga tat cac aca gga tcc cta gca ttt gga tct tta     1650
Gly Arg Ala Ile Arg Tyr His Thr Gly Ser Leu Ala Phe Gly Ser Leu
                510                 515                 520

att att gca tta att caa atg ttt aaa att gta cta gaa tac ttg gac     1698
Ile Ile Ala Leu Ile Gln Met Phe Lys Ile Val Leu Glu Tyr Leu Asp
            525                 530                 535

cac cgt ctt aaa cgt acc cag aac aca ttg tct aaa ttc cta cag tgc     1746
His Arg Leu Lys Arg Thr Gln Asn Thr Leu Ser Lys Phe Leu Gln Cys
        540                 545                 550

tgc ctg aga tgc tgc ttc tgg tgt ttg gaa aat gca ata aag ttt tta     1794
Cys Leu Arg Cys Cys Phe Trp Cys Leu Glu Asn Ala Ile Lys Phe Leu
    555                 560                 565

aac aga aat gcc tat att atg att gca ata tat ggc aga aac ttc tgc     1842
Asn Arg Asn Ala Tyr Ile Met Ile Ala Ile Tyr Gly Arg Asn Phe Cys
570                 575                 580                 585

agg tca gca aaa gat gct ttc aat ctg ctg atg aga aat gtt ttg aaa     1890
Arg Ser Ala Lys Asp Ala Phe Asn Leu Leu Met Arg Asn Val Leu Lys

                  590                 595                 600

gtt gca gtt aca gat gaa gtt aca tac ttt gta tta ttc ctg ggg aaa     1938
Val Ala Val Thr Asp Glu Val Thr Tyr Phe Val Leu Phe Leu Gly Lys
            605                 610                 615

ctt cta gtt gct gga agt ata ggt gtt ctg gcc ttc cta ttc ttc aca     1986
Leu Leu Val Ala Gly Ser Ile Gly Val Leu Ala Phe Leu Phe Phe Thr
        620                 625                 630

caa aga ctg cca gtg att gca caa gga cca gca tct tta aat tac tac     2034
Gln Arg Leu Pro Val Ile Ala Gln Gly Pro Ala Ser Leu Asn Tyr Tyr
    635                 640                 645

tgg gta cct ttg ctg aca gtc att ttt ggg tct tac ctg att gca cat     2082
Trp Val Pro Leu Leu Thr Val Ile Phe Gly Ser Tyr Leu Ile Ala His
650                 655                 660                 665

ggg ttc ttc agc gtc tat gca atg tgt gtt gaa aca att ttc atc tgc     2130
Gly Phe Phe Ser Val Tyr Ala Met Cys Val Glu Thr Ile Phe Ile Cys
                670                 675                 680

ttc ttg gaa gat tta gaa aga aat gat ggt tct act gca aga cct tat     2178
Phe Leu Glu Asp Leu Glu Arg Asn Asp Gly Ser Thr Ala Arg Pro Tyr
            685                 690                 695

tat gtg agt caa cct ttg ctg aag att ttc cag gag gaa aat cca caa     2226
Tyr Val Ser Gln Pro Leu Leu Lys Ile Phe Gln Glu Glu Asn Pro Gln
        700                 705                 710

act agg aag cag tagaagagca aactggtcgt cctacagctg tgtgttacct         2278
Thr Arg Lys Gln
    715

tttctccatc tgctgtgtct gtgcaacatt tgtttcataa gtgctttgtg tttagcaaca 2338
ctgtattcac gaccttgttg gcttgcattt gcatgtttta taccaaagct tatactgtac 2398
tatgtgaagc catcagaagt cgcaagggaa ttgttaataa cataaaacat ttttatacta 2458
aaaaaaaaaa aaaaa                                                  2473

<210> 2
<211> 717
<212> PRT
<213> Homo sapiens

<400> 2
Met Asn Asp Thr Glu Lys Pro Ala Asp Thr Pro Ser Glu Glu Glu Asp
1               5                   10                  15
Phe Gly Asp Pro Arg Thr Tyr Asp Pro Asp Phe Lys Gly Pro Val Ala
            20                  25                  30
Asn Arg Ser Cys Thr Asp Val Leu Cys Cys Met Ile Phe Leu Leu Cys
        35                  40                  45
Ile Ile Gly Tyr Ile Val Leu Gly Leu Val Ala Trp Val His Gly Asp
    50                  55                  60
Pro Arg Arg Ala Ala Tyr Pro Thr Asp Ser Gln Gly His Phe Cys Gly
65                  70                  75                  80
Gln Lys Gly Thr Pro Asn Glu Asn Lys Thr Ile Leu Phe Tyr Phe Asn
                85                  90                  95
Leu Leu Arg Cys Thr Ser Pro Ser Val Leu Leu Asn Leu Gln Cys Pro
            100                 105                 110
Thr Thr Gln Ile Cys Val Ser Lys Cys Pro Glu Lys Phe Leu Thr Tyr
        115                 120                 125

Val Glu Met Gln Leu Leu Tyr Thr Lys Asp Lys Ser Tyr Trp Glu Asp
    130                 135                 140
Tyr Arg Gln Phe Cys Lys Thr Thr Ala Lys Pro Val Lys Ser Leu Thr
145                 150                 155                 160
Gln Leu Leu Leu Asp Asp Asp Cys Pro Thr Ala Ile Phe Pro Ser Lys
                165                 170                 175
Pro Phe Leu Gln Arg Cys Phe Pro Asp Phe Ser Thr Lys Asn Gly Thr
            180                 185                 190
Leu Thr Ile Gly Ser Lys Met Met Phe Gln Asp Gly Asn Gly Gly Thr
        195                 200                 205
Arg Ser Val Val Glu Leu Gly Ile Ala Ala Asn Gly Ile Asn Lys Leu
    210                 215                 220
Leu Asp Ala Lys Ser Leu Gly Leu Lys Val Phe Glu Asp Tyr Ala Arg
225                 230                 235                 240
Thr Trp Tyr Trp Ile Leu Ile Gly Leu Thr Ile Ala Met Val Leu Ser
                245                 250                 255
Trp Ile Phe Leu Ile Leu Leu Arg Phe Ile Ala Gly Cys Leu Phe Trp
            260                 265                 270
Val Phe Met Ile Gly Val Ile Gly Ile Ile Gly Tyr Gly Ile Trp His
        275                 280                 285
Cys Tyr Gln Gln Tyr Thr Asn Leu Gln Glu Arg Pro Ser Ser Val Leu
    290                 295                 300
Thr Ile Tyr Asp Ile Gly Ile Gln Thr Asn Ile Ser Met Tyr Phe Glu
305                 310                 315                 320
Leu Gln Gln Thr Trp Phe Thr Phe Met Ile Ile Leu Cys Ile Ile Glu
                325                 330                 335
Val Ile Val Ile Leu Met Leu Ile Phe Leu Arg Asn Arg Ile Arg Val
            340                 345                 350
Ala Ile Ile Leu Leu Lys Glu Gly Ser Lys Ala Ile Gly Tyr Val Pro
        355                 360                 365
Ser Thr Leu Val Tyr Pro Ala Leu Thr Phe Ile Leu Leu Ser Ile Cys
    370                 375                 380
Ile Cys Tyr Trp Val Val Thr Ala Val Phe Leu Ala Thr Ser Gly Val
385                 390                 395                 400
Pro Val Tyr Lys Val Ile Ala Pro Gly Gly His Cys Ile His Glu Asn
                405                 410                 415
Gln Thr Cys Asp Pro Glu Ile Phe Asn Thr Thr Glu Ile Ala Lys Ala
            420                 425                 430
Cys Pro Gly Ala Leu Cys Asn Phe Ala Phe Tyr Gly Gly Lys Ser Leu
        435                 440                 445
Tyr His Gln Tyr Ile Pro Thr Phe His Val Tyr Asn Leu Phe Val Phe
    450                 455                 460
Leu Trp Leu Ile Asn Phe Val Ile Ala Leu Gly Gln Cys Ala Leu Ala
465                 470                 475                 480
Gly Ala Phe Ala Thr Tyr Tyr Trp Ala Met Lys Lys Pro Asp Asp Ile
                485                 490                 495
Pro Arg Tyr Pro Leu Phe Thr Ala Phe Gly Arg Ala Ile Arg Tyr His
            500                 505                 510
Thr Gly Ser Leu Ala Phe Gly Ser Leu Ile Ile Ala Leu Ile Gln Met
        515                 520                 525
Phe Lys Ile Val Leu Glu Tyr Leu Asp His Arg Leu Lys Arg Thr Gln
    530                 535                 540
Asn Thr Leu Ser Lys Phe Leu Gln Cys Cys Leu Arg Cys Cys Phe Trp
545                 550                 555                 560
Cys Leu Glu Asn Ala Ile Lys Phe Leu Asn Arg Asn Ala Tyr Ile Met
                565                 570                 575
Ile Ala Ile Tyr Gly Arg Asn Phe Cys Arg Ser Ala Lys Asp Ala Phe
            580                 585                 590
Asn Leu Leu Met Arg Asn Val Leu Lys Val Ala Val Thr Asp Glu Val
        595                 600                 605
Thr Tyr Phe Val Leu Phe Leu Gly Lys Leu Leu Val Ala Gly Ser Ile

610                 615                 620
Gly Val Leu Ala Phe Leu Phe Phe Thr Gln Arg Leu Pro Val Ile Ala
625                 630                 635                 640
Gln Gly Pro Ala Ser Leu Asn Tyr Tyr Trp Val Pro Leu Leu Thr Val
                645                 650                 655
Ile Phe Gly Ser Tyr Leu Ile Ala His Gly Phe Phe Ser Val Tyr Ala
            660                 665                 670
Met Cys Val Glu Thr Ile Phe Ile Cys Phe Leu Glu Asp Leu Glu Arg
        675                 680                 685
Asn Asp Gly Ser Thr Ala Arg Pro Tyr Tyr Val Ser Gln Pro Leu Leu
    690                 695                 700
Lys Ile Phe Gln Glu Glu Asn Pro Gln Thr Arg Lys Gln
705                 710                 715

<210> 3
<211> 2151
<212> DNA
<213> Homo sapiens

<220>
<221> CDS
<222> (1)...(2151)

<400> 3
atg aat gac aca gaa aaa cca gca gat act ccc tct gag gaa gag gac      48
Met Asn Asp Thr Glu Lys Pro Ala Asp Thr Pro Ser Glu Glu Glu Asp
 1               5                  10                  15

ttt ggt gat cca agg aca tat gac cca gat ttc aag ggg cct gtt gcc      96
Phe Gly Asp Pro Arg Thr Tyr Asp Pro Asp Phe Lys Gly Pro Val Ala
            20                  25                  30

aac agg agt tgt aca gat gtt ctg tgc tgt atg atc ttc cta ctg tgt     144
Asn Arg Ser Cys Thr Asp Val Leu Cys Cys Met Ile Phe Leu Leu Cys
        35                  40                  45

att att ggc tac att gtt tta gga ctt gtg gcc tgg gta cat ggg gac     192
Ile Ile Gly Tyr Ile Val Leu Gly Leu Val Ala Trp Val His Gly Asp
    50                  55                  60

ccc aga aga gca gcc tat cct aca gac agc cag ggc cac ttt tgt ggc     240
Pro Arg Arg Ala Ala Tyr Pro Thr Asp Ser Gln Gly His Phe Cys Gly
65                  70                  75                  80

cag aag ggc act ccc aat gag aac aag acc att ttg ttt tac ttt aac     288
Gln Lys Gly Thr Pro Asn Glu Asn Lys Thr Ile Leu Phe Tyr Phe Asn
                85                  90                  95

ctg tta cgc tgt acc agt ccc tcc gtg ttg cta aac cta cag tgc cct     336
Leu Leu Arg Cys Thr Ser Pro Ser Val Leu Leu Asn Leu Gln Cys Pro
            100                 105                 110

acc aca cag atc tgt gtc tcc aag tgc cca gaa aaa ttt tta acc tat     384
Thr Thr Gln Ile Cys Val Ser Lys Cys Pro Glu Lys Phe Leu Thr Tyr
        115                 120                 125

gtg gaa atg caa ctt ttg tac aca aaa gac aaa agc tac tgg gaa gac     432
Val Glu Met Gln Leu Leu Tyr Thr Lys Asp Lys Ser Tyr Trp Glu Asp
    130                 135                 140

tac cgt cag ttc tgt aag acc act gct aag cct gtg aag tct ctc aca 480
Tyr Arg Gln Phe Cys Lys Thr Thr Ala Lys Pro Val Lys Ser Leu Thr
145                 150                 155                 160

cag ctt tta ctg gat gat gat tgt cca aca gcg att ttt ccc agc aaa 528
Gln Leu Leu Leu Asp Asp Asp Cys Pro Thr Ala Ile Phe Pro Ser Lys
                165                 170                 175

cct ttt ctc cag aga tgt ttc cct gac ttc tct acc aaa aat ggc act 576
Pro Phe Leu Gln Arg Cys Phe Pro Asp Phe Ser Thr Lys Asn Gly Thr
            180                 185                 190

tta aca ata gga agt aag atg atg ttt caa gat gga aat gga ggg aca 624
Leu Thr Ile Gly Ser Lys Met Met Phe Gln Asp Gly Asn Gly Gly Thr
        195                 200                 205

aga agt gtt gta gaa ctc ggg att gct gca aat ggt atc aat aaa ctt 672
Arg Ser Val Val Glu Leu Gly Ile Ala Ala Asn Gly Ile Asn Lys Leu
    210                 215                 220

ctt gat gca aag tca ctt gga ttg aaa gtg ttt gaa gac tat gca aga 720
Leu Asp Ala Lys Ser Leu Gly Leu Lys Val Phe Glu Asp Tyr Ala Arg
225                 230                 235                 240

act tgg tat tgg att ctt att ggc ctg acg atc gcc atg gtc ctt agt 768
Thr Trp Tyr Trp Ile Leu Ile Gly Leu Thr Ile Ala Met Val Leu Ser
                245                 250                 255

tgg ata ttt ttg ata ctt ctg agg ttc ata gct gga tgc ctc ttc tgg 816
Trp Ile Phe Leu Ile Leu Leu Arg Phe Ile Ala Gly Cys Leu Phe Trp
            260                 265                 270

gtc ttc atg att ggt gtg att gga att ata ggt tat gga ata tgg cac 864
Val Phe Met Ile Gly Val Ile Gly Ile Ile Gly Tyr Gly Ile Trp His
        275                 280                 285

tgt tac cag cag tac acc aat ctt cag gaa cgc cca agt tct gta tta 912
Cys Tyr Gln Gln Tyr Thr Asn Leu Gln Glu Arg Pro Ser Ser Val Leu
    290                 295                 300

act atc tat gac atc ggg att cag act aac ata agc atg tac ttt gaa 960
Thr Ile Tyr Asp Ile Gly Ile Gln Thr Asn Ile Ser Met Tyr Phe Glu
305                 310                 315                 320

ctg caa caa aca tgg ttc aca ttt atg ata ata ctc tgc atc att gaa 1008
Leu Gln Gln Thr Trp Phe Thr Phe Met Ile Ile Leu Cys Ile Ile Glu
                325                 330                 335

gtg att gtc atc ctc atg ctg atc ttc ctc agg aat cga atc cga gtc 1056
Val Ile Val Ile Leu Met Leu Ile Phe Leu Arg Asn Arg Ile Arg Val
            340                 345                 350

gcc att atc ctg ctg aag gaa gga agc aaa gcc att gga tat gtt cct 1104
Ala Ile Ile Leu Leu Lys Glu Gly Ser Lys Ala Ile Gly Tyr Val Pro
        355                 360                 365

agt aca tta gtc tat cca gct tta act ttc att ttg ctc tca atc tgc 1152
Ser Thr Leu Val Tyr Pro Ala Leu Thr Phe Ile Leu Leu Ser Ile Cys
    370                 375                 380

att tgc tac tgg gtc gtg aca gca gtt ttc ttg gcg aca tcg ggg gta 1200

Ile Cys Tyr Trp Val Val Thr Ala Val Phe Leu Ala Thr Ser Gly Val
385                 390                 395                 400

cct gta tac aaa gtc ata gct cca ggg ggg cat tgt ata cat gaa aat      1248
Pro Val Tyr Lys Val Ile Ala Pro Gly Gly His Cys Ile His Glu Asn
                405                 410                 415

caa acc tgt gac cca gag att ttt aat aca act gaa att gcc aaa gct      1296
Gln Thr Cys Asp Pro Glu Ile Phe Asn Thr Thr Glu Ile Ala Lys Ala
            420                 425                 430

tgc cct ggg gct ctg tgt aac ttt gct ttc tat ggt gga aag agc ttg      1344
Cys Pro Gly Ala Leu Cys Asn Phe Ala Phe Tyr Gly Gly Lys Ser Leu
        435                 440                 445

tac cat cag tac atc cct acc ttc cat gta tac aac tta ttt gtc ttt      1392
Tyr His Gln Tyr Ile Pro Thr Phe His Val Tyr Asn Leu Phe Val Phe
    450                 455                 460

ctc tgg ctt ata aac ttc gtc att gca tta ggt cag tgc gcc ctt gct      1440
Leu Trp Leu Ile Asn Phe Val Ile Ala Leu Gly Gln Cys Ala Leu Ala
465                 470                 475                 480

ggt gca ttc gct act tat tac tgg gcc atg aaa aaa cct gat gac atc      1488
Gly Ala Phe Ala Thr Tyr Tyr Trp Ala Met Lys Lys Pro Asp Asp Ile
                485                 490                 495

cca cga tat cca ctt ttt act gca ttt gga cga gcc ata cga tat cac      1536
Pro Arg Tyr Pro Leu Phe Thr Ala Phe Gly Arg Ala Ile Arg Tyr His
            500                 505                 510

aca gga tcc cta gca ttt gga tct tta att att gca tta att caa atg      1584
Thr Gly Ser Leu Ala Phe Gly Ser Leu Ile Ile Ala Leu Ile Gln Met
        515                 520                 525

ttt aaa att gta cta gaa tac ttg gac cac cgt ctt aaa cgt acc cag      1632
Phe Lys Ile Val Leu Glu Tyr Leu Asp His Arg Leu Lys Arg Thr Gln
    530                 535                 540

aac aca ttg tct aaa ttc cta cag tgc tgc ctg aga tgc tgc ttc tgg      1680
Asn Thr Leu Ser Lys Phe Leu Gln Cys Cys Leu Arg Cys Cys Phe Trp
545                 550                 555                 560

tgt ttg gaa aat gca ata aag ttt tta aac aga aat gcc tat att atg      1728
Cys Leu Glu Asn Ala Ile Lys Phe Leu Asn Arg Asn Ala Tyr Ile Met
                565                 570                 575

att gca ata tat ggc aga aac ttc tgc agg tca gca aaa gat gct ttc      1776
Ile Ala Ile Tyr Gly Arg Asn Phe Cys Arg Ser Ala Lys Asp Ala Phe
            580                 585                 590

aat ctg ctg atg aga aat gtt ttg aaa gtt gca gtt aca gat gaa gtt      1824
Asn Leu Leu Met Arg Asn Val Leu Lys Val Ala Val Thr Asp Glu Val
        595                 600                 605

aca tac ttt gta tta ttc ctg ggg aaa ctt cta gtt gct gga agt ata      1872
Thr Tyr Phe Val Leu Phe Leu Gly Lys Leu Leu Val Ala Gly Ser Ile
    610                 615                 620

ggt gtt ctg gcc ttc cta ttc ttc aca caa aga ctg cca gtg att gca      1920
Gly Val Leu Ala Phe Leu Phe Phe Thr Gln Arg Leu Pro Val Ile Ala

625                 630                 635                 640

caa gga cca gca tct tta aat tac tac tgg gta cct ttg ctg aca gtc    1968
Gln Gly Pro Ala Ser Leu Asn Tyr Tyr Trp Val Pro Leu Leu Thr Val
                645                 650                 655

att ttt ggg tct tac ctg att gca cat ggg ttc ttc agc gtc tat gca    2016
Ile Phe Gly Ser Tyr Leu Ile Ala His Gly Phe Phe Ser Val Tyr Ala
            660                 665                 670

atg tgt gtt gaa aca att ttc atc tgc ttc ttg gaa gat tta gaa aga    2064
Met Cys Val Glu Thr Ile Phe Ile Cys Phe Leu Glu Asp Leu Glu Arg
        675                 680                 685

aat gat ggt tct act gca aga cct tat tat gtg agt caa cct ttg ctg    2112
Asn Asp Gly Ser Thr Ala Arg Pro Tyr Tyr Val Ser Gln Pro Leu Leu
    690                 695                 700

aag att ttc cag gag gaa aat cca caa act agg aag cag                2151
Lys Ile Phe Gln Glu Glu Asn Pro Gln Thr Arg Lys Gln
705                 710                 715


<210> 4
<211> 2233
<212> DNA
<213> Homo sapiens

<220>
<221> CDS
<222> (110)...(2068)

<221> 5'UTR
<222> (1)...(109)

<221> 3'UTR
<222> (2069)...(2233)

<400> 4
   cccacgcgtc cgccagcccc ggccccggcc ccggctcgcg ggcgctgcgt ctccgcgtac 60
   aggaggcggc ggcggctccc agtcaccggc ccccgccggc gagcgcacg atg cac tgc 118
   ctg ggc gcc gag tac ctg gtt tct gca gaa gga gcc cct agg caa agg    166
   gag tgg cga ccc cag att tat agg aaa tgc aca gat acg gca tgg tta    214
   ttc ctg ttc ttt ctc ttt tgg act ggt ttg gtg ttt atc atg ggc tac    262
   tcg gtg gtg gct gga gcc gcg gga aga ctc ctc ttt ggc tat gac agc    310
   ttt ggc aac atg tgt ggc aag aag aac tcc ccc gtg gaa ggg gcc cct    358
   ctt tca ggg cag gac atg acc cta aaa aaa cac gtg ttc ttt atg aat     406
   tcc tgc aac ctg gaa gtc aaa ggt acg cag ctc aac cgc atg gcc ctc     454
   tgt gta tcc aac tgc cct gaa gag cag ctt gac tcc ctg gaa gag gtc     502
   cag ttc ttt gca aac acc agt ggg tcc ttc ctg tgt gtt tat agt ttg     550
   aat tcc ttc aac tat acc cac agt cca aaa gca gac tca ctg tgt ccc     598
   agg cta cca gtt cct cca agc aag tca ttt ccc tta ttt aac cga tgt     646
   gtc cct caa aca cct gag tgc tac tcc cta ttt gca tct gtt ttg ata     694
   aat gat gtt gac acc ctc cac cga att cta agt gga atc atg tcg gga     742
   aga gat aca atc ctt ggc ctg tgt atc ctc gca tta gcc ttg tct ttg     790
   gcc atg atg ttt acc ttc aga ttc atc acc acc ctt ctg gtt cac att     838
   ttc att tca ttg gtt att ttg gga ttg ttg ttt gtc tgc ggt gtt tta     886
   tgg tgg ctg tat tat gac tat acc aac gac ctc agc ata gaa ttg gac     934
   aca gaa agg gaa aat atg aag tgc gtg ctg ggg ttt gct atc gta tcc     982
   aca ggc atc acg gca gtg ctg ctc gtc ttg att ttt gtt ctc aga aag     1030
   aga ata aaa ttg aca gtt gag ctt ttc caa atc aca aat aaa gcc atc     1078
   agc agt gct ccc ttc ctg ctg ttc cag cca ctg tgg aca ttt gcc atc     1126
   ctc att ttc ttc tgg gtc ctc tgg gtg gct gtg ctg ctg agc ctg gga     1174
   act gca gga gct gcc cag gtt atg gaa ggc ggc caa gtg gaa tat aag     1222
   ccc ctt tcg ggc att cgg tac atg tgg tcg tac cat tta att ggc ctc     1270
   atc tgg act agt gaa ttc atc ctt gcg tgc cag caa atg act ata gct     1318
   ggg gca gtg gtt act tgt tat ttc aac aga agt aaa aat gat cct cct     1366
   gat cat ccc atc ctt tcg tct ctc tcc att ctc ttc ttc tac cat caa     1414
   gga acc att gtg aaa ggg tca ttt tta atc tct gtg gtg agg att ccg     1462
   aga atc att gtc atg tac atg caa aac gca ctg aaa gaa cag cag cat     1510
   ggt gca ttg tcc agg tac ctg ttc cga tgc tgc tac tgc tgt ttc tgg     1558
   tgt ctt gac aaa tac ctg ctc cat ctc aac cag aat gca tat act aca     1606
   act gct att aat ggg aca gat ttc tgt aca tca gca aaa gat gca ttc     1654
   aaa atc ttg tcc aag aac tca agt cac ttt aca tct att aac tgc ttt     1702
   gga gac ttc ata att ttt cta gga aag gtg tta gtg gtg tgt ttc act     1750
   gtt ttt gga gga ctc atg gct ttt aac tac aat cgg gca ttc cag gtg     1798
   tgg gca gtc cct ctg tta ttg gta gct ttt ttt gcc tac tta gta gcc    1846
   cat agt ttt tta tct gtg ttt gaa act gtg ctg gat gca ctt ttc ctg    1894
   tgt ttt gct gtt gat ctg gaa aca aat gat gga tcg tca gaa aag ccc    1942
   tac ttt atg gat caa gaa ttt ctg agt ttc gta aaa agg agc aac aaa    1990
   tta aac aat gca agg gca cag cag gac aag cac tca tta agg aat gag    2038
   gag gga aca gaa ctc cag gcc att gtg aga tagataccca tttaggtatc      2088
   tgtacctgga aaacatttcc ttctaagagc catttacaga atagaagatg agaccactag 2148
   agaaaagtta gtgaattttt ttttaaaaga cctaataaac cctattcttc ctcaaaaaaa 2208
   aaaaaaaaaa aaaaaaaaaa aaaaa                                       2233

<210> 5
<211> 653
<212> PRT
<213> Homo sapiens

<400> 5

<210> 6
<211> 1959
<212> DNA

<213> Homo sapiens

<220>
<221> CDS
<222> (1)...(1959)

<400> 6
atg cac tgc ctg ggc gcc gag tac ctg gtt tct gca gaa gga gcc cct     48
agg caa agg gag tgg cga ccc cag att tat agg aaa tgc aca gat acg     96
gca tgg tta ttc ctg ttc ttt ctc ttt tgg act ggt ttg gtg ttt atc    144
atg ggc tac tcg gtg gtg gct gga gcc gcg gga aga ctc ctc ttt ggc    192
tat gac agc ttt ggc aac atg tgt ggc aag aag aac tcc ccc gtg gaa    240
ggg gcc cct ctt tca ggg cag gac atg acc cta aaa aaa cac gtg ttc    288
ttt atg aat tcc tgc aac ctg gaa gtc aaa ggt acg cag ctc aac cgc    336
atg gcc ctc tgt gta tcc aac tgc cct gaa gag cag ctt gac tcc ctg    384
gaa gag gtc cag ttc ttt gca aac acc agt ggg tcc ttc ctg tgt gtt    432
tat agt ttg aat tcc ttc aac tat acc cac agt cca aaa gca gac tca    480
ctg tgt ccc agg cta cca gtt cct cca agc aag tca ttt ccc tta ttt    528
aac cga tgt gtc cct caa aca cct gag tgc tac tcc cta ttt gca tct    576
gtt ttg ata aat gat gtt gac acc ctc cac cga att cta agt gga atc    624
atg tcg gga aga gat aca atc ctt ggc ctg tgt atc ctc gca tta gcc    672
ttg tct ttg gcc atg atg ttt acc ttc aga ttc atc acc acc ctt ctg     720
gtt cac att ttc att tca ttg gtt att ttg gga ttg ttg ttt gtc tgc     768
ggt gtt tta tgg tgg ctg tat tat gac tat acc aac gac ctc agc ata     816
gaa ttg gac aca gaa agg gaa aat atg aag tgc gtg ctg ggg ttt gct     864
atc gta tcc aca ggc atc acg gca gtg ctg ctc gtc ttg att ttt gtt     912
ctc aga aag aga ata aaa ttg aca gtt gag ctt ttc caa atc aca aat     960
aaa gcc atc agc agt gct ccc ttc ctg ctg ttc cag cca ctg tgg aca     1008
ttt gcc atc ctc att ttc ttc tgg gtc ctc tgg gtg gct gtg ctg ctg     1056
agc ctg gga act gca gga gct gcc cag gtt atg gaa ggc ggc caa gtg     1104
gaa tat aag ccc ctt tcg ggc att cgg tac atg tgg tcg tac cat tta     1152
att ggc ctc atc tgg act agt gaa ttc atc ctt gcg tgc cag caa atg     1200
act ata gct ggg gca gtg gtt act tgt tat ttc aac aga agt aaa aat     1248
gat cct cct gat cat ccc atc ctt tcg tct ctc tcc att ctc ttc ttc     1296
tac cat caa gga acc att gtg aaa ggg tca ttt tta atc tct gtg gtg     1344
agg att ccg aga atc att gtc atg tac atg caa aac gca ctg aaa gaa     1392
cag cag cat ggt gca ttg tcc agg tac ctg ttc cga tgc tgc tac tgc     1440
tgt ttc tgg tgt ctt gac aaa tac ctg ctc cat ctc aac cag aat gca     1488
tat act aca act gct att aat ggg aca gat ttc tgt aca tca gca aaa     1536
gat gca ttc aaa atc ttg tcc aag aac tca agt cac ttt aca tct att     1584
aac tgc ttt gga gac ttc ata att ttt cta gga aag gtg tta gtg gtg     1632
tgt ttc act gtt ttt gga gga ctc atg gct ttt aac tac aat cgg gca     1680
ttc cag gtg tgg gca gtc cct ctg tta ttg gta gct ttt ttt gcc tac     1728
tta gta gcc cat agt ttt tta tct gtg ttt gaa act gtg ctg gat gca     1776
ctt ttc ctg tgt ttt gct gtt gat ctg gaa aca aat gat gga tcg tca     1824
gaa aag ccc tac ttt atg gat caa gaa ttt ctg agt ttc gta aaa agg     1872
agc aac aaa tta aac aat gca agg gca cag cag gac aag cac tca tta     1920
agg aat gag gag gga aca gaa ctc cag gcc att gtg aga                 1959

<210> 7
<211> 654
<212> PRT
<213> Homo sapiens

<400> 7
Met Gly Cys Cys Ser Ser Ala Ser Ser Ala Ala Gln Ser Ser Lys Arg
 1               5                  10                  15
Glu Trp Lys Pro Leu Glu Asp Arg Ser Cys Thr Asp Ile Pro Trp Leu
            20                  25                  30
Leu Leu Phe Ile Leu Phe Cys Ile Gly Met Gly Phe Ile Cys Gly Phe

35                  40                  45
Ser Ile Ala Thr Gly Ala Ala Ala Arg Leu Val Ser Gly Tyr Asp Ser
    50                  55                  60
Tyr Gly Asn Ile Cys Gly Gln Lys Asn Thr Lys Leu Glu Ala Ile Pro
65                  70                  75                  80
Asn Ser Gly Met Asp His Thr Gln Arg Lys Tyr Val Phe Phe Leu Asp
                85                  90                  95
Pro Cys Asn Leu Asp Leu Ile Asn Arg Lys Ile Lys Ser Val Ala Leu
            100                 105                 110
Cys Val Ala Ala Cys Pro Arg Gln Glu Leu Lys Thr Leu Ser Asp Val
        115                 120                 125
Gln Lys Phe Ala Glu Ile Asn Gly Ser Ala Leu Cys Ser Tyr Asn Leu
    130                 135                 140
Lys Pro Ser Glu Tyr Thr Thr Ser Pro Lys Ser Ser Val Leu Cys Pro
145                 150                 155                 160
Lys Leu Pro Val Pro Ala Ser Ala Pro Ile Pro Phe Phe His Arg Cys
                165                 170                 175
Ala Pro Val Asn Ile Ser Cys Tyr Ala Lys Phe Ala Glu Ala Leu Ile
            180                 185                 190
Thr Phe Val Ser Asp Asn Ser Val Leu His Arg Leu Ile Ser Gly Val
        195                 200                 205
Met Thr Ser Lys Glu Ile Ile Leu Gly Leu Cys Leu Leu Ser Leu Val
    210                 215                 220
Leu Ser Met Ile Leu Met Val Ile Ile Arg Tyr Ile Ser Arg Val Leu
225                 230                 235                 240
Val Trp Ile Leu Thr Ile Leu Val Ile Leu Gly Ser Leu Gly Gly Thr
                245                 250                 255
Gly Val Leu Trp Trp Leu Tyr Ala Lys Gln Arg Arg Ser Pro Lys Glu
            260                 265                 270
Thr Val Thr Pro Glu Gln Leu Gln Ile Ala Glu Asp Asn Leu Arg Ala
        275                 280                 285
Leu Leu Ile Tyr Ala Ile Ser Ala Thr Val Phe Thr Val Ile Leu Phe
    290                 295                 300
Leu Ile Met Leu Val Met Arg Lys Arg Val Ala Leu Thr Ile Ala Leu
305                 310                 315                 320
Phe His Val Ala Gly Lys Val Phe Ile His Leu Pro Leu Leu Val Phe
                325                 330                 335
Gln Pro Phe Trp Thr Phe Phe Ala Leu Val Leu Phe Trp Val Tyr Trp
            340                 345                 350
Ile Met Thr Leu Leu Phe Leu Gly Thr Thr Gly Ser Pro Val Gln Asn
        355                 360                 365
Glu Gln Gly Phe Val Glu Phe Lys Ile Ser Gly Pro Leu Gln Tyr Met
    370                 375                 380
Trp Trp Tyr His Val Val Gly Leu Ile Trp Ile Ser Glu Phe Ile Leu
385                 390                 395                 400
Ala Cys Gln Gln Met Thr Val Ala Gly Ala Val Val Thr Tyr Tyr Phe
                405                 410                 415
Thr Arg Asp Lys Arg Asn Leu Pro Phe Thr Pro Ile Leu Ala Ser Val
            420                 425                 430
Asn Arg Leu Ile Arg Tyr His Leu Gly Thr Val Ala Lys Gly Ser Phe
        435                 440                 445
Ile Ile Thr Leu Val Lys Ile Pro Arg Met Ile Leu Met Tyr Ile His
    450                 455                 460
Ser Gln Leu Lys Gly Lys Glu Asn Ala Cys Ala Arg Cys Val Leu Lys
465                 470                 475                 480
Ser Cys Ile Cys Cys Leu Trp Cys Leu Glu Lys Cys Leu Asn Tyr Leu
                485                 490                 495
Asn Gln Asn Ala Tyr Thr Ala Thr Ala Ile Asn Ser Thr Asn Phe Cys
            500                 505                 510
Thr Ser Ala Lys Asp Ala Phe Val Ile Leu Val Glu Asn Ala Leu Arg
        515                 520                 525

Val Ala Thr Ile Asn Thr Val Gly Asp Phe Met Leu Phe Leu Gly Lys
        530                 535                 540
Val Leu Ile Val Cys Ser Thr Gly Leu Ala Gly Ile Met Leu Leu Asn
545                 550                 555                 560
Tyr Gln Gln Asp Tyr Thr Val Trp Val Leu Pro Leu Ile Ile Val Cys
                565                 570                 575
Leu Phe Ala Phe Leu Val Ala His Cys Phe Leu Ser Ile Tyr Glu Met
            580                 585                 590
Val Val Asp Val Leu Phe Leu Cys Phe Ala Ile Asp Thr Lys Tyr Asn
        595                 600                 605
Asp Gly Ser Pro Gly Arg Glu Phe Tyr Met Asp Lys Val Leu Met Glu
    610                 615                 620
Phe Val Glu Asn Ser Arg Lys Ala Met Lys Glu Ala Gly Lys Gly Gly
625                 630                 635                 640
Val Ala Asp Ser Arg Glu Leu Lys Pro Met Leu Lys Lys Arg
                645                 650

<210> 8
<211> 706
<212> PRT
<213> Homo sapiens

<400> 8
Met Gly Asp Glu Arg Pro His Tyr Tyr Gly Lys His Gly Thr Pro Gln
 1               5                  10                  15
Lys Tyr Asp Pro Thr Phe Lys Gly Pro Ile Tyr Asn Arg Gly Cys Thr
            20                  25                  30
Asp Ile Ile Cys Cys Val Phe Leu Leu Leu Ala Ile Val Gly Tyr Val
        35                  40                  45
Ala Val Gly Ile Ile Ala Trp Thr His Gly Asp Pro Arg Lys Val Ile
    50                  55                  60
Tyr Pro Thr Asp Ser Arg Gly Glu Phe Cys Gly Gln Lys Gly Thr Lys
65                  70                  75                  80
Asn Glu Asn Lys Pro Tyr Leu Phe Tyr Phe Asn Ile Val Lys Cys Ala
                85                  90                  95
Ser Pro Leu Val Leu Leu Glu Phe Gln Cys Pro Thr Pro Gln Ile Cys
            100                 105                 110
Val Glu Lys Cys Pro Asp Arg Tyr Leu Thr Tyr Leu Asn Ala Arg Ser
        115                 120                 125
Ser Arg Asp Phe Glu Tyr Tyr Lys Gln Phe Cys Val Pro Gly Phe Lys
    130                 135                 140
Asn Asn Lys Gly Val Ala Glu Val Leu Arg Asp Gly Asp Cys Pro Ala
145                 150                 155                 160
Val Leu Ile Pro Ser Lys Pro Leu Ala Arg Arg Cys Phe Pro Ala Ile
                165                 170                 175
His Ala Tyr Lys Gly Val Leu Met Val Gly Asn Glu Thr Thr Tyr Glu
            180                 185                 190
Asp Gly His Gly Ser Arg Lys Asn Ile Thr Asp Leu Val Glu Gly Ala
        195                 200                 205
Lys Lys Ala Asn Gly Val Leu Glu Ala Arg Gln Leu Ala Met Arg Ile
    210                 215                 220
Phe Glu Asp Tyr Thr Val Ser Trp Tyr Trp Ile Ile Ile Gly Leu Val
225                 230                 235                 240
Ile Ala Met Ala Met Ser Leu Leu Phe Ile Ile Leu Leu Arg Phe Leu
                245                 250                 255
Ala Gly Ile Met Val Trp Val Met Ile Ile Met Val Ile Leu Val Leu
            260                 265                 270
Gly Tyr Gly Ile Phe His Cys Tyr Met Glu Tyr Ser Arg Leu Arg Gly
        275                 280                 285
Glu Ala Gly Ser Asp Val Ser Leu Val Asp Leu Gly Phe Gln Thr Asp

290                 295                 300
Phe Arg Val Tyr Leu His Leu Arg Gln Thr Trp Leu Ala Phe Met Ile
305                 310                 315                 320
Ile Leu Ser Ile Leu Glu Val Ile Ile Ile Leu Leu Leu Ile Phe Leu
                325                 330                 335
Arg Lys Arg Ile Leu Ile Ala Ile Ala Leu Ile Lys Glu Ala Ser Arg
            340                 345                 350
Ala Val Gly Tyr Val Met Cys Ser Leu Leu Tyr Pro Leu Val Thr Phe
        355                 360                 365
Phe Leu Leu Cys Leu Cys Ile Ala Tyr Trp Ala Ser Thr Ala Val Phe
    370                 375                 380
Leu Ser Thr Ser Asn Glu Ala Val Tyr Lys Ile Phe Asp Asp Ser Pro
385                 390                 395                 400
Cys Pro Phe Thr Ala Lys Thr Cys Asn Pro Glu Thr Phe Pro Ser Ser
                405                 410                 415
Asn Glu Ser Arg Gln Cys Pro Asn Ala Arg Cys Gln Phe Ala Phe Tyr
            420                 425                 430
Gly Gly Glu Ser Gly Tyr His Arg Ala Leu Leu Gly Leu Gln Ile Phe
        435                 440                 445
Asn Ala Phe Met Phe Phe Trp Leu Ala Asn Phe Val Leu Ala Leu Gly
    450                 455                 460
Gln Val Thr Leu Ala Gly Ala Phe Ala Ser Tyr Tyr Trp Ala Leu Arg
465                 470                 475                 480
Lys Pro Asp Asp Leu Pro Ala Phe Pro Leu Phe Ser Ala Phe Gly Arg
                485                 490                 495
Ala Leu Arg Tyr His Thr Gly Ser Leu Ala Phe Gly Ala Leu Ile Leu
            500                 505                 510
Ala Ile Val Gln Ile Ile Arg Val Ile Leu Glu Tyr Leu Asp Gln Arg
        515                 520                 525
Leu Lys Gly Ala Glu Asn Lys Phe Ala Lys Cys Leu Met Thr Cys Leu
    530                 535                 540
Lys Cys Cys Phe Trp Cys Leu Glu Lys Phe Ile Lys Phe Leu Asn Arg
545                 550                 555                 560
Asn Ala Tyr Ile Met Ile Ala Ile Tyr Gly Thr Asn Phe Cys Thr Ser
                565                 570                 575
Ala Arg Asn Ala Phe Phe Leu Leu Met Arg Asn Ile Ile Arg Val Ala
            580                 585                 590
Val Leu Asp Lys Val Thr Asp Phe Leu Phe Leu Leu Gly Lys Leu Leu
        595                 600                 605
Ile Val Gly Ser Val Gly Ile Leu Ala Phe Phe Phe Phe Thr His Arg
    610                 615                 620
Ile Arg Ile Val Gln Asp Thr Ala Pro Pro Leu Asn Tyr Tyr Trp Val
625                 630                 635                 640
Pro Ile Leu Thr Val Ile Val Gly Ser Tyr Leu Ile Ala His Gly Phe
                645                 650                 655
Phe Ser Val Tyr Gly Met Cys Val Asp Thr Leu Phe Leu Cys Phe Leu
            660                 665                 670
Glu Asp Leu Glu Arg Asn Asp Gly Ser Ala Glu Arg Pro Tyr Phe Met
        675                 680                 685
Ser Ser Thr Leu Lys Lys Leu Leu Asn Lys Thr Asn Lys Lys Ala Ala
    690                 695                 700
Glu Ser
705

<210> 9
<211> 653
<212> PRT
<213> Rattus norvegicus

<400> 9

Met Gly Cys Cys Ser Ser Ala Ser Ala Ala Gln Ser Ser Lys Arg Glu
1               5                   10                  15
Trp Lys Pro Leu Glu Asp Arg Ser Cys Thr Asp Ile Pro Trp Leu Leu
            20                  25                  30
Leu Phe Val Leu Phe Cys Ile Gly Met Gly Phe Ile Cys Gly Phe Ser
        35                  40                  45
Val Ala Thr Gly Ala Ala Ala Arg Leu Val Ser Gly Tyr Asp Ser Tyr
    50                  55                  60
Gly Asn Ile Cys Gly Gln Arg Asn Ala Lys Leu Glu Ala Ile Ala Asn
65                  70                  75                  80
Ser Gly Leu Asp His Thr His Arg Lys Tyr Val Phe Phe Leu Asp Pro
                85                  90                  95
Cys Asn Leu Asp Leu Ile Asn Arg Lys Ile Lys Ser Met Ala Leu Cys
            100                 105                 110
Val Ala Ala Cys Pro Arg Gln Glu Leu Lys Thr Leu Ser Asp Val Gln
        115                 120                 125
Lys Phe Ala Glu Ile Asn Gly Ser Ala Leu Cys Ser Tyr Asn Ile Lys
    130                 135                 140
Pro Ser Glu Tyr Thr Leu Thr Ala Lys Ser Ser Ala Phe Cys Pro Lys
145                 150                 155                 160
Leu Pro Val Pro Ala Ser Ala Pro Ile Pro Phe Phe His Arg Cys Ala
                165                 170                 175
Pro Val Asn Ile Ser Cys Tyr Ala Lys Phe Ala Glu Ala Leu Ile Thr
            180                 185                 190
Phe Val Ser Asp Asn Ser Val Leu His Arg Leu Ile Ser Gly Val Met
        195                 200                 205
Thr Ser Lys Glu Ile Ile Leu Gly Leu Cys Leu Leu Ser Leu Val Leu
    210                 215                 220
Ser Met Ile Leu Met Val Ile Ile Arg Tyr Ile Ser Arg Val Leu Val
225                 230                 235                 240
Trp Ile Leu Thr Ile Leu Val Ile Leu Gly Ser Leu Gly Gly Thr Gly
                245                 250                 255
Val Leu Trp Trp Leu Tyr Ala Lys Gln Arg Ser Ser Pro Lys Glu Thr
            260                 265                 270
Val Ile Pro Glu Gln Leu Gln Ile Ala Glu Asp Asn Leu Arg Ala Leu
        275                 280                 285
Leu Ile Tyr Ala Ile Ser Ala Thr Val Phe Thr Val Ile Leu Phe Leu
    290                 295                 300
Ile Met Leu Val Met Arg Lys Arg Val Ala Leu Thr Ile Ala Leu Phe
305                 310                 315                 320
His Val Ala Gly Lys Val Phe Ile His Leu Pro Leu Leu Val Phe Gln
                325                 330                 335
Pro Phe Trp Thr Phe Phe Ala Leu Val Leu Phe Trp Ala Tyr Trp Ile
            340                 345                 350
Met Thr Leu Leu Phe Leu Gly Thr Thr Gly Ser Ala Val Gln Asn Glu
        355                 360                 365
Gln Gly Phe Val Glu Tyr Lys Ile Ser Gly Pro Leu Gln Tyr Met Trp
    370                 375                 380
Trp Tyr His Val Val Gly Leu Ile Trp Ile Ser Glu Phe Ile Leu Ala
385                 390                 395                 400
Cys Gln Gln Met Thr Val Ala Gly Ala Val Val Thr Tyr Tyr Phe Thr
                405                 410                 415
Arg Asp Lys Arg Asn Leu Pro Phe Thr Pro Ile Leu Ala Ser Val Asn
            420                 425                 430
Arg Leu Ile Arg Tyr His Leu Gly Thr Val Ala Lys Gly Ser Phe Ile
        435                 440                 445
Ile Thr Leu Val Lys Ile Pro Arg Met Ile Leu Met Tyr Ile His Ser
    450                 455                 460
Gln Leu Lys Gly Lys Glu Asn Ala Cys Ala Arg Cys Met Leu Lys Ser
465                 470                 475                 480
Cys Ile Cys Cys Leu Trp Cys Leu Glu Lys Cys Leu Ser Tyr Leu Asn

                485                 490                 495
Gln Asn Ala Tyr Thr Ala Thr Ala Ile Asn Ser Thr Asn Phe Cys Thr
            500                 505                 510
Ser Ala Lys Asp Ala Phe Val Ile Leu Val Glu Asn Ala Leu Arg Val
        515                 520                 525
Ala Ala Ile Asn Thr Val Gly Asp Phe Met Leu Phe Leu Gly Lys Val
    530                 535                 540
Leu Ile Val Cys Ser Thr Gly Leu Ala Gly Ile Met Leu Leu Asn Tyr
545                 550                 555                 560
Gln Gln Asp Tyr Thr Val Trp Val Leu Pro Leu Ile Ile Val Cys Leu
                565                 570                 575
Phe Ala Phe Leu Val Ala His Cys Phe Leu Ser Ile Tyr Glu Met Val
            580                 585                 590
Val Asp Val Leu Phe Leu Cys Phe Ala Ile Asp Thr Lys Tyr Asn Asp
        595                 600                 605
Gly Ser Pro Gly Arg Glu Phe Tyr Met Asp Lys Val Leu Met Glu Phe
    610                 615                 620
Val Glu Asn Ser Arg Lys Ala Met Lys Glu Ala Gly Lys Gly Gly Ala
625                 630                 635                 640
Ala Asp Ala Arg Glu Leu Lys Pro Met Leu Arg Lys Arg
                645                 650

<210> 10
<211> 646
<212> PRT
<213> Torpedo marmorata

<400> 10
Met Gly Cys Cys Gly Cys Gly Ser Glu Glu Gly Ser Val Arg Gln Trp
1               5                   10                  15
Lys Pro Leu Glu Gln Arg Ser Cys Thr Asp Val Leu Trp Leu Leu Ile
            20                  25                  30
Phe Val Leu Phe Cys Ile Gly Met Ala Ile Ile Cys Gly Phe Ala Ile
        35                  40                  45
Ala Ser Gly Ala Ala Gln Arg Leu Val Phe Gly Tyr Asp Ser Tyr Gly
    50                  55                  60
Asn Ile Cys Gly His Lys Asn Thr Glu Ile Lys Asp Val Thr Met Ser
65                  70                  75                  80
Gly Leu Asp His Thr Asp Lys Lys Tyr Val Phe Phe Phe Glu Pro Cys
                85                  90                  95
Asn Trp Asp Met Val His Leu Lys Ile Leu Ser Val Ala Leu Cys Val
            100                 105                 110
Thr Lys Cys Pro Asp Met Asp Leu Lys Thr Leu Glu Asp Val Arg Asn
        115                 120                 125
Phe Ala Lys Tyr Asn Gly Ser Arg Leu Cys Leu Tyr Asn Leu Asp Pro
    130                 135                 140
Thr Gln Tyr Thr Ser Lys Asn Ser Lys Ser Cys Pro Ile Leu Pro Val
145                 150                 155                 160
Lys Ser Ser Lys Pro Ile Pro Phe Phe His Arg Cys Val Pro Met Asp
                165                 170                 175
Ser Gly Cys Lys Ile Asn Phe Lys Ala Leu Thr Thr Phe Val Ser Tyr
            180                 185                 190
Asn Ser Val Leu Gln Arg Val Ile Thr Gly Val Met Thr Ser Lys Glu
        195                 200                 205
Ile Ile Val Gly Leu Cys Leu Met Ser Leu Val Leu Ser Ile Leu Leu
    210                 215                 220
Met Val Ile Ile Arg Tyr Ile Ser Lys Val Leu Val Trp Ile Leu Ala
225                 230                 235                 240
Ile Leu Thr Ile Ile Gly Ser Ile Gly Gly Thr Ala Val Leu Trp Trp
                245                 250                 255

Leu Tyr Ala Asp His Lys Lys Thr Leu Lys Leu Asp Pro Ser Gln Gly
            260                 265                 270
Asp Val Ala Ala Asp Asn Val Thr Ala Leu Leu Val Cys Ala Ile Ile
        275                 280                 285
Ala Thr Val Ile Thr Val Ile Leu Leu Leu Leu Met Leu Ile Met Arg
    290                 295                 300
Lys Arg Val Ala Leu Thr Ile Ala Leu Phe His Val Ala Gly Lys Val
305                 310                 315                 320
Phe Ile His Ile Pro Phe Leu Ile Phe Gln Ser Leu Trp Thr Phe Leu
                325                 330                 335
Ala Leu Ala Phe Phe Trp Ile Tyr Trp Ile Ala Val Leu Leu Leu Leu
            340                 345                 350
Ala Thr Ala Gly Tyr Pro Gln Lys Lys Asp Gln Gly Tyr Val Glu Phe
        355                 360                 365
Lys Val Ser Gly Pro Leu Gln Tyr Thr Trp Ile Tyr His Leu Val Gly
    370                 375                 380
Leu Ile Trp Ile Ser Glu Phe Ile Leu Ala Cys Gln Gln Met Thr Ile
385                 390                 395                 400
Ala Gly Ala Val Val Thr Tyr Tyr Phe Thr Arg Asp Lys His Asn Leu
                405                 410                 415
Pro Ala Thr Pro Ile Leu Ala Ser Met Cys Arg Leu Ile Lys Tyr His
            420                 425                 430
Leu Gly Thr Val Ala Lys Gly Ser Phe Ile Ile Thr Leu Ile Lys Ile
        435                 440                 445
Pro Gln Met Ile Leu Val Tyr Ile His Ser Gln Leu Lys Gly Lys Glu
    450                 455                 460
Asn Ala Cys Ala Lys Cys Met Leu Lys Ala Cys Met Cys Cys Leu Trp
465                 470                 475                 480
Cys Leu Glu Lys Cys Leu Leu Tyr Leu Asn Arg Asn Ala Tyr Ile Ala
                485                 490                 495
Thr Ser Ile Asn Gly Thr Ser Phe Cys Thr Ser Ala Lys Asp Ala Ile
            500                 505                 510
Val Ile Leu Val Glu Asn Ala Met Arg Val Ala Ala Ile Asn Thr Val
        515                 520                 525
Gly Asp Phe Val Leu Phe Leu Gly Lys Leu Leu Ile Val Leu Val Thr
    530                 535                 540
Gly Phe Val Gly Ile Ile Leu Leu Asn Tyr Gln Arg Asp Tyr Thr Val
545                 550                 555                 560
Trp Val Leu Pro Leu Ile Ile Ile Cys Leu Phe Ala Phe Phe Val Ser
                565                 570                 575
His Cys Phe Leu Ser Ile Tyr Glu Met Val Val Asp Val Leu Phe Leu
            580                 585                 590
Cys Phe Ala Val Asp Cys Lys His Asn Asp Gly Ser Pro Gly Arg Glu
        595                 600                 605
Tyr Tyr Met Asp Lys Ser Leu Met Glu Phe Met Asp Glu Ser Arg Lys
    610                 615                 620
Ala Met Arg Ser Val Thr Gly Ser Gly Ala Glu Met Lys Ser Met Ala
625                 630                 635                 640
Ser Gly Ser Asp Asn Ala
                645

<210> 11
<211> 52
<212> PRT
<213> Homo sapiens

<220>
<221> VARIANT
<222> (1)...(1)
<223> The L in position 1 can be V or I.

<223> The X at positions 5 and 6 can be any amino acid.

<221> VARIANT
<222> (7)...(7)
<223> The S in position 7 can be T.

<221> VARIANT
<222> (8)...(8)
<223> The C in position 8 can be Y.

<221> VARIANT
<222> (10)...(10)
<223> The F in position 10 can be W.

<223> The X at positions 11-13 can be any amino acid.

<223> The X at position 15 can be as few as 3, up to 4,
      amino acids, and can be any amio acid.

<223> The X at positions 17 and 18 can be any amino
      acid.

<221> VARIANT
<222> (20)...(20)
<223> The L in position 20 can be I.

<223> The X at positions 21-25 can be any amino acids.

<221> VARIANT
<222> (26)...(26)
<223> The I at position 26 can be R

<223> The X at positions 27-29 can be any amino acids.

<223> The X at position 32 can be any amino acid.

<223> The X at positions 34-37 can be any amino acid.

<223> The X at positions 39-40 can be any amino acid.

<221> VARIANT
<222> (41)...(41)
<223> The L at position 41 can be I.

<221> VARIANT
<222> (42)...(42)
<223> The L at position 42 can be I.

<223> The X at positions 43-46 can be any amino acid.

<221> VARIANT
<222> (47)...(47)
<223> The I at position 47 can be M.

<223> The X at positions 48-49 can be any amino acid.

<221> VARIANT
<222> (50)...(50)
<223> The V at position 50 can be M or I.

<221> VARIANT
<222> (51)...(51)
<223> The V at position 51 can be I.

<221> VARIANT
<222> (52)...(52)
<223> The V at position 52 can be L.

<400> 11
Leu Ala Gly Ala Xaa Xaa Ser Cys Tyr Phe Xaa Xaa Xaa Lys Xaa Pro
 1               5                  10                  15
Xaa Xaa Pro Leu Xaa Xaa Xaa Xaa Xaa Ile Xaa Xaa Xaa Tyr His Xaa
            20                  25                  30
Gly Xaa Xaa Xaa Xaa Gly Xaa Xaa Leu Leu Xaa Xaa Xaa Xaa Ile Xaa
        35                  40                  45
Xaa Val Val Val
    50


<210> 12
<211> 49
<212> PRT
<213> Homo sapiens

<220>
<221> VARIANT
<222> (3)...(3)
<223> The E at position 3 can be R or G.

<223> The X at positions 4-5 can be any amino acid.

<221> VARIANT
<222> (6)...(6)
<223> The H at position 6 can be N.

<223> The amino acid at position 7 can be as few as 11,
      up to 12, amino acids, and can be any amio acid.

<223> The X at position 10 can be any amino acid.

<221> VARIANT
<222> (14)...(14)
<223> The D at position 14 can be E.

<223> The X at positions 15-22 can be any amino acid.

<223> The X at positions 26-28 can be any amino acid.

<221> VARIANT
<222> (29)...(29)
<223> The A at position 29 can be S.

<223> The X at positions 31-34 can be any amino acid.

<223> The X at position 37 can be any amino acid.

<221> VARIANT
<222> (43)...(43)
<223> The F at position 43 can be I.

<223> The X at position 44 can be any amino acid.

<221> VARIANT
<222> (45)...(45)
<223> The I at position 45 can be L.

<223> The X at positions 47-48 can be any amino acid.

<400> 12
Leu Lys Glu Xaa Xaa His Xaa Cys Cys Xaa Trp Cys Leu Asp Xaa Xaa
1               5                   10                  15
Xaa Xaa Xaa Xaa Xaa Xaa Asn Ala Tyr Xaa Xaa Xaa Ala Ile Xaa Xaa
            20                  25                  30
Xaa Xaa Phe Cys Xaa Ser Ala Lys Asp Ala Phe Xaa Ile Leu Xaa Xaa
        35                  40                  45
Asn

## Description

### Cross Reference to Related Applications

This application claims the benefit of U.S. Provisional Application Number 60/267,076, filed February 1, 2001, the contents of which are incorporated herein by this reference.

### Background of the Invention

Choline is an essential nutrient (metabolite) in all cells that humans can synthesize in small amounts, but which also needs to be consumed in the diet for optimal health maintenance. The majority of the body's choline is found in specialized fat molecules called phospholipids, the most common of which is phosphatidylcholine (also known as lecithin). In particular, the phospholipids phosphatidylcholine (lecithin) and sphingomyelin are the major components of, and lend structural integrity to, biomembranes such as cell plasma membranes.

Choline is also implicated in cell signaling, as its metabolites and the compounds it synthesizes can serve as cell signaling molecules. Phosphatidylcholine (lecithin) and sphingomyelin are precursors for the intracellular messenger molecules diaglycerol and ceramide, and choline metabolites sphingophosphorylcholine and platelet activating factor (PAF) are cell signaling molecules as well.

Phosphatidylcholine is important for the metabolism and transport of lipids, since phosphatidylcholine is a required component of very low density lipoproteins (VLDLs), which carry fat and cholesterol from the liver to tissues that require them. This is important not only for metabolism and homeostasis, but also for preventing the harmful accumulation of the same in the liver (e.g., which can contribute to such conditions as "fatty liver"). In fact, studies have shown that liver cells initiate programmed cell death (apoptosis) when deprived of choline (Zeisel et al. (2000) *Nutrition* 16:669-671).

Choline may be oxidized in the body to form a metabolite called betaine, which is a source of methyl (CH₃) required for methylation reactions, and which can be used to convert homocysteine to methionine. This processing is useful because elevated levels of homocysteine in the blood have been associated with increased risk of cardiovascular diseases.

Furthermore, choline is a precursor for acetylcholine, an important neurotransmitter involved in nervous system message transmission (e.g., released at autonomic synapses and neuromuscular junctions), e.g., for muscle control, memory, and other functions. Choline acetyltransferase catalyzes the synthesis of acetylcholine from acetyl coenzyme A produced within nerve endings (e.g., cholinergic nerve endings (those which employ acetylcholine as a neurotransmitter)) and from choline taken up from extracellular fluid (e.g., by choline transporters).

Choline transporters are involved in supplying choline to certain cell types, for such processes as phospholipid synthesis and acetylcholine synthesis, and to maintain homeostatic levels of choline in the body. Choline transporters maintain homeostatic levels of choline in organs such as the brain, to protect them from choline deficiency or excess, and they distribute choline appropriately where needed, as when maternal levels are low during pregnancy and lactation.

Choline transporters also play a role in helping choline be absorbed and disbursed by the body, e.g., after it is consumes as part of one's diet, e.g., by facilitating choline's crossing of the blood-brain barrier into the central nervous system (CNS). Free choline can directly cross the blood-brain barrier by a choline-specific transport system (involving choline transporters). All animal cells absorb free choline as a nutrient (e.g., brought to cells by choline transporters), some of which is incorporated in cell membranes (e.g., via phospholipid synthesis). Uptake by the liver of choline (e.g., by choline transporters) and organic cations is necessary for the hepatic synthesis of phosphatidylcholine (lecithin), and for the metabolism and secretion of endobiotics and drugs.

Also, cholinergic neurons employ a sodium-dependent, voltage dependent, high-affinity choline uptake mechanism to import choline (e.g., by choline transporters) from extracellular fluid, e.g., as part of acetylcholine synthesis. In fact, the choline uptake mechanism is the rate limiting step in acetylcholine synthesis.

In view of the important physiological activities attributable to choline and choline transporters, including the facilitation of membrane synthesis and synaptic message transmission, and further in view of the limited extent to which the key molecular mechanisms of choline transporters have been identified, a need exists for discovery of further members of this protein family.

### Summary of the Invention

The present invention is based, in part, on the discovery of novel genes encoding choline transporters, the genes being referred to herein as "59914 and 59921". The nucleotide sequences of cDNAs encoding 59914 and 59921 are shown in SEQ ID NO:1 and SEQ ID NO:4, respectively, and the amino acid sequences of 59914 and 59921 polypeptides are shown in SEQ ID NO:2 and SEQ ID NO:5, respectively. In addition, the nucleotide sequences of the coding regions of 59914 and 59921 are depicted in SEQ ID NO:3 and SEQ ID NO:6, respectively.

Accordingly, in one aspect, the invention features nucleic acid molecules that encode a 59914 or 59921 protein or polypeptide, e.g., a biologically active portion of the 59914 or 59921 protein. In a preferred embodiment the isolated nucleic acid molecules encode polypeptides having the amino acid sequences SEQ ID NO:2 and SEQ ID NO:5. In other embodiments, the invention provides isolated 59914 and 59921 nucleic acid molecules having the nucleotide sequences of one of SEQ ID NO:1 and SEQ ID NO:4, SEQ ID NO:3 and SEQ ID NO:6.

In still other embodiments, the invention provides nucleic acid molecules that have sequences that are substantially identical (e.g., naturally occurring allelic variants) to the nucleotide sequences of one of SEQ ID NO:1 and SEQ ID NO:4, SEQ ID NO:3 and SEQ ID NO:6, and the deposited nucleotide sequences. In other embodiments, the invention provides nucleic acid molecules which hybridize under stringent hybridization conditions with a nucleic acid molecule having a sequence comprising the nucleotide sequence of one of SEQ ID NO:1 and SEQ ID NO:4, SEQ ID NO:3 and SEQ ID NO:6, and the deposited nucleotide sequences, wherein the nucleic acids encode full length 59914 and 59921 proteins or an active fragment thereof.

In a related aspect, the invention further provides nucleic acid constructs that include 59914 and 59921 nucleic acid molecules described herein. In certain embodiments, the nucleic acid molecules of the invention are operatively linked to native or heterologous regulatory sequences. Also included are vectors and host cells containing the 59914 and 59921 nucleic acid molecules of the invention, e.g., vectors and host cells suitable for producing 59914 and 59921 nucleic acid molecules and polypeptides.

In another related aspect, the invention provides nucleic acid fragments suitable as primers or hybridization probes for detection of 59914 and 59921-encoding nucleic acids.

In still another related aspect, isolated nucleic acid molecules that are antisense to 59914 and 59921-encoding nucleic acid molecules are provided.

In another aspect, the invention features 59914 and 59921 polypeptides, and biologically active or antigenic fragments thereof, that are useful, e.g., as reagents or targets in assays applicable to treatment and diagnosis of 59914 and 59921-mediated or -related disorders. In another embodiment, the invention provides 59914 and 59921 polypeptides having a 59914 and 59921 activity. Preferred polypeptides are 59914 and 59921 proteins including at least one transmembrane domain (and preferably at least 10 transmembrane domains) and at least one conserved choline transporter domain.

In other embodiments, the invention provides 59914 and 59921 polypeptides, e.g., 59914 and 59921 polypeptides having the amino acid sequences shown in SEQ ID NO:2 and SEQ ID NO:5; amino acid sequences that are substantially identical to the amino acid sequences shown in SEQ ID NO:2 and SEQ ID NO:5; or amino acid sequences encoded by nucleic acid molecules having nucleotide sequences which hybridize under stringent hybridization conditions to a nucleic acid molecule comprising the nucleotide sequence of any of SEQ ID NO:1 and SEQ ID NO:4, SEQ ID NO:3 and SEQ ID NO:6, and the deposited nucleotide sequences, wherein the nucleic acids encode full length 59914 and 59921 proteins or an active fragment thereof.

In a related aspect, the invention further provides nucleic acid constructs that include 59914 and 59921 nucleic acid molecules described herein.

In a related aspect, the invention provides 59914 and 59921 polypeptides or fragments operatively linked to non-59914 and 59921 polypeptides to form fusion proteins.

In another aspect, the invention features antibodies and antigen-binding fragments thereof, that react with, or more preferably, specifically or selectively bind, 59914 and 59921 polypeptides.

In another aspect, the invention provides methods of screening for compounds that modulate the expression or activity of the 59914 and 59921 polypeptides or nucleic acids.

In still another aspect, the invention provides a process for modulating 59914 and 59921 polypeptide or nucleic acid expression or activity, e.g., using the compounds identified in the screens described herein. In certain embodiments, the methods involve treatment of conditions related to aberrant activity or expression of the 59914 and 59921 polypeptides or nucleic acids, such as CNS-related disorders, liver disorders, cardiovascular disorders, and metabolic disorders.

The invention also provides assays for determining the activity of or the presence or absence of 59914 and 59921 polypeptides or nucleic acid molecules in a biological sample, including for disease diagnosis.

In further aspect the invention provides assays for determining the presence or absence of a genetic alteration in a 59914 and 59921 polypeptide or nucleic acid molecule, including for disease diagnosis.

Other features and advantages of the invention will be apparent from the following detailed description, and from the claims.

### Brief Description of the Drawings

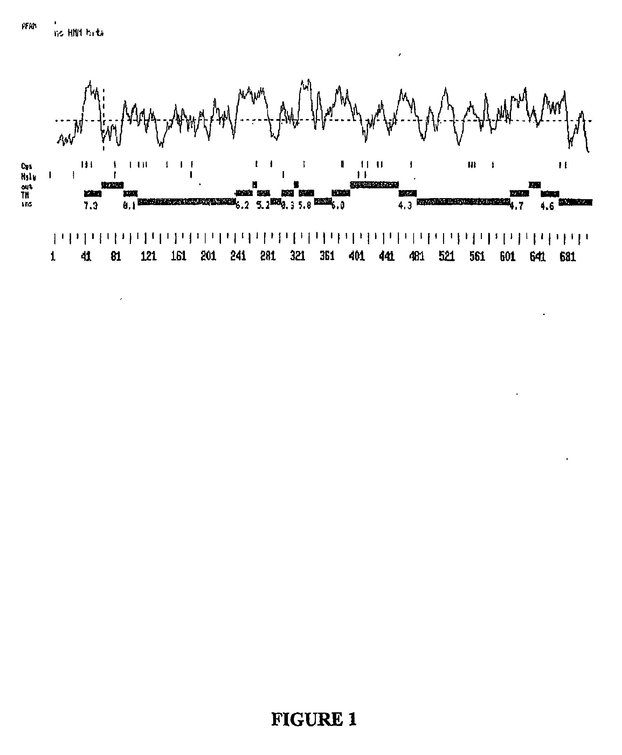

*Figure 1* depicts a hydropathy plot of human 59914. Relatively hydrophobic residues are shown above the dashed horizontal line, and relatively hydrophilic residues are below the dashed horizontal line. The cysteine residues (cys) are indicated by short vertical lines below the hydropathy trace. The numbers corresponding to the amino acid sequence of human 59914 are indicated. Polypeptides of the invention include fragments which include: all or part of a hydrophobic sequence, i.e., a sequence above the dashed line, e.g., the sequences of about residues 39-61, 242-263, and 326-346 of SEQ ID NO:2; all or part of a hydrophilic sequence, i.e., a sequence below the dashed line, e.g., the sequences of residues 62-90, 347-370, and 633-648 of SEQ ID NO:2; a sequence which includes a cysteine residue; or a glycosylation site.

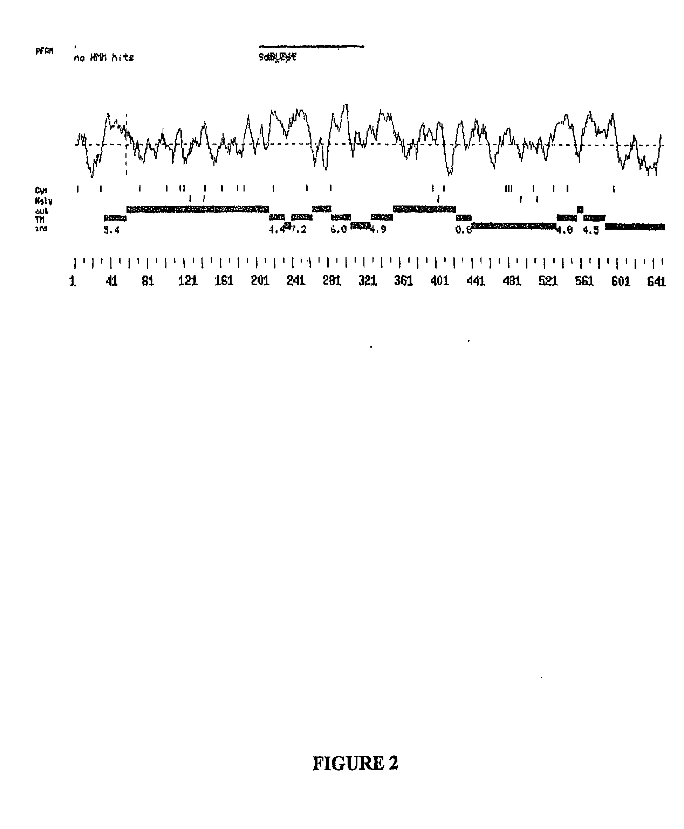

*Figure 2* depicts a hydropathy plot of human 59921. Relatively hydrophobic residues are shown above the dashed horizontal line, and relatively hydrophilic residues are below the dashed horizontal line. The cysteine residues (cys) are indicated by short vertical lines below the hydropathy trace. The numbers corresponding to the amino acid sequence of human 59921 are indicated. Polypeptides of the invention include fragments which include: all or part of a hydrophobic sequence, i.e., a sequence above the dashed line, e.g., the sequences of about residues 33-57, 215-231, and 328-352 of SEQ ID NO:5; all or part of a hydrophilic sequence, i.e., a sequence below the dashed line, e.g., the sequences of residues 58-214, 263-283, and 306-327 of SEQ ID NO:5; a sequence which includes a cysteine residue; or a glycosylation site.

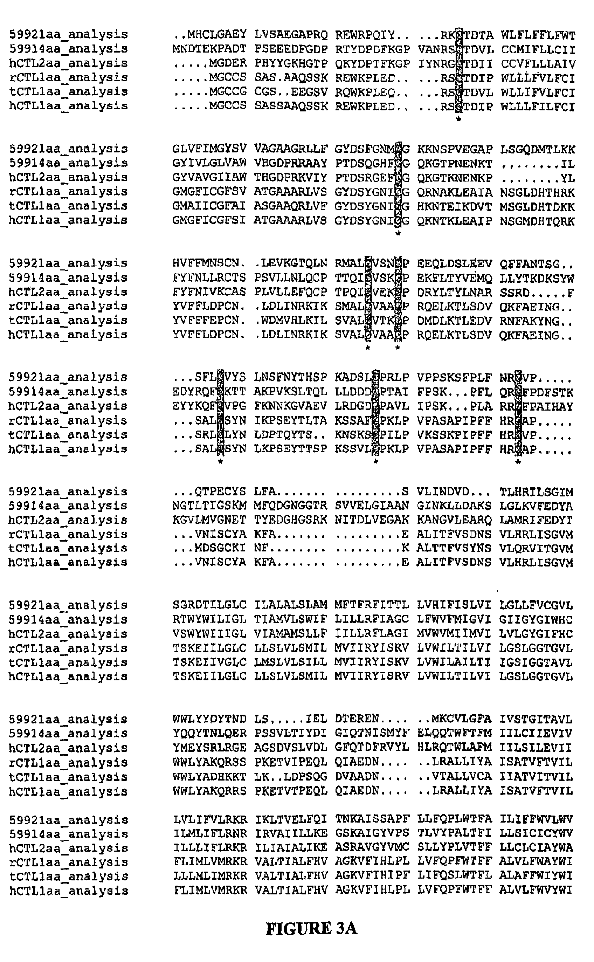

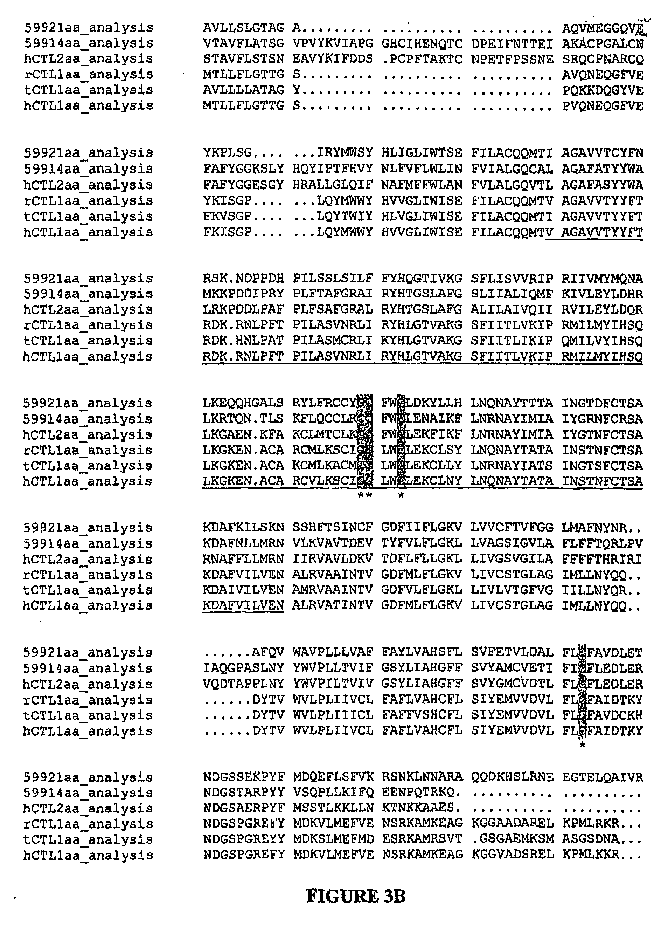

*Figures 3A-3B* depict a multiple alignment between the amino acid sequences of 59914 (SEQ ID NO:2), 59921 (SEQ ID NO:5), hCTL1 (SEQ ID NO:7), hCTL2 (SEQ ID NO:8), rCTL1 (SEQ ID NO:9), and tCTL1 (SEQ ID NO:10), all of which are described herein. The regions which are shaded and marked by an asterisk are conserved cysteine residues, and the underlined region is a consensus sequence shared by all of the aligned sequences and described herein.

### Detailed Description of the Invention

The human 59914 cDNA sequence (SEQ ID NO:1), which is approximately 2473 nucleotide residues long including non-translated regions, contains a methionine-initiated coding sequence (without the 5'- and 3'-non-translated regions) of about 2151 nucleotide residues, excluding termination codon (i.e., nucleotide residues 88-2238 of SEQ ID NO:1; also shown in SEQ ID NO:3). The coding sequence encodes a 717 amino acid protein having the amino acid sequence SEQ ID NO:2.

The human 59921 cDNA sequence (SEQ ID NO:4), which is approximately 2233 nucleotide residues long including non-translated regions, contains a methionine-initiated coding sequence (without the 5'- and 3'-non-translated regions) of about 1959 nucleotide residues, excluding termination codon (i.e., nucleotide residues 110-2068 of SEQ ID NO:4; also shown in SEQ ID NO:6). The coding sequence encodes a 653 amino acid protein having the amino acid sequence SEQ ID NO:5.

Human 59914 and 59921 contain the following regions or other structural features:

a conserved region of sequence which is shared by both 59914 and 59921 proteins, and by other choline transporter (or choline transporter-like) proteins described herein and in O'Regan et al. (2000) *PNAS* 97(4):1835-1840. This region will henceforth be referred to as "conserved choline transporter domain", and is located at about amino acid residues 479-598 of SEQ ID NO:2 and about amino acid residues 402-521 of SEQ ID NO:5;

transmembrane domains at about amino acid residues 39-61, 242-263, 270-287, 326-346, 371-395, 461-484, 514-536, 591-605, 608-632, and 649-672 of SEQ ID NO:2, and at about 33-57, 215-231, 239-262, 284-305, 328-352, 384-411, 436-458, 514-532, 534-555, and 563-586 and SEQ ID NO:5. 59914 and 59921 proteins therefore have about 10 transmembrane domains, as is characteristic of previously characterized choline transporters;

conserved cysteine residues at about amino acid residues 36, 79, 117, 121, 149, 168, 182, 557, 558, 561, and 681 of SEQ ID NO:2 and about amino acid residues 29, 72, 116, 120, 143, 162, 179, 480, 481, 484, and 596 of SEQ ID NO:5, and also found in other choline transporter (and choline transporter-like) proteins described herein and in O'Regan et al. (2000) *PNAS* 97(4):1835-1840. 59914 and 59921 proteins therefore have about 11 conserved cysteines, as is characteristic of previously characterized choline transporters; and

post translational modification sites including: predicted N-glycosylation sites (Pfam accession number PS00001) at about amino acid residues 2-5, 33-36, 88-91, 190-193, 314-317, 416-419, and 425-428 of SEQ ID NO:2 and at about amino acid residues 136-139, 151-154, 412-415, 503-506, and 521-524 of SEQ ID NO:5; predicted protein kinase C phosphorylation sites (Pfam accession number PS00005) at about amino acid residues 4-6, 152-154, 187-189, 587-589, 633-635, 693-695, and 714-716 of SEQ ID NO:2 and at about amino acid residues 90-92, 155-157, 210-212, 232-234, 276-278, 319-321, 510-512, 608-610, 625-627, and 639-641 of SEQ ID NO:5; predicted CAMP- and cGMP-dependent kinase phosphorylation sites (Pfam accession number PS00004) at about amino acid residues 27-30 and 74-77 of SEQ ID NO:5; predicted casein kinase II phosphorylation sites (Pfam accession number PS00006) located at about amino acid residues 10-13, 35-38, 84-87, 127-130, 140-143, 210-213, 305-308, and 587-590 of SEQ ID NO:2 and at about amino acid residues 77-80, 85-88, 127-130, 210-213, 276-279, 414-417, 510-513, 584-587, and 588-591 of SEQ ID NO:5; predicted N-myristoylation sites (Pfam accession number PS00008) at about amino acid residues 204-209, 215-220, 248-253, 285-290, 310-315, 435-440, and 481-486 of SEQ ID NO:2 and at about amino acid residues 69-74, 81-86, 107-112, 139-144, 207-212, 355-360, 386-391, 404-409, 504-509, and 550-555 of SEQ ID NO:5; a predicted tyrosine kinase phosphorylation site (Pfam accession number PS00007) at about amino acid residues 491-499 of SEQ ID NO:2; and a predicted amidation site (Pfam accession number PS00009) at about amino acid residues 72-75 of SEQ ID NO:5.

The 59914 and 59921 proteins contain a number of structural characteristics in common with members of the choline transporter family. The term "family" when referring to the protein and nucleic acid molecules of the invention means two or more proteins or nucleic acid molecules having common structural domains (e.g., 10 transmembrane domains or a conserved choline transporter domain) or motifs and having sufficient amino acid or nucleotide sequence homology as defined herein. Such family members can be naturally or non-naturally occurring and can be from either the same or different species. For example, a family can contain a first protein of human origin as well as other distinct proteins of human origin, or alternatively, can contain homologues of non-human origin, e.g., choline transporter proteins for any species described in the art (e.g., Steiner et al. (1995) *Mol. Microbiol.* 16:825-834, and references cited therein). Members of a family can also have common functional characteristics.

Choline transporter family members all show several transmembrane domains and can reasonably be thought to traverse the membrane about 10 times. In one embodiment, a first, large and variable loop between transmembrane domains 1 and 2 is potentially extracellular and glycosylated. In one embodiment, a highly conserved region covers the last four transmembrane domains and includes the fourth extracellular loop that contains about three conserved cysteines (this area is underlined in the multi sequence alignment depicted in *Figures 3A-3B*). Choline transporter family members generally lack a clear signal peptide and are targeted to the plasma membrane via their transmembrane domains.

59914 and 59921 proteins can include a conserved choline transporter domain. As used herein, a "conserved choline transporter domain" refers to a protein domain having an amino acid sequence of about 50-250 amino acid residues in length, preferably about 75-175 amino acid residues in length, more preferably about 100-150 amino acid residues in length, and most preferably about 119-121 amino acid residues in length; and which has about 1-10 conserved cysteine residues, preferably about 2-8 conserved cysteine residues, and more preferably about 3-7 conserved cysteine residues. The conserved choline transporter domains are underlined in the multi sequence alignment depicted in *Figures 3A-3B*

In one embodiment, the conserved choline transporter domain can have one, preferably both, of the following consensus sequences:

[LVI]-A-G-A-Xaa(2)-[ST]-[CY]-Y-[FW]-Xaa(3)-K-Xaa(n1)-P-
Xaa(2)-P-[LI]-Xaa(5)-[IR]-Xaa(3)-Y-H-Xaa-G-Xaa(4)-G-Xaa(2)-[LI]-
[LI]-Xaa(4)-[IM]-Xaa(2)-[VMI]-[VI]-[VL]     (SEQ ID NO:11)

L-K-[ERG]-Xaa(2)-[HN]-Xaa(n2)-C-C-Xaa-W-C-L-[DE]-Xaa(8)-
N-A-Y-Xaa(3)-[AS]-I-Xaa(4)-F-C-Xaa-S-A-K-D-A-[FI]-Xaa-[IL]-L-
Xaa(2)-N     (SEQ ID NO:12)

In these consensus sequence patterns, each element in the pattern is separated by a dash (-); square [ ] brackets indicate the particular residues that are accepted at that position; Xaa indicates any residue is accepted at that position; repetition of a particular element is indicated by following the element with a numerical value or variable enclosed in parentheses (i.e., above, Xaa(2) indicates 2 residues of any type are repeated, and Xaa(n1) indicates that a range of residues of any type are repeated, as described herein); and the standard IUPAC one-letter code for the amino acids is used. n1 in the first consensus sequence (SEQ ID NO:11) can be 1-8, preferably 2-6, more preferably 3-4, and n2 in the second consensus sequence (SEQ ID NO:12) can be 8-15, preferably 10-13, more preferably 11-12.

These consensus sequences are found from about residues 479-533 and 540-598, of the 59914 protein (of SEQ ID NO:2), respectively; and from about residues 402-455 and 462-521 of the 59921 protein (of SEQ ID NO:5), respectively.

A conserved choline transporter domain is found in at least the following choline transporter (or choline transporter-like (CTL)) proteins: human CTL1 (Genbank accession number CAB75541; SEQ ID NO:7); human CTL2 (Genbank accession number CAB75542; SEQ ID NO:8); rat CTL1 (Genbank accession number CAB75555; SEQ ID NO:9); and torpedo CTL1 (Genbank accession number CAB75556; SEQ ID NO:10). For example, in the human CTL1 protein (Genbank accession number CAB75541; SEQ ID NO:7), the conserved choline transporter domain as described herein is found from about amino acids 407-525 (of SEQ ID NO:7), and the consensus sequences as described herein (SEQ ID NO:11 and SEQ ID NO:12) are found from about amino acids 407-460 and 467-525, respectively (of SEQ ID NO:7).

In one embodiment, the 59914 and 59921 polypeptides or proteins have a conserved choline transporter domain which includes at least about 50-250, preferably about 75-175, more preferably about 100-150, and most preferably about 119-121 amino acid residues in length and has at least about 60%, 70%, 80%, 90%, 95%, 99%, or 100% homology with a conserved choline transporter domain, e.g., the conserved choline transporter domain of human 59914 or 59921 (e.g., residues 479-598 of SEQ ID NO:2 or residues 402-521 of SEQ ID NO:5). In another embodiment, the 59914 and 59921 polypeptides or proteins have a conserved choline transporter domain which includes at least about 50-250, preferably about 75-175, more preferably about 100-150, and most preferably about 119-121 amino acid residues in length; has at least about 60%, 70%, 80%, 90%, 95%, 99%, or 100% homology with a conserved choline transporter domain, e.g., the conserved choline transporter domain of human 59914 or 59921 (e.g., residues 479-598 of SEQ ID NO:2 or residues 402-521 of SEQ ID NO:5); and has about 1-10, preferably about 2-8, and more preferably about 3-7 conserved cysteine residues (e.g., at about positions 553, 554, 557, 558, 561, and 585 of SEQ ID NO:2, and at about positions 409, 477, 478, 480, 481, 484, and 508 of SEQ ID NO:5)

In another embodiment, the 59914 and 59921 polypeptides or proteins have a conserved choline transporter domain which includes at least about 50-250, preferably about 75-175, more preferably about 100-150, and most preferably about 119-121 amino acid residues in length; has at least about 60%, 70%, 80%, 90%, 95%, 99%, or 100% homology with a conserved choline transporter domain, e.g., the conserved choline transporter domain of human 59914 or 59921 (e.g., residues 479-598 of SEQ ID NO:2 or residues 402-521 of SEQ ID NO:5); has about 1-10, preferably about 2-8, and more preferably about 3-7 conserved cysteine residues (e.g., at about positions 553, 554, 557, 558, 561, and 585 of SEQ ID NO:2, and at about positions 409, 477, 478, 480, 481, 484, and 508 of SEQ ID NO:5); and has one or more of the conserved choline transporter domain consensus sequences described herein. In still another embodiment, the 59914 and 59921 polypeptides or proteins have a conserved choline transporter domain which includes at least about 50-250, preferably about 75-175, more preferably about 100-150, and most preferably about 119-121 amino acid residues in length; has at least about 60%, 70%, 80%, 90%, 95%, 99%, or 100% homology with a conserved choline transporter domain, e.g., the conserved choline transporter domain of human 59914 or 59921 (e.g., residues 479-598 of SEQ ID NO:2 or residues 402-521 of SEQ ID NO:5); has about 1-10, preferably about 2-8, and more preferably about 3-7 conserved cysteine residues (e.g., at about positions 553, 554, 557, 558, 561, and 585 of SEQ ID NO:2, and at about positions 409, 477, 478, 480, 481, 484, and 508 of SEQ ID NO:5); has one or more of the conserved choline transporter domain consensus sequences described herein; and has at least one 59914 and 59921 biological activity as described herein

In one embodiment, 59914 and 59921 proteins include at least ten transmembrane domains. As used herein, the term "transmembrane domain" includes an amino acid sequence of about 5 amino acid residues in length that spans the plasma membrane. More preferably, a transmembrane domain includes about at least 10, 15, 20 or 22-25 amino acid residues and spans a membrane. Transmembrane domains are rich in hydrophobic residues, and typically have an alpha-helical structure. In a preferred embodiment, at least 50%, 60%, 70%, 80%, 90%, or 95% or more of the amino acids of a transmembrane domain are hydrophobic, e.g., leucines, isoleucines, tyrosines, or tryptophans. Transmembrane domains are described in, for example, htto://pfam.wustl.edu/cgi-bin/getdesc?name=7tm-1, and Zagotta W.N. et al. (1996) *Annu. Rev. Neurosci.* 19:235-263, the contents of which are incorporated herein by reference. Transmembrane domains exist at least from about amino acid residues 39-61, 242-263, 270-287, 326-346, 371-395, 461-484, 514-536, 591-605, 608-632, and 649-672 of SEQ ID NO:2, and at least from about amino acid residues 33-57, 215-231, 239-262, 284-305, 328-352, 384-411, 436-458, 514-532, 534-555, and 563-586 of SEQ ID NO:5.

A 59914 and 59921 family member can include at least one conserved choline transporter domain. Furthermore, a 59914 and 59921 family member can include at least one, preferably at least 5, more preferably at least 9, and still more preferably 10 transmembrane domains; at least one, preferably 5-7, N-glycosylation sites; at least one, preferably 7-10, protein kinase C phosphorylation sites; at least one, preferably 8-9 casein kinase II phosphorylation sites; and at least one, preferably 7-10 N-myristoylation sites.

59914 and 59921 are homologous to human CTL1 (Genbank accession number CAB75541; SEQ ID NO:7) and human CTL2 (Genbank accession number CAB75542; SEQ ID NO:8), both human choline transporter-like proteins known in the art. An alignment of hCTL1 with the amino acid sequence of 59914 (SEQ ID NO:2) reveals 39.7% identity and 29.4% homology. An alignment of hCTL2 with the amino acid sequence of 59914 (SEQ ID NO:2) reveals 66.6% identity and 54.8% homology. An alignment of hCTL1 with the amino acid sequence of 59921 (SEQ ID NO:5) reveals 57.3% identity and 47.7% homology. An alignment of hCTL2 with the amino acid sequence of 59921 (SEQ ID NO:5) reveals 41.5% identity and 29.6% homology. [The alignments described in this paragraph were performed using the GAP alignment program with a BLOSUM62 scoring matrix, a gap open penalty of 12, and a gap extend penalty of 4.]

Like the 59914 and 59921 proteins, hCTL1 and hCTL2 contain a conserved choline transporter domain (from about amino acid residues 407-525 of SEQ ID NO:7 and about amino acid residues 468-587 of SEQ ID NO:8, respectively); 10 transmembrane domains, as described in O'Regan, *supra*; and 10 conserved cysteines, as described in O'Regan, *supra*, and as seen in the multi sequence alignment in *Figures 3A-3B*.

59914 and 59921 are also homologous to rat and torpedo (marbled electric ray) choline transporter like proteins (rCTL1 (Genbank accession number CAB75555; SEQ ID NO:9) and tCTL1 (Genbank accession number CAB75556; SEQ ID NO:10), respectively). rCTL1 and tCTL1 are described in O'Regan, *supra*, and were discovered in the context of suppressing a yeast choline transport mutation (the addition of tCTL1 to yeast increased high-affinity choline uptake in mutant yeast). An alignment of rCTL1 with the amino acid sequence of 59914 (SEQ ID NO:2) reveals 39.5% identity and 29.2% homology. An alignment of tCTL1 with the amino acid sequence of 59914 (SEQ ID NO:2) reveals 40.2% identity and 29.3% homology. An alignment of rCTL1 with the amino acid sequence of 59921 (SEQ ID NO:5) reveals 57.5% identity and 47.4% homology. An alignment of tCTL1 with the amino acid sequence of 59921 (SEQ ID NO:5) reveals 56.3% identity and 45.2% homology. [The alignments described in this paragraph were performed using the GAP alignment program with a BLOSUM62 scoring matrix, a gap open penalty of 12, and a gap extend penalty of 4.]

Like the 59914 and 59921 proteins, rCTL1 and tCTL1 contain a conserved choline transporter domain (from about amino acid residues 406-524 of SEQ ID NO:9 and about amino acid residues 399-518 of SEQ ID NO:10, respectively); 10 transmembrane domains, as described in O'Regan, *supra;* and 10 conserved cysteines, as described in O'Regan, *supra,* and as seen in the multi sequence alignment in *Figures 3A-3B*.

Based on the above described sequence similarities, the 59914 and 59921 molecules of the present invention belong to the choline transporter family (as described herein). Consequently, the 59914 and 59921 molecules of the invention have similar biological activities as choline transporter family members, and are useful in treating the same disorders as choline transporter family members.

Based on sequence similarities of 59914 and 59921 to sequences of known expression pattern, 59914 and 59921 molecules of the invention can exhibit similar expression patterns, and therefore can be useful in treating disorders associated with tissues in which they are expressed.

Because the 59914 and 59921 polypeptides of the invention can modulate 59914 and 59921-mediated activities, they can be used as novel diagnostic and therapeutic agents or used to develop novel diagnostic and therapeutic agents for 59914 and 59921-mediated or related disorders (e.g., disorders associated with choline transporter family members), as described below.

As used herein, a "59914 and 59921 activity", "biological activity of 59914 and 59921", or "functional activity of 59914 and 59921", refers to an activity of a choline transporter family member, and refers to an activity exerted by 59914 and 59921 proteins, polypeptides or nucleic acid molecules on, for example, 59914 and 59921-responsive cells or on 59914 and 59921 substrates (e.g., protein substrates) as determined *in vivo* or *in vitro*. In one embodiment, a 59914 and 59921 activity is a direct activity, such as association with 59914 and 59921 target molecules. "Target molecules" or "binding partners" of 59914 and 59921 proteins are molecules with which the 59914 and 59921 proteins bind or interact in nature. In an exemplary embodiment, such target molecules include choline, its metabolites, and/or compounds of which choline is a component or precursor, e.g., which 59914 and 59921 proteins can transport into cells from the extracellular fluid, e.g., for plasma membrane synthesis.

A 59914 and 59921 activity can also be an indirect activity, such as an activity mediated by interaction of the 59914 and 59921 protein with a 59914 and 59921 target molecule such that the target molecule modulates a downstream cellular activity, e.g., a cellular signaling activity modulated indirectly by interaction of the 59914 and 59921 protein with a 59914 and 59921 target molecule (e.g., choline, its metabolites, and/or compounds of which choline is a component or precursor).

For example, the 59914 and 59921 proteins of the present invention can have one or more of the following activities: (1) the ability to modulate (e.g., promote, catalyze, regulate, initiate, facilitate or inhibit) the manufacture of choline metabolites and/or compounds of which choline is a component or precursor, e.g., phospholipids (e.g., phosphatidylcholine (lecithin), sphingomyelin, sphingophosphorylcholine, and platelet activating factor), acetylcholine, very low density lipoproteins (VLDLs), and betaine, e.g., by transporting choline into or out of cells; (2) the ability to modulate (e.g., promote, catalyze, regulate, initiate, facilitate or inhibit) transport of choline, its metabolites, and/or compounds of which choline is a component or precursor across membranes (e.g., plasma membranes), e.g., from an extracellular medium into a cell, or vice versa; (3) the ability to modulate (e.g., promote, catalyze, regulate, initiate, facilitate or inhibit) transport of choline, its metabolites, and/or compounds of which choline is a component or precursor across barriers between tissues (e.g., the blood-brain barrier).

Other activities of the 59914 and 59921 proteins of the present invention include one or more of the following: (1) the ability to modulate (e.g., promote, regulate, initiate, facilitate or inhibit) the synthesis of, and the structural maintenance and reinforcement of, cellular components (e.g., membranes (e.g., plasma membranes) and microsomes); (2) the ability to modulate (e.g., promote, regulate, initiate, facilitate or inhibit) cellular nutrition; (3) the ability to modulate (e.g., promote, regulate, initiate, facilitate or inhibit) muscle control; (4) the ability to modulate (e.g., promote, regulate, initiate, facilitate or inhibit) memory; and (5) the ability to modulate (e.g., promote, regulate, initiate, facilitate or inhibit) message transmission (e.g., nervous system message transmission).

Still other activities of the 59914 and 59921 proteins of the present invention include one or more of the following: (1) the ability to modulate (e.g., promote, regulate, initiate, facilitate or inhibit) liver homeostasis, e.g., by transporting fat and/or cholesterol from the liver, e.g., by modulating the transport of choline, its metabolites, and/or compounds of which choline is a component or precursor (e.g., VLDLs); and (2) the ability to modulate (e.g., promote, regulate, initiate, facilitate or inhibit) cellular signaling, e.g., by modulating the transport of choline, its metabolites, and/or compounds of which choline is a component or precursor (e.g., sphingophosphorylcholine and platelet activating factor).

Other activities of the 59914 and 59921 proteins of the present invention include one or more of the following: (1) the ability to modulate (e.g., promote, regulate, initiate, facilitate or inhibit) liver disorders (e.g., hepatocyte apoptosis and others described herein), e.g., by maintaining proper choline levels (e.g., by preventing choline deficiency), e.g., by modulating the transport of choline, its metabolites, and/or compounds of which choline is a component or precursor; (2) the ability to modulate (e.g., promote, regulate, initiate, facilitate or inhibit) central nervous system (CNS) disorders (e.g., hepatocyte apoptosis, and others described herein), e.g., by maintaining proper choline levels (e.g., by preventing choline excess), e.g., by modulating the transport of choline, its metabolites, and/or compounds of which choline is a component or precursor; and (3) the ability to modulate (e.g., promote, regulate, initiate, facilitate or inhibit) cardiovascular disorders, e.g., by preventing buildup of homocysteines in the blood (e.g., by converting them to methionine) e.g., by modulating the transport of choline, its metabolites, and/or compounds of which choline is a component or precursor (e.g., betaine).

Other activities, as described below, include the ability to modulate function, survival, morphology, proliferation and/or differentiation of cells of tissues in which 59914 and 59921 molecules are expressed. Thus, the 59914 and 59921 molecules can act as novel diagnostic targets and therapeutic agents for controlling disorders involving aberrant activities of these cells. 59914 and 59921 molecules described herein can act as novel diagnostic targets and therapeutic agents for prognosticating, diagnosing, preventing, inhibiting, alleviating, or curing choline transporter-related disorders. As the 59914 and 59921 molecules of the invention can modulate choline transporter activities, they are useful for developing novel diagnostic and therapeutic agents for 59914 and 59921-mediated or related disorders, as described herein.

As used herein, a "choline transporter disorder" includes a disorder, disease or condition which is caused by, characterized by, or associated with a misregulation (e.g., an aberrant downregulation or upregulation) of an choline transporter activity or an abnormal choline transporter activity. Choline transporter disorders can detrimentally affect cellular functions such as amino acid nutrition, cellular regulation of homeostasis, membrane structural integrity, and inter- or intra-cellular communication.

Accordingly, the 59914 and 59921 molecules of the invention, as choline transporters, can mediate, and can act as novel diagnostic targets and therapeutic agents for controlling, one or more choline transporter-associated disorders, including CNS-related (e.g., neurological) disorders; liver-related (i.e., hepatic) disorders; skeletal muscle-related disorders; lung-related (i.e., pulmonary) disorders, prostate-related disorders, kidney-related (i.e., renal) disorders, pancreas-related disorders, colon-related disorders, cellular proliferative and/or differentiative disorders; hormonal disorders; immune and inflammatory disorders; cardiovascular disorders; blood vessel disorders; and platelet disorders.

Further, polymorphisms associated with particular 59914 and 59921 alleles, such as those associated with risk of choline transporter-associated disorders, can be used as markers to diagnose abnormal function of tissues and/or cells in which 59914 and 59921 are expressed (described herein), and therefore can be used as markers for disorders associated with such tissues. For example, abnormal and/or aberrant 59914 and 59921 expression (*e.g.*, expression of 59914 and 59921 in cells, such as tumor cells, that do not normally express them, or increased expression of 59914 and 59921 in cells that do normally express them) can be used as a marker for the progression, migration and metastasis of cancerous cells. In particular, abnormal and/or aberrant 59914 and 59921 expression can be used as a marker for the progression, migration and metastasis of cancers of the tissues and/or cells in which 59914 and 59921 are expressed (described herein).

Additional choline transporter disorders include CNS-related (e.g., neurological) disorders. Neurological disorders which can be treated or diagnosed by methods described herein include, but are not limited to, disorders involving neurons, and disorders involving glia, such as astrocytes, oligodendrocytes, ependymal cells, and microglia; cerebral edema, raised intracranial pressure and herniation, and hydrocephalus; malformations and developmental diseases, such as neural tube defects, forebrain anomalies, posterior fossa anomalies, and syringomyelia and hydromyelia; perinatal brain injury; cerebrovascular diseases, such as those related to hypoxia, ischemia, and infarction, including hypotension, hypoperfusion, and low-flow states--global cerebral ischemia and focal cerebral ischemia--infarction from obstruction of local blood supply, intracranial hemorrhage, including intracerebral (intraparenchymal) hemorrhage, subarachnoid hemorrhage and ruptured berry aneurysms, and vascular malformations, hypertensive cerebrovascular disease, including lacunar infarcts, slit hemorrhages, and hypertensive encephalopathy; infections, such as acute meningitis, including acute pyogenic (bacterial) meningitis and acute aseptic (viral) meningitis, acute focal suppurative infections, including brain abscess, subdural empyema, and extradural abscess, chronic bacterial meningoencephalitis, including tuberculosis and mycobacterioses, neurosyphilis, and neuroborreliosis (Lyme disease), viral meningoencephalitis, including arthropod-borne (Arbo) viral encephalitis, *Herpes simplex* virus Type 1, *Herpes simplex* virus Type 2, *Varicella-zoster* virus (*Herpes zoster*), cytomegalovirus, poliomyelitis, rabies, and human immunodeficiency virus 1, including HIV-1 meningoencephalitis (subacute encephalitis), vacuolar myelopathy, AIDS-associated myopathy, peripheral neuropathy, and AIDS in children, progressive multifocal leukoencephalopathy, subacute sclerosing panencephalitis, fungal meningoencephalitis, other infectious diseases of the nervous system; transmissible spongiform encephalopathies (prion diseases); demyelinating diseases, including multiple sclerosis, multiple sclerosis variants, acute disseminated encephalomyelitis and acute necrotizing hemorrhagic encephalomyelitis, and other diseases with demyelination; degenerative diseases, such as degenerative diseases affecting the cerebral cortex, including Alzheimer's disease and Pick's disease, degenerative diseases of basal ganglia and brain stem, including Parkinsonism, idiopathic Parkinson's disease (paralysis agitans), progressive supranuclear palsy, corticobasal degeneration, multiple system atrophy, including striatonigral degeneration, Shy-Drager syndrome, and olivopontocerebellar atrophy, and Huntington's disease; spinocerebellar degenerations, including spinocerebellar ataxias, including Friedreich ataxia, and ataxia-telanglectasia, degenerative diseases affecting motor neurons, including amyotrophic lateral sclerosis (motor neuron disease), bulbospinal atrophy (Kennedy syndrome), and spinal muscular atrophy; inborn errors of metabolism, such as leukodystrophies, including Krabbe disease, metachromatic leukodystrophy, adrenoleukodystrophy, Pelizaeus-Merzbacher disease, and Canavan disease, mitochondrial encephalomyopathies, including Leigh disease and other mitochondrial encephalomyopathies; toxic and acquired metabolic diseases, including vitamin deficiencies such as thiamine (vitamin B₁) deficiency and vitamin B₁₂ deficiency, neurologic sequelae of metabolic disturbances, including hypoglycemia, hyperglycemia, and hepatic encephatopathy, toxic disorders, including carbon monoxide, methanol, ethanol, and radiation, including combined methotrexate and radiation-induced injury; tumors, such as gliomas, including astrocytoma, including fibrillary (diffuse) astrocytoma and glioblastoma multiforme, pilocytic astrocytoma, pleomorphic xanthoastrocytoma, and brain stem glioma, oligodendroglioma, and ependymoma and related paraventricular mass lesions, neuronal tumors, poorly differentiated neoplasms, including medulloblastoma, other parenchymal tumors, including primary brain lymphoma, germ cell tumors, and pineal parenchymal tumors, meningiomas, metastatic tumors, paraneoplastic syndromes, peripheral nerve sheath tumors, including schwannoma, neurofibroma, and malignant peripheral nerve sheath tumor (malignant schwannoma), and neurocutaneous syndromes (phakomatoses), including neurofibromotosis, including Type 1 neurofibromatosis (NF1) and TYPE 2 neurofibromatosis (NF2), tuberous sclerosis, and Von Hippel-Lindau disease.

Additional choline transporter disorders include hepatic disorders. Hepatic disorders which can be treated or diagnosed by methods described herein include, but are not limited to, disorders associated with an accumulation in the liver of fibrous tissue, such as that resulting from an imbalance between production and degradation of the extracellular matrix accompanied by the collapse and condensation of preexisting fibers. The methods described herein can be used to diagnose or treat hepatocellular necrosis or injury induced by a wide variety of agents including processes which disturb homeostasis, such as an inflammatory process, tissue damage resulting from toxic injury or altered hepatic blood flow, and infections (e.g., bacterial, viral and parasitic). For example, the methods can be used for the early detection of hepatic injury, such as portal hypertension or hepatic fibrosis. In addition, the methods can be employed to detect liver fibrosis attributed to inborn errors of metabolism, for example, fibrosis resulting from a storage disorder such as Gaucher's disease (lipid abnormalities) or a glycogen storage disease, A1-antitrypsin deficiency; a disorder mediating the accumulation (e.g., storage) of an exogenous substance, for example, hemochromatosis (iron-overload syndrome) and copper storage diseases (Wilson's disease), disorders resulting in the accumulation of a toxic metabolite (e.g., tyrosinemia, fructosemia and galactosemia) and peroxisomal disorders (e.g., Zellweger syndrome). Additionally, the methods described herein may be useful for the early detection and treatment of liver injury associated with the administration of various chemicals or drugs, such as for example, methotrexate, isonizaid, oxyphenisatin, methyldopa, chlorpromazine, tolbutamide or alcohol, or which represents a hepatic manifestation of a vascular disorder such as obstruction of either the intrahepatic or extrahepatic bile flow or an alteration in hepatic circulation resulting, for example, from chronic heart failure, veno-occlusive disease, portal vein thrombosis or Budd-Chiari syndrome.

Additional choline transporter disorders include skeletal muscle-related disorders. Skeletal muscle-related disorders which can be treated or diagnosed by methods described herein include, but are not limited to, muscular dystrophy (e.g., duchenne muscular dystrophy, becker muscular dystrophy, emery-dreifuss muscular dystrophy, limb-girdle muscular dystrophy, facioscapulohumeral muscular dystrophy, myotonic dystrophy, oculopharyngeal muscular dystrophy, distal muscular dystrophy, and congenital muscular dystrophy), motor neuron diseases (e.g., amyotrophic lateral sclerosis, infantile progressive spinal muscular atrophy, intermediate spinal muscular atrophy, spinal bulbar muscular atrophy, and adult spinal muscular atrophy), myopathies (e.g., inflammatory myopathies (e.g., dermatomyositis and polymyositis), myotonia congenita, paramyotonia congenita, central core disease, nemaline myopathy, myotubular myopathy, and periodic paralysis), and metabolic diseases of muscle (e.g., phosphorylase deficiency, acid maltase deficiency, phosphofructokinase deficiency, debrancher enzyme deficiency, mitochondrial myopathy, carnitine deficiency, carnitine palmityl transferase deficiency, phosphoglycerate kinase deficiency, phosphoglycerate mutase deficiency, lactate dehydrogenase deficiency, and myoadenylate deaminase deficiency).

Additional choline transporter disorders include pulmonary (lung) disorders, which include, but are not limited to, atelectasis, cystic fibrosis, rheumatoid lung disease, pulmonary congestion or edema, chronic obstructive airway disease (e.g., emphysema, chronic bronchitis, bronchial asthma, and bronchiectasis), diffuse interstitial diseases (e.g., sarcoidosis, pneumoconiosis, hypersensitivity pneumonitis, bronchiolitis, Goodpasture's syndrome, idiopathic pulmonary fibrosis, idiopathic pulmonary hemosiderosis, pulmonary alveolar proteinosis, desquamative interstitial pneumonitis, chronic interstitial pneumonia, fibrosing alveolitis, hamman-rich syndrome, pulmonary eosinophilia, diffuse interstitial fibrosis, Wegener's granulomatosis, lymphomatoid granulomatosis, and lipid pneumonia), or tumors (e.g., bronchogenic carcinoma, bronchiolveolar carcinoma, bronchial carcinoid, hamartoma, and mesenchymal tumors).

Additional choline transporter disorders include prostate disorders, which include, but are not limited to, inflammatory diseases (e.g., acute and chronic prostatitis and granulomatous prostatitis), hyperplasia (e.g., benign prostatic hypertrophy or hyperplasia), and tumors (e.g., carcinomas).

Additional choline transporter disorders include renal (kidney) disorders, which include, but are not limited to, glomerular diseases (e.g., acute and chronic glomerulonephritis, rapidly progressive glomerulonephritis, nephrotic syndrome, focal proliferative glomerulonephritis, glomerular lesions associated with systemic disease, such as systemic lupus erythematosus, Goodpasture's syndrome, multiple myeloma, diabetes, polycystic kidney disease, neoplasia, sickle cell disease, and chronic inflammatory diseases), tubular diseases (e.g., acute tubular necrosis and acute renal failure, polycystic renal diseasemedullary sponge kidney, medullary cystic disease, nephrogenic diabetes, and renal tubular acidosis), tubulointerstitial diseases (e.g., pyelonephritis, drug and toxin induced tubulointerstitial nephritis, hypercalcemic nephropathy, and hypokalemic nephropathy) acute and rapidly progressive renal failure, chronic renal failure, nephrolithiasis, gout, vascular diseases (e.g., hypertension and nephrosclerosis, microangiopathic hemolytic anemia, atheroembolic renal disease, diffuse cortical necrosis, and renal infarcts), or tumors (e.g., renal cell carcinoma and nephroblastoma).

Additional choline transporter disorders include pancreatic disorders, which include, but are not limited to, pancreatitis (e.g., acute hemorrhagic pancreatitis and chronic pancreatitis), pancreatic cysts (e.g., congenital cysts, pseudocysts, and benign or malignant neoplastic cysts), pancreatic tumors (e.g., pancreatic carcinoma and adenoma), diabetes mellitus (e.g., insulin- and non-insulin-dependent types, impaired glucose tolerance, and gestational diabetes), or islet cell tumors (e.g., insulinomas, adenomas, Zollinger-Ellison syndrome, glucagonomas, and somatostatinoma).

Additional choline transporter disorders include colonic disorders, which include, but are not limited to, congenital anomalies (e.g., megacolon and imperforate anus), idiopathic disorders (e.g., diverticular disease and melanosis coli), vascular lesions (e.g., ischemic colistis, hemorrhoids, angiodysplasia), inflammatory diseases (e.g., colitis (e.g., idiopathic ulcerative colitis, pseudomembranous colitis), and lymphopathia venereum), Crohn's disease, and tumors (e.g., hyperplastic polyps, adenomatous polyps, bronchogenic cancer, colonic carcinoma, squamous cell carcinoma, adenoacanthomas, sarcomas, lymphomas, argentaffinomas, carcinoids, and melanocarcinomas).

Examples of cellular proliferative and/or differentiative disorders include cancer, e.g., carcinoma, sarcoma, metastatic disorders or hematopoietic neoplastic disorders, e.g., leukemias. A metastatic tumor can arise from a multitude of primary tumor types, including but not limited to those of prostate, colon, lung, breast and liver origin.

As used herein, the term "cancer" (also used interchangeably with the terms, "hyperproliferative" and "neoplastic") refers to cells having the capacity for autonomous growth, i.e., an abnormal state or condition characterized by rapidly proliferating cell growth. Cancerous disease states may be categorized as pathologic, i.e., characterizing or constituting a disease state, e.g., malignant tumor growth, or may be categorized as non-pathologic, i.e., a deviation from normal but not associated with a disease state, e.g., cell proliferation associated with wound repair. The term is meant to include all types of cancerous growths or oncogenic processes, metastatic tissues or malignantly transformed cells, tissues, or organs, irrespective of histopathologic type or stage of invasiveness. The term "cancer" includes malignancies of the various organ systems, such as those affecting lung, breast, thyroid, lymphoid, gastrointestinal, and genito-urinary tract, as well as adenocarcinomas which include malignancies such as most colon cancers, renal-cell carcinoma, prostate cancer and/or testicular tumors, non-small cell carcinoma of the lung, cancer of the small intestine and cancer of the esophagus. The term "carcinoma" is art recognized and refers to malignancies of epithelial or endocrine tissues including respiratory system carcinomas, gastrointestinal system carcinomas, genitourinary system carcinomas, testicular carcinomas, breast carcinomas, prostatic carcinomas, endocrine system carcinomas, and melanomas. Exemplary carcinomas include those forming from tissue of the cervix, lung, prostate, breast, head and neck, colon and ovary. The term "carcinoma" also includes carcinosarcomas, e.g., which include malignant tumors composed of carcinomatous and sarcomatous tissues. An "adenocarcinoma" refers to a carcinoma derived from glandular tissue or in which the tumor cells form recognizable glandular structures. The term "sarcoma" is art recognized and refers to malignant tumors of mesenchymal derivation.

The 59914 and 59921 molecules of the invention can be used to monitor, treat and/or diagnose a variety of proliferative disorders. Such disorders include hematopoietic neoplastic disorders. As used herein, the term "hematopoietic neoplastic disorders" includes diseases involving hyperplastic/neoplastic cells of hematopoietic origin, *e.g.*, arising from myeloid, lymphoid or erythroid lineages, or precursor cells thereof. Typically, the diseases arise from poorly differentiated acute leukemias, *e.g.*, erythroblastic leukemia and acute megakaryoblastic leukemia. Additional exemplary myeloid disorders include, but are not limited to, acute promyeloid leukemia (APML), acute myelogenous leukemia (AML) and chronic myelogenous leukemia (CML) (reviewed in Vaickus, L., (1991) *Crit. Rev. in Oncol.*/*Hemotol.* 11:267-97); lymphoid malignancies include, but are not limited to acute lymphoblastic leukemia (ALL) which includes B-lineage ALL and T-lineage ALL, chronic lymphocytic leukemia (CLL), prolymphocytic leukemia (PLL), hairy cell leukemia (HLL) and Waldenstrom's macroglobulinemia (WM). Additional forms of malignant lymphomas include, but are not limited to non-Hodgkin lymphoma and variants thereof, peripheral T cell lymphomas, adult T cell leukemia/lymphoma (ATL), cutaneous T-cell lymphoma (CTCL), large granular lymphocytic leukemia (LGF), Hodgkin's disease and Reed-Sternberg disease.

Choline transporter disorders can include hormonal disorders, such as conditions or diseases in which the production and/or regulation of hormones in an organism is aberrant. Examples of such disorders and diseases include type I and type II diabetes mellitus, pituitary disorders (*e.g.*, growth disorders), thyroid disorders (*e.g.*, hypothyroidism or hyperthyroidism), and reproductive or fertility disorders (*e.g.*, disorders which affect the organs of the reproductive system, *e.g.*, the prostate gland, the uterus, or the vagina; disorders which involve an imbalance in the levels of a reproductive hormone in a subject; disorders affecting the ability of a subject to reproduce; and disorders affecting secondary sex characteristic development, *e.g.*, adrenal hyperplasia).

Choline transporter disorders also include immune disorders, such as autoimmune disorders or immune deficiency disorders, *e.g.*, congenital X-linked infantile hypogammaglobulinemia, transient hypogammaglobulinemia, common variable immunodeficiency, selective IgA deficiency, chronic mucocutaneous candidiasis, or severe combined immunodeficiency. Other examples of disorders include autoimmune diseases (including, for example, diabetes mellitus, arthritis (including rheumatoid arthritis, juvenile rheumatoid arthritis, osteoarthritis, psoriatic arthritis), multiple sclerosis, encephalomyelitis, myasthenia gravis, systemic lupus erythematosis, autoimmune thyroiditis, dermatitis (including atopic dermatitis and eczematous dermatitis), psoriasis, Sjögren's Syndrome, sepsis, acne, inflammatory bowel disease (e.g., Crohn's disease and ulcerative colitis), aphthous ulcer, iritis, conjunctivitis, keratoconjunctivitis, respiratory inflammation (e.g., asthma, allergic asthma, and chronic obstructive pulmonary disease), cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves' disease, sarcoidosis, primary biliary cirrhosis, uveitis posterior, and interstitial lung fibrosis), graft-versus-host disease, cases of transplantation, and allergy such as, atopic allergy.

Cardiovascular disorders include, but are not limited to, heart failure, including but not limited to, cardiac hypertrophy, left-sided heart failure, and right-sided heart failure; ischemic heart disease, including but not limited to angina pectoris, myocardial infarction, chronic ischemic heart disease, and sudden cardiac death; hypertensive heart disease, including but not limited to, systemic (left-sided) hypertensive heart disease and pulmonary (right-sided) hypertensive heart disease; valvular heart disease, including but not limited to, valvular degeneration caused by calcification, such as calcification of a congenitally bicuspid aortic valve, and mitral annular calcification, and myxomatous degeneration of the mitral valve (mitral valve prolapse), rheumatic fever and rheumatic heart disease, infective endocarditis, and noninfected vegetations, such as nonbacterial thrombotic endocarditis and endocarditis of systemic lupus erythematosus (Libman-Sacks disease), carcinoid heart disease, and complications of artificial valves; myocardial disease, including but not limited to dilated cardiomyopathy, hypertrophic cardiomyopathy, restrictive cardiomyopathy, and myocarditis; pericardial disease, including but not limited to, pericardial effusion and hemopericardium and pericarditis, including acute pericarditis and healed pericarditis, and rheumatoid heart disease; neoplastic heart disease, including but not limited to, primary cardiac tumors, such as myxoma, lipoma, papillary fibroelastoma, rhabdomyoma, and sarcoma, and cardiac effects of noncardiac neoplasms; congenital heart disease, including but not limited to, left-to-right shunts--late cyanosis, such as atrial septal defect, ventricular septal defect, patent ductus arteriosus, and atrioventricular septal defect, right-to-left shunts--early cyanosis, such as tetralogy of fallot, transposition of great arteries, truncus arteriosus, tricuspid atresia, and total anomalous pulmonary venous connection, obstructive congenital anomalies, such as coarctation of aorta, pulmonary stenosis and atresia, and aortic stenosis and atresia, disorders involving cardiac transplantation, and congestive heart failure.

Disorders involving blood vessels include, but are not limited to, responses of vascular cell walls to injury, such as endothelial dysfunction and endothelial activation and intimal thickening; vascular diseases including, but not limited to, congenital anomalies, such as arteriovenous fistula, atherosclerosis, and hypertensive vascular disease, such as hypertension; inflammatory disease--the vasculitides, such as giant cell (temporal) arteritis, Takayasu arteritis, polyarteritis nodosa (classic), Kawasaki syndrome (mucocutaneous lymph node syndrome), microscopic polyanglitis (microscopic polyarteritis, hypersensitivity or leukocytoclastic anglitis), Wegener granulomatosis, thromboanglitis obliterans (Buerger disease), vasculitis associated with other disorders, and infectious arteritis; Raynaud disease; aneurysms and dissection, such as abdominal aortic aneurysms, syphilitic (luetic) aneurysms, and aortic dissection (dissecting hematoma); disorders of veins and lymphatics, such as varicose veins, thrombophlebitis and phlebothrombosis, obstruction of superior vena cava (superior vena cava syndrome), obstruction of inferior vena cava (inferior vena cava syndrome), and lymphangitis and lymphedema; tumors, including benign tumors and tumor-like conditions, such as hemangioma, lymphangioma, glomus tumor (glomangioma), vascular ectasias, and bacillary angiomatosis, and intermediate-grade (borderline low-grade malignant) tumors, such as Kaposi's sarcoma and hemangloendothelioma, and malignant tumors, such as angiosarcoma and hemangiopericytoma; and pathology of therapeutic interventions in vascular disease, such as balloon angioplasty and related techniques and vascular replacement, such as coronary artery bypass graft surgery.

Blood platelet disorders include, but are not limited to, thrombocytopenia due to a reduced number of megakaryocytes in the bone marrow, for example, as a result of chemotherapy; invasive disorders, such as leukemia, idiopathic or drug- or toxin-induced aplasia of the marrow, or rare hereditary amegakaryocytic thrombocytopenias; ineffective thrombopoiesis, for example, as a result of megaloblastic anemia, alcohol toxicity, vitamin B12 or folate deficiency, myelodysplastic disorders, or rare hereditary disorders (e.g., Wiskott-Aldrich syndrome and May-hegglin anomaly); a reduction in platelet distribution, for example, as a result of cirrhosis, a splenic invasive disease (e.g., Gaucher's disease), or myelofibrosis with extramedullary myeloid metaplasia; increased platelet destruction, for example, as a result of removal of IgG-coated platelets by the mononuclear phagocytic system (e.g., idiopathic thrombocytopenic purpura (ITP), secondary immune thrombocytopenia (e.g., systemic lupus erythematosus, lymphoma, or chronic lymphocytic leukemia), drug-related immune thrombocytopenias (e.g., as with quinidine, aspirin, and heparin), post-transfusion purpura, and neonatal thrombocytopenia as a result of maternal platelet autoantibodies or maternal platelet alloantibodies). Also included are thrombocytopenia secondary to intravascular clotting and thrombin induced damage to platelets as a result of, for example, obstetric complications, metastatic tumors, severe gram-negative bacteremia, thrombotic thrombocytopenic purpura, or severe illness. Also included is dilutional thrombocytopenia, for example, due to massive hemorrhage. Blood platelet disorders also include, but are not limited to, essential thrombocytosis and thrombocytosis associated with, for example, splenectomy, acute or chronic inflammatory diseases, hemolytic anemia, carcinoma, Hodgkin's disease, lymphoproliferative disorders, and malignant lymphomas.

The 59914 and 59921 proteins, fragments thereof, and derivatives and other variants of the sequences in SEQ ID NO:2 and SEQ ID NO:5 thereof are collectively referred to as "polypeptides or proteins of the invention" or "59914 and 59921 polypeptides or proteins". Nucleic acid molecules encoding such polypeptides or proteins are collectively referred to as "nucleic acids of the invention" or "59914 and 59921 nucleic acids." 59914 and 59921 molecules refer to 59914 and 59921 nucleic acids, polypeptides, antibodies, as well as modulators and variants thereof.

As used herein, the term "nucleic acid molecule" includes DNA molecules (e.g., a cDNA or genomic DNA) and RNA molecules (e.g., an mRNA) and analogs of the DNA or RNA generated, e.g., by the use of nucleotide analogs. The nucleic acid molecule can be single-stranded or double-stranded, but preferably is double-stranded DNA.

The term "isolated or purified nucleic acid molecule" includes nucleic acid molecules that are separated from other nucleic acid molecules that are present in the natural source of the nucleic acid. For example, with regards to genomic DNA, the term "isolated" includes nucleic acid molecules that are separated from the chromosome with which the genomic DNA is naturally associated. Preferably, an "isolated" nucleic acid is free of sequences that naturally flank the nucleic acid (i.e., sequences located at the 5'- and/or 3'-ends of the nucleic acid) in the genomic DNA of the organism from which the nucleic acid is derived. For example, in various embodiments, the isolated nucleic acid molecule can contain less than about 5 kilobases, 4 kilobases, 3 kilobases, 2 kilobases, 1 kilobase, 0.5 kilobase or 0.1 kilobase of 5'- and/or 3'-nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

As used herein, the term "hybridizes under stringent conditions" describes conditions for hybridization and washing. Stringent conditions are known to those skilled in the art and can be found in available references (e.g., Current Protocols in Molecular Biology, John Wiley & Sons, N.Y., 1989, 6.3.1-6.3.6). Aqueous and non-aqueous methods are described in that reference and either can be used. A preferred example of stringent hybridization conditions are hybridization in 6× sodium chloride/sodium citrate (SSC) at about 45°C, followed by one or more washes in 0.2× SSC, 0.1% (w/v) SDS at 50°C. Another example of stringent hybridization conditions are hybridization in 6× SSC at about 45°C, followed by one or more washes in 0.2× SSC, 0.1% (w/v) SDS at 55°C. A further example of stringent hybridization conditions are hybridization in 6× SSC at about 45°C, followed by one or more washes in 0.2× SSC, 0.1% (w/v) SDS at 60°C. Preferably, stringent hybridization conditions are hybridization in 6× SSC at about 45°C, followed by one or more washes in 0.2× SSC, 0.1% (w/v) SDS at 65°C. Particularly preferred stringency conditions (and the conditions that should be used if the practitioner is uncertain about what conditions should be applied to determine if a molecule is within a hybridization limitation of the invention) are 0.5 molar sodium phosphate, 7% (w/v) SDS at 65°C, followed by one or more washes at 0.2× SSC, 1% (w/v) SDS at 65°C. Preferably, an isolated nucleic acid molecule of the invention that hybridizes under stringent conditions to the sequence of SEQ ID NO:1 and SEQ ID NO:4 or SEQ ID NO:3 and SEQ ID NO:6, corresponds to a naturally-occurring nucleic acid molecule.

As used herein, a "naturally-occurring" nucleic acid molecule refers to an RNA or DNA molecule having a nucleotide sequence that occurs in nature (e.g., encodes a natural protein).

As used herein, the terms "gene" and "recombinant gene" refer to nucleic acid molecules which include an open reading frame encoding 59914 and 59921 proteins, preferably mammalian 59914 and 59921 proteins, and can further include non-coding regulatory sequences and introns.

An "isolated" or "purified" polypeptide or protein is substantially free of cellular material or other contaminating proteins from the cell or tissue source from which the protein is derived, or substantially free from chemical precursors or other chemicals when chemically synthesized. In one embodiment, the language "substantially free" means preparation of 59914 and 59921 proteins having less than about 30%, 20%, 10% and more preferably 5% (by dry weight), of non-59914 and 59921 proteins (also referred to herein as a "contaminating proteins"), or of chemical precursors or non-59914 and 59921 chemicals. When the 59914 and 59921 proteins or biologically active portions thereof are recombinantly produced, they are also preferably substantially free of culture medium, i.e., culture medium represents less than about 20%, more preferably less than about 10%, and most preferably less than about 5% of the volume of the protein preparation. The invention includes isolated or purified preparations of at least 0.01, 0.1, 1.0, and 10 milligrams in dry weight.

A "non-essential" amino acid residue is a residue that can be altered from the wild-type sequence of 59914 and 59921 (e.g., the sequence of SEQ ID NO:1 and SEQ ID NO:4, SEQ ID NO:3 and SEQ ID NO:6 or the deposited nucleotide sequences) without abolishing or, more preferably, without substantially altering a biological activity, whereas an "essential" amino acid residue results in such a change. For example, amino acid residues that are conserved among the polypeptides of the present invention, e.g., those present in the conserved choline transporter domain are predicted to be particularly non-amenable to alteration, except that amino acid residues in transmembrane domains can generally be replaced by other residues having approximately equivalent hydrophobicity without significantly altering 59914 and 59921 activity.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a nonessential amino acid residue in 59914 and 59921 proteins is preferably replaced with another amino acid residue from the same side chain family. Alternatively, in another embodiment, mutations can be introduced randomly along all or part of 59914 and 59921 coding sequences, such as by saturation mutagenesis, and the resultant mutants can be screened for 59914 and 59921 biological activity to identify mutants that retain activity. Following mutagenesis of SEQ ID NO:1 and SEQ ID NO:4, SEQ ID NO:3 and SEQ ID NO:6, or the deposited nucleotide sequences, the encoded proteins can be expressed recombinantly and the activity of the protein can be determined.

As used herein, a "biologically active portion" of 59914 and 59921 proteins includes fragment of 59914 and 59921 proteins that participate in an interaction between 59914 and 59921 molecules and non-59914 and 59921 molecules. Biologically active portions of 59914 and 59921 proteins include peptides comprising amino acid sequences sufficiently homologous to or derived from the amino acid sequences of the 59914 and 59921 proteins, e.g., the amino acid sequences shown in SEQ ID NO:2 and SEQ ID NO:5, which include fewer amino acids than the full length 59914 and 59921 proteins, and exhibit at least one activity of 59914 and 59921 proteins. Typically, biologically active portions comprise a domain or motif with at least one activity of the 59914 and 59921 proteins, e.g., the ability to modulate (e.g., promote, catalyze, regulate, initiate, facilitate or inhibit) transmembrane transport of amino acids (e.g., amino acids associated with N system transport, e.g., histidine, asparagine, and glutamine) across the plasma membrane, e.g., from an extracellular medium into a cell, or vice versa.

A biologically active portion of 59914 and 59921 proteins can be a polypeptide that is, for example, 100, 200, 300, 400, 500, 600, or 650 or more amino acids in length. Biologically active portions of 59914 and 59921 proteins can be used as targets for developing agents that modulate 59914 and 59921-mediated activities, e.g., biological activities described herein.

Calculations of sequence homology or identity (the terms are used interchangeably herein) between sequences are performed as follows.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 60%, 70%, 80%, 82%, 84%, 85%, 86%, 87%, 88%, 89%, 90%, 91%, 92%, 93%, 94%, 95%, 96%, 97%, 98%, 99%, or 100% of the length of the reference sequence (e.g., when aligning a second sequence to the 59914 amino acid sequences of SEQ ID NO:2 having 717 amino acid residues, at least 358, preferably at least 370, more preferably at least 400, even more preferably at least 450, and even more preferably at least 460, 470, 480, 500, 520, 540, 560, 580, 600, 620, 640, 660, 680, 700, 710, or 717 amino acid residues are aligned; and when aligning a second sequence to the 59921 amino acid sequences of SEQ ID NO:5 having 653 amino acid residues, at least 326, preferably at least 350, more preferably at least 400, even more preferably at least 450, and even more preferably at least 460, 470, 480, 500, 520, 540, 560, 580, 600, 620, 640, or 653 amino acid residues are aligned). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences, taking into account the number of gaps, and the length of each gap, which need to be introduced for optimal alignment of the two sequences.

The comparison of sequences and determination of percent identity between two sequences can be accomplished using a mathematical algorithm. In a preferred embodiment, the percent identity between two amino acid sequences is determined using the Needleman et al. (1970) *J. Mol. Biol*. 48:444-453) algorithm which has been incorporated into the GAP program in the GCG software package (available at http://www.gcg.com), using either a BLOSUM 62 matrix or a PAM250 matrix, and a gap weight of 16, 14, 12, 10, 8, 6, or 4 and a length weight of 1, 2, 3, 4, 5, or 6. In yet another preferred embodiment, the percent identity between two nucleotide sequences is determined using the GAP program in the GCG software package (available at http://www.gcg.com), using a NWSgapdna.CMP matrix and a gap weight of 40, 50, 60, 70, or 80 and a length weight of 1, 2, 3, 4, 5, or 6. A particularly preferred set of parameters (and the one that should be used if the practitioner is uncertain about what parameters should be applied to determine if a molecule is within a sequence identity or homology limitation of the invention) are a BLOSUM 62 scoring matrix with a gap penalty of 12, a gap extend penalty of 4, and a frameshift gap penalty of 5.

The percent identity between two amino acid or nucleotide sequences can be determined using the algorithm of Meyers et al. (1989) *CABIOS* 4:11-17) which has been incorporated into the ALIGN program (version 2.0), using a PAM120 weight residue table, a gap length penalty of 12 and a gap penalty of 4.

The nucleic acid and protein sequences described herein can be used as a "query sequence" to perform a search against public databases to, for example, identify other family members or related sequences. Such searches can be performed using the NBLAST and XBLAST programs (version 2.0) of Altschul, et al. (1990) *J. Mol. Biol*. 215:403-410). BLAST nucleotide searches can be performed with the NBLAST program, score = 100, wordlength = 12 to obtain nucleotide sequences homologous to 59914 and 59921 nucleic acid molecules of the invention. BLAST protein searches can be performed with the XBLAST program, score = 50, wordlength = 3 to obtain amino acid sequences homologous to 59914 and 59921 protein molecules of the invention. To obtain gapped alignments for comparison purposes, gapped BLAST can be utilized as described in Altschul et al. (1997, Nucl. Acids Res. 25:3389-3402). When using BLAST and gapped BLAST programs, the default parameters of the respective programs (e.g., XBLAST and NBLAST) can be used. See <http://www.ncbi.nlm.nih.gov>.

59914 and 59921 polypeptides of the present invention can have amino acid sequences sufficiently or substantially identical to the amino acid sequences of SEQ ID NO:2 and SEQ ID NO:5. The terms "sufficiently identical" or "substantially identical" are used herein to refer to a first amino acid or nucleotide sequence that contains a sufficient or minimum number of identical or equivalent (e.g., with a similar side chain) amino acid residues or nucleotides to a second amino acid or nucleotide sequence such that the first and second amino acid or nucleotide sequences have a common structural domain or common functional activity. For example, amino acid or nucleotide sequences that contain a common structural domain having at least about 60%, or 65% identity, likely 75% identity, more likely 85%, 90%. 91%, 92%, 93%, 94%, 95%, 96%, 97%, 98% or 99% identity are defined herein as sufficiently or substantially identical.

"Misexpression or aberrant expression", as used herein, refers to a non-wild type pattern of gene expression, at the RNA or protein level. It includes: expression at non-wild type levels, i.e., over- or under-expression; a pattern of expression that differs from wild type in terms of the time or stage at which the gene is expressed, e.g., increased or decreased expression (as compared with wild type) at a predetermined developmental period or stage; a pattern of expression that differs from wild type in terms of decreased expression (as compared with wild type) in a predetermined cell type or tissue type; a pattern of expression that differs from wild type in terms of the splicing size, amino acid sequence, post-transitional modification, or biological activity of the expressed polypeptide; a pattern of expression that differs from wild type in terms of the effect of an environmental stimulus or extracellular stimulus on expression of the gene, e.g., a pattern of increased or decreased expression (as compared with wild type) in the presence of an increase or decrease in the strength of the stimulus.

"Subject," as used herein, can refer to a mammal, e.g., a human, or to an experimental animal or disease model. The subject can also be a non-human animal, e.g., a horse, cow, goat, or other domestic animal.

A "purified preparation of cells," as used herein, refers to, in the case of plant or animal cells, an in vitro preparation of cells and not an entire intact plant or animal. In the case of cultured cells or microbial cells, it consists of a preparation of at least 10%, and more preferably, 50% of the subject cells.

Various aspects of the invention are described in further detail below.

### Isolated Nucleic Acid Molecules

In one aspect, the invention provides, an isolated or purified, nucleic acid molecule that encodes 59914 and 59921 polypeptides described herein, e.g., full length 59914 and 59921 proteins or fragments thereof, e.g., biologically active portions of 59914 and 59921 proteins. Also included are nucleic acid fragments suitable for use as hybridization probes, which can be used, e.g., to identify a nucleic acid molecules encoding polypeptide of the inventions, 59914 and 59921 mRNA, and fragments suitable for use as primers, e.g., PCR primers for the amplification or mutation of nucleic acid molecules.

In one embodiment, an isolated nucleic acid molecule of the invention includes the nucleotide sequences shown in SEQ ID NO:1 and SEQ ID NO:4, or portions or fragments thereof. In one embodiment, the nucleic acid molecules include sequences encoding the human 59914 and 59921 proteins (i.e., "the coding region", from nucleotides 88-2238 and 110-2068 of SEQ ID NO:1 and SEQ ID NO:4, respectively, excluding the termination codon, shown as in SEQ ID NO:3 and SEQ ID NO:6), as well as untranslated (e.g., noncoding) sequences, e.g., 5' untranslated sequence (i.e., nucleotides 1-87 and 1-109 of SEQ ID NO:1 and SEQ ID NO:4, respectively) and/or 3' untranslated sequence (i.e., nucleotides 2239-2473 and 2069-2233 of SEQ ID NO:1 and SEQ ID NO:4, respectively). Alternatively, the nucleic acid molecules can include only the coding regions of SEQ ID NO:1 and SEQ ID NO:4 (e.g., nucleotides 1-2151 and 1-1959 of SEQ ID NO:3 and SEQ ID NO:6, respectively) and, e.g., no flanking sequences which normally accompany the subject sequence. In another embodiment, the nucleic acid molecules encode sequences corresponding to the mature proteins of SEQ ID NO:2 and SEQ ID NO:5. In yet another embodiment, the nucleic acid molecules encode sequences corresponding to fragments of the proteins from about amino acid 479-598 or 402-521 (of SEQ ID NO:2 and SEQ ID NO:5, respectively).

In another embodiment, an isolated nucleic acid molecule of the invention includes nucleic acid molecules which are complements of the nucleotide sequences shown in SEQ ID NO:1 and SEQ ID NO:4 or SEQ ID NO:3 and SEQ ID NO:6, or portions or fragments thereof. In other embodiments, the nucleic acid molecules of the invention are sufficiently complementary to the nucleotide sequences shown in SEQ ID NO:1 and SEQ ID NO:4 or SEQ ID NO:3 and SEQ ID NO:6 such that they can hybridize to the nucleotide sequences shown in SEQ ID NO:1 and SEQ ID NO:4 or 3, thereby forming stable duplexes.

In one embodiment, isolated nucleic acid molecules of the present invention include nucleotide sequences which are at least about: 60%, 65%, 70%, 75%, 80%, 85%, 90%, 91%, 92%, 93%, 94%, 95%, 96%, 97%, 98%, 99% or more, homologous to the entire length of the nucleotide sequences shown in SEQ ID NO:1 and SEQ ID NO:4 or SEQ ID NO:3 and SEQ ID NO:6, or portions or fragments thereof, preferably of the same length, of any of these nucleotide sequences.

### 59914 and 59921 Nucleic Acid Fragments

A nucleic acid molecule of the invention can include only a portion or fragment of the nucleic acid sequences of SEQ ID NO:1 or 3 and SEQ ID NO:4 or 6. For example, such a nucleic acid molecule can include fragments which can be used as probes or primers or fragments encoding a portion of 59914 and 59921 proteins, e.g., immunogenic or biologically active portions of 59914 and 59921 proteins. A fragment can comprise those nucleotides of SEQ ID NO:1 and SEQ ID NO:4 which encode a conserved choline transporter domain of human 59914 and 59921. The nucleotide sequences determined from the cloning of the 59914 and 59921 genes allow for the generation of probes and primers designed for use in identifying and/or cloning other 59914 and 59921 family members, or fragments thereof, as well as 59914 and 59921 homologues, or fragments thereof, from other species.

In another embodiment, a nucleic acid includes a nucleotide sequence that includes part, or all, of the coding region and extends into either (or both) the 5' or 3' noncoding or untranslated region. Other embodiments include a fragment which includes a nucleotide sequence encoding an amino acid fragment described herein. Nucleic acid fragments can encode a specific domain or site described herein or fragments thereof, particularly fragments thereof which are at least 75 amino acids in length. Fragments also include nucleic acid sequences corresponding to specific amino acid sequences described above or fragments thereof. Nucleic acid fragments should not to be construed as encompassing those fragments that may have been disclosed prior to the invention.

A nucleic acid fragment can include a sequence corresponding to a domain, region, or functional site described herein. A nucleic acid fragment can also include one or more domain, region, or functional site described herein. Thus, for example, 59914 and 59921 nucleic acid fragments can include sequences corresponding to a conserved choline transporter domain.

59914 and 59921 probes and primers are provided. Typically a probe/primer is an isolated or purified oligonucleotide. The oligonucleotide typically includes a region of nucleotide sequence that hybridizes under stringent conditions to at least about 7, 12 or 15, preferably about 20 or 25, more preferably about 30, 35, 40, 45, 50, 55, 60, 65, or 75 consecutive nucleotides of a sense or antisense sequence of SEQ ID NO:1 and SEQ ID NO:4 or SEQ ID NO:3 and SEQ ID NO:6, or of a naturally occurring allelic variant or mutant of SEQ ID NO:1 and SEQ ID NO:4 or SEQ ID NO:3 and SEQ ID NO:6.

In a preferred embodiment the nucleic acid is a probe which is at least 5 or 10, and less than 200, more preferably less than 100, or less than 50, base pairs in length. It should be identical, or differ by 1, or less than in 5 or 10 bases, from a sequence disclosed herein. If alignment is needed for this comparison the sequences should be aligned for maximum homology. "Looped" out sequences from deletions or insertions, or mismatches, are considered differences.

A probe or primer can be derived from the sense or anti-sense strand of a nucleic acid which encodes a conserved choline transporter domain (e.g., at about nucleotides 1522-1881 of SEQ ID NO:1 and at about nucleotides 1313-1672 of SEQ ID NO:4), or a fragment thereof.

In another embodiment a set of primers is provided, e.g., primers suitable for use in a PCR, which can be used to amplify a selected region of a 59914 and 59921 sequence, e.g., a domain, region, site or other sequence described herein. The primers should be at least 5, 10, or 50 base pairs in length and less than 100, or less than 200, base pairs in length. The primers should be identical, or differs by one base from a sequence disclosed herein or from a naturally occurring variant. For example, primers suitable for amplifying all or a portion of any of the following regions are provided: a conserved choline transporter domain from about amino acids 479-598 of SEQ ID NO:2 and from about amino acids 402-521 of SEQ ID NO:5; or transmembrane domains at about amino acid residues 39-61, 242-263, 270-287, 326-346, 371-395, 461-484, 514-536, 591-605, 608-632, and 649-672 of SEQ ID NO:2, and at about 33-57, 215-231, 239-262, 284-305, 328-352, 384-411, 436-458, 514-532, 534-555, and 563-586 and SEQ ID NO:5.

A nucleic acid fragment can encode an epitope bearing region of a polypeptide described herein.

A nucleic acid fragment encoding a "biologically active portion of 59914 and 59921 polypeptides" can be prepared by isolating a portion of the nucleotide sequences of SEQ ID NO:1 or 3 and SEQ ID NO:4 or 6, which encode polypeptides having a 59914 and 59921 biological activity (e.g., the biological activities of the 59914 and 59921 proteins are described herein), expressing the encoded portion of the 59914 and 59921 proteins (e.g., by recombinant expression *in vitro*) and assessing the activity of the encoded portions of the 59914 and 59921 proteins. For example, nucleic acid fragments encoding biologically active portions of 59914 and 59921 include a conserved choline transporter domain, e.g., amino acid residues about 479-598 and 402-521 of SEQ ID NO:2 and SEQ ID NO:5, respectively. A nucleic acid fragment encoding a biologically active portion of a 59914 and 59921 polypeptide, may comprise a nucleotide sequence which is greater than 80 or more nucleotides in length.

In preferred embodiments, a nucleic acid includes a nucleotide sequence which is about 400, 440, 480, 500, 520, 530, 560, 600, 640, 680, 700, 720, 740, 760, 780, 800, 820, 840, 860, 880, 900, 920, 940, 960, 980, 1000, 1020, 1040, 1060, 1080, 1100, 1120, 1140, 1160, 1180, 1200, 1220, 1240, 1260, 1280, 1300, 1340, 1360, 1380, 1400, 1420, 1440, 1460, 1480, 1500, 1520, 1540, 1560, 1580, 1600, 1620, 1640, 1660, 1680, 1700, 1720, 1740, 1760, 1780, 1800, 1820, 1840, 1860, 1880, 1900, 1920, 1940, 1960, 1980, 2000, 2020, 2040, 2060, 2080, 2100, 2120, 2140, 2160, 2180, 2200, 2220, 2240, 2260, 2280, 2300, 2320, 2340, 2360, 2380, 2400, 2420, 2440, 2460, or more nucleotides in length and hybridizes under stringent hybridization conditions to a nucleic acid molecule of SEQ ID NO:1, or SEQ ID NO:3, or a complement thereof.

In preferred embodiments, a nucleic acid includes a nucleotide sequence which is about 400, 440, 480, 500, 520, 530, 560, 600, 640, 680, 700, 720, 740, 760, 780, 800, 820, 840, 860, 880, 900, 920, 940, 960, 980, 1000, 1020, 1040, 1060, 1080, 1100, 1120, 1140, 1160, 1180, 1200, 1220, 1240, 1260, 1280, 1300, 1340, 1360, 1380, 1400, 1420, 1440, 1460, 1480, 1500, 1520, 1540, 1560, 1580, 1600, 1620, 1640, 1660, 1680, 1700, 1720, 1740, 1760, 1780, 1800, 1820, 1840, 1860, 1880, 1900, 1920, 1940, 1960, 1980, 2000, 2020, 2040, 2060, 2080, 2100, 2120, 2140, 2160, 2180, 2200, 2220, or more nucleotides in length and hybridizes under stringent hybridization conditions to a nucleic acid molecule of SEQ ID NO:4, or SEQ ID NO:6, or a complement thereof.

### 59914 and 59921 Nucleic Acid Variants

The invention further encompasses nucleic acid molecules that differ from the nucleotide sequences shown in SEQ ID NO:1 and SEQ ID NO:4 or SEQ ID NO:3 and SEQ ID NO:6. Such differences can be due to degeneracy of the genetic code and result in a nucleic acid which encodes the same 59914 and 59921 proteins as those encoded by the nucleotide sequence disclosed herein. In another embodiment, an isolated nucleic acid molecule of the invention has a nucleotide sequence encoding a protein having an amino acid sequence which differs, by at least 1, but less than 5, 10, 20, 50, or 100 amino acid residues that shown in SEQ ID NO:2 and SEQ ID NO:5. If alignment is needed for this comparison, the sequences should be aligned for maximum homology. "Looped" out sequences from deletions or insertions, or mismatches, are considered differences.

Nucleic acids of the invention can be chosen for having codons which are preferred or non-preferred for a particular expression system. For example, the nucleic acid can be one in which at least one codon, preferably at least 10% or 20% of the codons, has been altered such that the sequence is optimized for expression in bacterial (e.g., *E. coli*), yeast, human, insect, or nonhuman mammalian (e.g., CHO) cells.

Nucleic acid variants can be naturally occurring, such as allelic variants (same locus), homologs (different locus), and orthologs (different organism) or can be non-naturally occurring. Non-naturally occurring variants can be made by mutagenesis techniques, including those applied to polynucleotides, cells, or organisms. The variants can contain nucleotide substitutions, deletions, inversions and insertions. Variation can occur in either or both the coding and non-coding regions. The variations can produce both conservative and non-conservative amino acid substitutions (as compared in the encoded product).

In a preferred embodiment, the nucleic acid differs from that of SEQ ID NO:1 or 3 and SEQ ID NO:4 or 6, e.g., as follows: by at least one but less than 10, 20, 30, or 40 nucleotides; at least one, but less than 1%, 5%, 10% or 20%, of the nucleotides in the subject nucleic acid. If necessary for this analysis the sequences should be aligned for maximum homology. "Looped" out sequences from deletions or insertions, or mismatches, are considered differences.

Orthologs, homologs, and allelic variants can be identified using methods known in the art. These variants comprise a nucleotide sequence encoding a polypeptide that is 50%, at least about 55%, typically at least about 70-75%, more typically at least about 80-85%, and most typically at least about 90-95% or more, identical to the nucleotide sequences shown in SEQ ID NO:2 and SEQ ID NO:5, or fragments of these sequences. Such nucleic acid molecules can readily be identified as being able to hybridize under stringent conditions to the nucleotide sequences shown in SEQ ID NO:2 and SEQ ID NO:5, or fragments of the sequences. Nucleic acid molecules corresponding to orthologs, homologs, and allelic variants of the 59914 and 59921 cDNAs of the invention can further be isolated by mapping to the same chromosome or locus as the 59914 and 59921 genes.

Preferred variants include those that are correlated with at least one of the following 59914 and 59921 biological activities:

(1) the ability to modulate (e.g., promote, catalyze, regulate, initiate, facilitate or inhibit) the manufacture of choline metabolites and/or compounds of which choline is a component or precursor, e.g., phospholipids (e.g., phosphatidylcholine (lecithin), sphingomyelin, sphingophosphorylcholine, and platelet activating factor), acetylcholine, very low density lipoproteins (VLDLs), and betaine, e.g., by transporting choline into or out of cells; (2) the ability to modulate (e.g., promote, catalyze, regulate, initiate, facilitate or inhibit) transport of choline, its metabolites, and/or compounds of which choline is a component or precursor across membranes (e.g., plasma membranes), e.g., from an extracellular medium into a cell, or vice versa; (3) the ability to modulate (e.g., promote, catalyze, regulate, initiate, facilitate or inhibit) transport of choline, its metabolites, and/or compounds of which choline is a component or precursor across barriers between tissues (e.g., the blood-brain barrier);

Other activities of the 59914 and 59921 proteins of the present invention include one or more of the following: (1) the ability to modulate (e.g., promote, regulate, initiate, facilitate or inhibit) the synthesis of, and the structural maintenance and reinforcement of, cellular components (e.g., membranes (e.g., plasma membranes) and microsomes) e.g., by modulating the transport of choline, its metabolites, and/or compounds of which choline is a component or precursor; (2) the ability to modulate (e.g., promote, regulate, initiate, facilitate or inhibit) cellular nutrition, e.g., by modulating the transport of choline, its metabolites, and/or compounds of which choline is a component or precursor; (3) the ability to modulate (e.g., promote, regulate, initiate, facilitate or inhibit) muscle control, e.g., by modulating the transport of choline, its metabolites, and/or compounds of which choline is a component or precursor (e.g., neurotransmitter acetylcholine); (4) the ability to modulate (e.g., promote, regulate, initiate, facilitate or inhibit) memory, e.g., by modulating the transport of choline, its metabolites, and/or compounds of which choline is a component or precursor (e.g., neurotransmitter acetylcholine); (5) the ability to modulate (e.g., promote, regulate, initiate, facilitate or inhibit) message transmission (e.g., nervous system message transmission), e.g., by modulating the transport of choline, its metabolites, and/or compounds of which choline is a component or precursor (e.g., neurotransmitter acetylcholine).

Still other activities of the 59914 and 59921 proteins of the present invention include one or more of the following: (1) the ability to modulate (e.g., promote, regulate, initiate, facilitate or inhibit) liver homeostasis, e.g., by transporting fat and/or cholesterol from the liver, e.g., by modulating the transport of choline, its metabolites, and/or compounds of which choline is a component or precursor (e.g., VLDLs); and (2) the ability to modulate (e.g., promote, regulate, initiate, facilitate or inhibit) cellular signaling, e.g., by modulating the transport of choline, its metabolites, and/or compounds of which choline is a component or precursor (e.g., sphingophosphorylcholine and platelet activating factor).

Other activities of the 59914 and 59921 proteins of the present invention include one or more of the following: (1) the ability to modulate (e.g., promote, regulate, initiate, facilitate or inhibit) liver disorders (e.g., hepatocyte apoptosis, and others described herein), e.g., by maintaining proper choline levels (e.g., preventing choline deficiency), e.g., by modulating the transport of choline, its metabolites, and/or compounds of which choline is a component or precursor; (2) the ability to modulate (e.g., promote, regulate, initiate, facilitate or inhibit) neurological disorders (e.g., central nervous system (CNS) disorders and others described herein), e.g., by maintaining proper choline levels (e.g., preventing choline excess), e.g., by modulating the transport of choline, its metabolites, and/or compounds of which choline is a component or precursor; (3) the ability to modulate (e.g., promote, regulate, initiate, facilitate or inhibit) cardiovascular disorders, e.g., by preventing buildup of homocysteines in the blood (e.g., by converting them to methionine) e.g., by modulating the transport of choline, its metabolites, and/or compounds of which choline is a component or precursor (e.g., betaine).

Allelic variants of 59914 and 59921, e.g., human 59914 and 59921, include both functional and non-functional proteins. Functional allelic variants are naturally occurring amino acid sequence variants of the 59914 and 59921 proteins within a population that maintain the 59914 and 59921 biological activities described herein.

Functional allelic variants will typically contain only conservative substitution of one or more amino acids of SEQ ID NO:2 and SEQ ID NO:5, or substitution, deletion or insertion of non-critical residues in non-critical regions of the protein. Non-functional allelic variants are naturally-occurring amino acid sequence variants of the 59914 and 59921, e.g., human 59914 and 59921, protein within a population that do not demonstrate the 59914 and 59921 activities described herein.

Non-functional allelic variants can typically contain a non-conservative substitution, a deletion, or insertion, or premature truncation of the amino acid sequences of SEQ ID NO:2 or SEQ ID NO:5, or a substitutions, insertions, or deletions in critical residues or critical regions of these proteins.

Moreover, nucleic acid molecules encoding other 59914 and 59921 family members and, thus, which have nucleotide sequences which differ from the 59914 and 59921 sequences of SEQ ID NO:1 and SEQ ID NO:4 or SEQ ID NO:3 and SEQ ID NO:6 are intended to be within the scope of the invention.

### 1.1 Antisense Nucleic Acid Molecules, Ribozymes and Modified 59914 and 59921 Nucleic Acid Molecules

In another aspect, the invention features isolated nucleic acid molecules which are antisense to 59914 and 59921. An "antisense" nucleic acid can include a nucleotide sequence which is complementary to a "sense" nucleic acid encoding a protein, e.g., complementary to the coding strand of a double-stranded cDNA molecule or complementary to an mRNA sequence. The antisense nucleic acids can be complementary to entire 59914 and 59921 coding strands, or to only portions thereof (e.g., the coding regions of human 59914 and 59921 corresponding to SEQ ID NO:3 and SEQ ID NO:6, respectively). In another embodiment, the antisense nucleic acid molecule is antisense to a "noncoding region" of the coding strands of nucleotide sequences encoding 59914 and 59921 (e.g., the 5' and 3' untranslated regions).

An antisense nucleic acid can be designed such that it is complementary to the entire coding regions of 59914 and 59921 mRNA, but more preferably is an oligonucleotide which is antisense to only portions of the coding or noncoding regions of 59914 and 59921 mRNA. For example, the antisense oligonucleotide can be complementary to the region surrounding the translation start sites of 59914 and 59921 mRNA, e.g., between the -10 and +10 regions of the target gene nucleotide sequence of interest. An antisense oligonucleotide can be, for example, about 7, 10, 15, 20, 25, 30, 35, 40, 45, 50, 55, 60, 65, 70, 75, 80, or more nucleotides in length.

An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. The antisense nucleic acid also can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject (e.g., by direct injection at a tissue site), or generated *in situ* such that they hybridize with or bind to cellular mRNA and/or genomic DNA encoding 59914 and 59921 proteins to thereby inhibit expression of the protein, e.g., by inhibiting transcription and/or translation. Alternatively, antisense nucleic acid molecules can be modified to target selected cells and then administered systemically. For systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the antisense molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) *Nucleic Acids. Res*. 15:6625-6641). The antisense nucleic acid molecule can also comprise a 2'-o-methylribonucleotide (Inoue et al. (1987) *Nucleic Acids Res*. 15:6131-6148) or a chimeric RNA-DNA analogue (Inoue et al. (1987) *FEBS Lett*. 215:327-330).

In still another embodiment, an antisense nucleic acid of the invention is a ribozyme. A ribozyme having specificity for a 59914 and 59921-encoding nucleic acid can include one or more sequences complementary to the nucleotide sequences of 59914 and 59921 cDNAs disclosed herein (i.e., SEQ ID NO:1 and SEQ ID NO:4 or SEQ ID NO:3 and SEQ ID NO:6), and a sequence having known catalytic sequence responsible for mRNA cleavage (see U.S. Pat. No. 5,093,246 or Haselhoff and Gerlach (1988) *Nature* 334:585-591). For example, a derivative of a *Tetrahymena* L-19 IVS RNA can be constructed in which the nucleotide sequence of the active site is complementary to the nucleotide sequence to be cleaved in a 59914 and 59921-encoding mRNA. See, e.g., Cech et al. U.S. Patent No. 4,987,071; and Cech et al. U.S. Patent No. 5,116,742. Alternatively, 59914 and 59921 mRNA can be used to select a catalytic RNA having a specific ribonuclease activity from a pool of RNA molecules. See, e.g., Bartel, D. and Szostak, J.W. (1993) *Science* 261:1411-1418.

59914 and 59921 gene expression can be inhibited by targeting nucleotide sequences complementary to the regulatory region of the 59914 and 59921 (e.g., the 59914 and 59921 promoters and/or enhancers) to form triple helical structures that prevent transcription of the 59914 and 59921 genes in target cells. See generally, Helene, C. (1991) *Anticancer Drug Des*. 6:569-84; Helene, C. i (1992) *Ann. N.Y. Acad. Sci*. 660:27-36; and Maher, L.J. (1992) *Bioassays* 14:807-15. The potential sequences that can be targeted for triple helix formation can be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

The invention also provides detectably labeled oligonucleotide primer and probe molecules. Typically, such labels are chemiluminescent, fluorescent, radioactive, or colorimetric.

59914 and 59921 nucleic acid molecules can be modified at the base moiety, sugar moiety or phosphate backbone to improve, e.g., the stability, hybridization, or solubility of the molecule. For example, the deoxyribose phosphate backbone of the nucleic acid molecules can be modified to generate peptide nucleic acids (see Hyrup B. et al. (1996) *Bioorganic & Medicinal Chemistry* 4: 5-23). As used herein, the terms "peptide nucleic acid" or "PNA" refers to a nucleic acid mimic, e.g., a DNA mimic, in which the deoxyribose phosphate backbone is replaced by a pseudopeptide backbone and only the four natural nucleobases are retained. The neutral backbone of a PNA can allow for specific hybridization to DNA and RNA under conditions of low ionic strength. The synthesis of PNA oligomers can be performed using standard solid phase peptide synthesis protocols as described in Hyrup B. et al. (1996) *supra;* Perry-O'Keefe et al. *Proc. Natl. Acad. Sci*. 93: 14670-675.

PNAs of 59914 and 59921 nucleic acid molecules can be used in therapeutic and diagnostic applications. For example, PNAs can be used as antisense or antigene agents for sequence-specific modulation of gene expression by, for example, inducing transcription or translation arrest or inhibiting replication. PNAs of 59914 and 59921 nucleic acid molecules can also be used in the analysis of single base pair mutations in a gene, (e.g., by PNA-directed PCR clamping); as 'artificial restriction enzymes' when used in combination with other enzymes, (e.g., S1 nucleases (Hyrup B. et al. (1996) *supra*)); or as probes or primers for DNA sequencing or hybridization (Hyrup B. et al. (1996) *supra*; Perry-O'Keefe *supra*).

In other embodiments, the oligonucleotide may include other appended groups such as peptides (e.g., for targeting host cell receptors *in vivo*), or agents facilitating transport across the cell membrane (see, e.g., Letsinger et al. (1989) *Proc. Natl. Acad. Sci. USA* 86:6553-6556; Lemaitre et al. (1987) *Proc. Natl. Acad. Sci. USA* 84:648-652; PCT Publication No. W088/09810) or the blood-brain barrier (see, e.g., PCT Publication No. W089/10134). In addition, oligonucleotides can be modified with hybridization-triggered cleavage agents (see, e.g., Krol et al. (1988) *BioTechniques* 6:958-976) or intercalating agents. (see, e.g., Zon (1988) *Pharm. Res*. 5:539-549). To this end, the oligonucleotide may be conjugated to another molecule, (e.g., a peptide, hybridization triggered cross-linking agent, transport agent, or hybridization-triggered cleavage agent).

The invention also includes molecular beacon oligonucleotide primer and probe molecules having at least one region which is complementary to a 59914 and 59921 nucleic acids of the invention, two complementary regions one having a fluorophore and one a quencher such that the molecular beacon is useful for quantitating the presence of the 59914 and 59921 nucleic acids of the invention in a sample. Molecular beacon nucleic acids are described, for example, in Lizardi et al., U.S. Patent No. 5,854,033; Nazarenko et al., U.S. Patent No. 5,866,336, and Livak et al., U.S. Patent 5,876,930.

### Isolated 59914 and 59921 Polypeptides

In another aspect, the invention features, isolated 59914 and 59921 proteins, or fragments, e.g., biologically active portions, for use as immunogens or antigens to raise or test (or more generally to bind) anti-59914 and 59921 antibodies. 59914 and 59921 proteins can be isolated from cells or tissue sources using standard protein purification techniques. 59914 and 59921 proteins, or fragments thereof, can be produced by recombinant DNA techniques or synthesized chemically.

Polypeptides of the invention include those which arise as a result of the existence of multiple genes, alternative transcription events, alternative RNA splicing events, and alternative translational and post-translational events. The polypeptide can be expressed in systems, e.g., cultured cells, which result in substantially the same post-translational modifications present when expressed the polypeptide is expressed in a native cell, or in systems which result in the alteration or omission of post-translational modifications, e.g., glycosylation or cleavage, present when expressed in a native cell.

In a preferred embodiment, 59914 and 59921 polypeptides have one or more of the following characteristics:

the ability to: (1) modulate (e.g., promote, catalyze, regulate, initiate, facilitate or inhibit) the manufacture of choline metabolites and/or the compounds of which choline is a component or precursor, e.g., phospholipids (e.g., phosphatidylcholine (lecithin), sphingomyelin, sphingophosphorylcholine, and platelet activating factor), acetylcholine, very low density lipoproteins (VLDLs), and betaine, e.g., by transporting choline into or out of cells; (2) modulate (e.g., promote, catalyze, regulate, initiate, facilitate or inhibit) transport of choline, its metabolites, and/or compounds of which choline is a component or precursor across membranes (e.g., plasma membranes), e.g., from an extracellular medium into a cell, or vice versa; (3) modulate (e.g., promote, catalyze, regulate, initiate, facilitate or inhibit) transport of choline, its metabolites, and/or compounds of which choline is a component or precursor across barriers between tissues (e.g., the blood-brain barrier);

the ability to: (1) modulate (e.g., promote, regulate, initiate, facilitate or inhibit) the synthesis of, and the structural maintenance and reinforcement of, cellular components (e.g., membranes (e.g., plasma membranes) and microsomes) e.g., by modulating the transport of choline, its metabolites, and/or compounds of which choline is a component or precursor; (2) modulate (e.g., promote, regulate, initiate, facilitate or inhibit) cellular nutrition, e.g., by modulating the transport of choline, its metabolites, and/or compounds of which choline is a component or precursor; (3) modulate (e.g., promote, regulate, initiate, facilitate or inhibit) muscle control, e.g., by modulating the transport of choline, its metabolites, and/or compounds of which choline is a component or precursor (e.g., neurotransmitter acetylcholine); (4) modulate (e.g., promote, regulate, initiate, facilitate or inhibit) memory, e.g., by modulating the transport of choline, its metabolites, and/or compounds of which choline is a component or precursor (e.g., neurotransmitter acetylcholine); (5) modulate (e.g., promote, regulate, initiate, facilitate or inhibit) message transmission (e.g., nervous system message transmission), e.g., by modulating the transport of choline, its metabolites, and/or compounds of which choline is a component or precursor (e.g., neurotransmitter acetylcholine).

the ability to (1) modulate (e.g., promote, regulate, initiate, facilitate or inhibit) liver homeostasis, e.g., by transporting fat and/or cholesterol from the liver, e.g., by modulating the transport of choline, its metabolites, and/or compounds of which choline is a component or precursor (e.g., VLDLs); and (2) modulate (e.g., promote, regulate, initiate, facilitate or inhibit) cellular signaling, e.g., by modulating the transport of choline, its metabolites, and/or compounds of which choline is a component or precursor (e.g., sphingophosphorylcholine and platelet activating factor).

the ability to: (1) modulate (e.g., promote, regulate, initiate, facilitate or inhibit) liver disorders (e.g., hepatocyte apoptosis, and others described herein), e.g., by maintaining proper choline levels (e.g., preventing choline deficiency), e.g., by modulating the transport of choline, its metabolites, and/or compounds of which choline is a component or precursor; (2) modulate (e.g., promote, regulate, initiate, facilitate or inhibit) central nervous system (CNS) disorders (e.g., hepatocyte apoptosis, and others described herein), e.g., by maintaining proper choline levels (e.g., preventing choline excess), e.g., by modulating the transport of choline, its metabolites, and/or compounds of which choline is a component or precursor; (3) modulate (e.g., promote, regulate, initiate, facilitate or inhibit) cardiovascular disorders, e.g., by preventing buildup of homocysteines in the blood (e.g., by converting them to methionine) e.g., by modulating the transport of choline, its metabolites, and/or compounds of which choline is a component or precursor (e.g., betaine).

a molecular weight, e.g., a deduced molecular weight (e.g., of 81.6 kDa for 59914 and of 73.8 kDa for 59921), preferably ignoring any contribution of post translational modifications, amino acid composition or other physical characteristic of SEQ ID NO:2 and SEQ ID NO:5;

a mapping position, e.g., a mapping position deduced by comparing 59914 and 59921 sequence with sequences of known positions in the genome. In one embodiment, 59921 maps to chromosomal position 1p21.3-22.3, based on at least several regions of homology to a clone in the art known to map to chromosomal position 1p21.3-22.3 (clone RP4-639P13, Genbank accession number AL359554), including by way of example: 98% homology over 250 base pairs (base pairs 1717-1966) of SEQ ID NO:4; 99% homology over 241 base pairs (base pairs 1961-2201) of SEQ ID NO:4; and 100% homology over 189 base pairs (base pairs 994-1182) of SEQ ID NO:4.

an overall sequence similarity of at least 50%, preferably at least 60%, more preferably at least 70, 80, 90, or 95%, with a polypeptide of SEQ ID NO:2 and SEQ ID NO:5;

an expression pattern as follows, based on TaqMan analysis of 59914 and 59921 expression in at least the following human tissues and cell lines (described below in the Exemplification): high levels of 59914 expression in normal brain cortex; moderate levels of 59914 expression in human umbilical vein endothelial cells (HUVEC), prostate tumor and lung tumor; low levels of 59914 expression in colon tumor, kidney, and hypothalamus; high 59921 levels of expression in kidney, pancreas, and colon tumor; and low to moderate 59921 levels of expression in spinal cord, hypothalamus, nerve, dorsal root ganglia, prostate tumor, lung tumor, salivary glands, and liver fibrosis; and

a conserved choline transporter domain which is preferably about 70%, 80%, 82%, 84%, 85%, 86%, 87%, 88%, 89%, 90%, 91%, 92%, 93%, 94%, 95%, 96%, 97%, 98%, 99%, or 100% identical with the sequence containing amino acid residues about 479-598 of SEQ ID NO:2 and at least 402-521 of SEQ ID NO:5.

In a preferred embodiment, the 59914 and 59921 proteins, or fragments thereof, differ from the corresponding sequences in SEQ ID NO:2 and SEQ ID NO:5. In one embodiment they differ by at least one, but by less than 15, 10 or 5, amino acid residues. In another they differ from the corresponding sequences in SEQ ID NO:2 and SEQ ID NO:5 by at least one residue, but less than 20%, 15%, 10% or 5%, of the residues in them differ from the corresponding sequences in SEQ ID NO:2 and SEQ ID NO:5. (If this comparison requires alignment the sequences should be aligned for maximum homology. "Looped" out sequences from deletions or insertions, or mismatches, are considered differences.) The differences are, preferably, differences or changes at a nonessential residue or a conservative substitution. In a preferred embodiment the differences are not in the conserved choline transporter domain. In another preferred embodiment one or more differences are in the conserved choline transporter domain.

Other embodiments include a protein that contains one or more changes in amino acid sequence, e.g., a change in an amino acid residue which is not essential for activity. Such 59914 and 59921 proteins differ in amino acid sequence from SEQ ID NO:2 and SEQ ID NO:5, yet retain biological activity.

In one embodiment, the protein includes an amino acid sequence at least about 50%, 55%, 60%, 65%, 70%, 75%, 60%, 70%, 80%, 82%, 84%, 85%, 86%, 87%, 88%, 89%, 90%, 91%, 92%, 93%, 94%, 95%, 96%, 97%, 98%, or more, homologous to SEQ ID NO:2 and SEQ ID NO:5.

59914 and 59921 proteins or fragment is provided which varies from the sequences of SEQ ID NO:2 and SEQ ID NO:5 in regions that do not correspond to a domain specifically defined herein (e.g., from about amino acids 1 to 140 or 551 to 561) by at least one, but by less than 15, 10 or 5, amino acid residues in the protein or fragment, but which does not differ from the sequences of SEQ ID NO:2 and SEQ ID NO:5 in regions that correspond to a domain specifically defined herein (e.g., from about amino acids about 141 to 551). (If this comparison requires alignment the sequences should be aligned for maximum homology. "Looped" out sequences from deletions or insertions, or mismatches, are considered differences.) In some embodiments the difference is at a non-essential residue or is a conservative substitution, while in others the difference is at an essential residue or is a non-conservative substitution.

In one embodiment, a biologically active portion of 59914 and 59921 proteins includes a conserved choline transporter domain. Moreover, other biologically active portions, in which other regions of the protein are deleted, can be prepared by recombinant techniques and evaluated for one or more of the functional activities of native 59914 and 59921 proteins.

In a preferred embodiment, the 59914 and 59921 protein has amino acid sequences shown in SEQ ID NO:2 and SEQ ID NO:5. In other embodiments, the 59914 and 59921 proteins are substantially identical to SEQ ID NO:2 and SEQ ID NO:5. In yet another embodiment, the 59914 and 59921 proteins are substantially identical to SEQ ID NO:2 and SEQ ID NO:5 and retain the functional activities of the proteins of SEQ ID NO:2 and SEQ ID NO:5, as described herein.

### 59914 and 59921 Chimeric or Fusion Proteins

In another aspect, the invention provides 59914 and 59921 chimeric or fusion proteins. As used herein, a 59914 and 59921 "chimeric protein" or "fusion protein" includes a 59914 and 59921 polypeptide linked to a non-59914 and 59921 polypeptide. A "non-59914 and 59921 polypeptide" refers to polypeptide having an amino acid sequence corresponding to a protein which is not substantially homologous to the 59914 and 59921 proteins, e.g., a protein which is different from the 59914 and 59921 proteins and which is derived from the same or a different organism. The 59914 and 59921 polypeptides of the fusion protein can correspond to all or a portion, e.g., a fragment, described herein of a 59914 and 59921 amino acid sequence. In a preferred embodiment, 59914 and 59921 fusion proteins include at least one (or two) biologically active portion of 59914 and 59921 proteins. The non-59914 and 59921 polypeptide can be fused to the N-terminus or C-terminus of the 59914 and 59921 polypeptide.

One useful fusion protein is a GST fusion protein in which the polypeptide of the invention is fused with the carboxyl terminus of GST sequences. Such fusion proteins can facilitate purification of a recombinant polypeptide of the invention.

In another embodiment, the fusion protein contains a heterologous signal sequence at its amino terminus. For example, the native signal sequence of a polypeptide of the invention can be removed and replaced with a signal sequence from another protein. For example, the gp67 secretory sequence of the baculovirus envelope protein can be used as a heterologous signal sequence (Current Protocols in Molecular Biology, Ausubel et al., eds., John Wiley & Sons, 1992). Other examples of eukaryotic heterologous signal sequences include the secretory sequences of melittin and human placental alkaline phosphatase (Stratagene; La Jolla, California). In yet another example, useful prokaryotic heterologous signal sequences include the phoA secretory signal (Sambrook et al., supra) and the protein A secretory signal (Pharmacia Biotech; Piscataway, New Jersey).

Fusion proteins can include all or a part of a serum protein, e.g., a portion of an immunoglobulin protein (e.g., IgG, IgA, or IgE); an Fc region; and/or the hinge C1 and C2 sequences of an immunoglobulin or human serum albumin.

Moreover, the immunoglobulin fusion proteins of the invention can be used as immunogens to produce anti-59914 and 59921 antibodies directed against a polypeptide of the invention in a subject, to purify 59914 and 59921 ligands and in screening assays to identify molecules which inhibit the interaction of 59914 and 59921 receptors with 59914 and 59921 ligands. The immunoglobulin fusion protein can, for example, comprise a portion of a polypeptide of the invention fused with the amino-terminus or the carboxyl-terminus of an immunoglobulin constant region, as disclosed in U.S. Patent No. 5,714,147, U.S. Patent No. 5,116,964, U.S. Patent No. 5,514,582, and U.S. Patent No. 5,455,165.

The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a polypeptide of the invention. Inhibition of ligand / receptor interaction can be useful therapeutically, both for treating disorders caused by, for example: (i) aberrant modification or mutation of a gene encoding 59914 and 59921 proteins; (ii) mis-regulation of the 59914 and 59921 genes; and (iii) aberrant post-translational modification of 59914 and 59921 proteins.

Chimeric and fusion proteins of the invention can be produced by standard recombinant DNA techniques. In another embodiment, the fusion gene can be synthesized by conventional techniques including automated DNA synthesizers. Alternatively, PCR amplification of gene fragments can be performed using anchor primers which give rise to complementary overhangs between two consecutive gene fragments and which can subsequently be annealed and re-amplified to generate a chimeric gene sequence (see, e.g., Ausubel et al., supra). Moreover, many expression vectors are commercially available that already encode a fusion moiety (e.g., a GST polypeptide). A nucleic acid encoding a polypeptide of the invention can be cloned into such an expression vector such that the fusion moiety is linked in-frame to the polypeptide of the invention.

### Variants of 59914 and 59921 Proteins

In another aspect, the invention also features a variants of 59914 and 59921 polypeptides, e.g., which function as agonists (mimetics) or as antagonists. Variants of the 59914 and 59921 proteins can be generated by mutagenesis, e.g., discrete point mutation, the insertion or deletion of sequences or the truncation of 59914 and 59921 proteins. An agonist of the 59914 and 59921 proteins can retain substantially the same, or a subset, of the biological activities of the naturally occurring form of 59914 and 59921 proteins. An antagonist of 59914 and 59921 proteins can inhibit one or more of the activities of the naturally occurring form of the 59914 and 59921 proteins by, for example, competitively modulating a 59914 and 59921-mediated activity of 59914 and 59921 proteins. Thus, specific biological effects can be elicited by treatment with a variant of limited function. Preferably, treatment of a subject with a variant having a subset of the biological activities of the naturally occurring form of the protein has fewer side effects in a subject relative to treatment with the naturally occurring form of the 59914 and 59921 proteins.

Variants of a protein of the invention which function as either agonists (e.g., mimetics) or as antagonists can be identified by screening combinatorial libraries of mutants, e.g., truncation mutants, of the 59914 and 59921 proteins for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences can be expressed as individual polypeptides, or alternatively, as a set of larger fusion proteins (e.g., for phage display). There are a variety of methods which can be used to produce libraries of potential variants of the polypeptides of the invention from a degenerate oligonucleotide sequence. Methods for synthesizing degenerate oligonucleotides are known in the art (see, e.g., Narang (1983) *Tetrahedron* 39:3; Itakura et al. (1984) *Annu. Rev. Biochem*. 53:323; Itakura et al. (1984) *Science* 198:1056; Ike et al. (1983) *Nucleic Acid Res*. 11:477).

In addition, libraries of fragments of the coding sequence of a polypeptide of the invention can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, re-naturing the DNA to form double stranded DNA which can include sense / antisense pairs from different nicked products, removing single stranded portions from reformed duplexes by treatment with S1 nuclease, and ligating the resulting fragment library into an expression vector. By this method, an expression library can be derived which encodes amino terminal and internal fragments of various sizes of the protein of interest.

Variants in which a cysteine residues is added or deleted or in which a residue which is glycosylated is added or deleted are particularly preferred.

Methods for screening gene products of combinatorial libraries made by point mutations or truncation, and for screening cDNA libraries for gene products having a selected property. Recursive ensemble mutagenesis (REM), a new technique which enhances the frequency of functional mutants in the libraries, can be used in combination with the screening assays to identify 59914 and 59921 variants (Arkin and Yourvan (1992) *Proc. Natl. Acad. Sci. USA 89*:7811-7815; Delgrave et al. (1993) *Protein Engineering* 6:327-331).

Cell based assays can be exploited to analyze variegated 59914 and 59921 libraries. For example, a library of expression vectors can be transfected into a cell line, e.g., a cell line, which ordinarily responds to 59914 and 59921 in a substrate-dependent manner. The transfected cells are then contacted with 59914 and 59921 and the effect of the expression of the mutant on signaling by the 59914 and 59921 substrates can be detected, for example, by assaying (i) the interaction of 59914 and 59921 proteins with 59914 and 59921 target molecules, e.g., choline, its metabolites, and/or compounds of which choline is a component or precursor, e.g., which 59914 and 59921 proteins can transport into cells from the extracellular fluid, e.g., for plasma membrane synthesis; (ii) the interaction of 59914 and 59921 proteins with 59914 and 59921 target molecules, wherein the 59914 and 59921 target is a ligand, e.g., a choline transporter ligand; or (iii) the interaction of 59914 and 59921 proteins with 59914 and 59921 target molecules, wherein the 59914 and 59921 target is a receptor, e.g., a choline transporter receptor. Plasmid DNA can then be recovered from the cells which score for inhibition, or alternatively, potentiation of signaling by the 59914 and 59921 substrate, and the individual clones further characterized.

In another aspect, the invention features a method of making 59914 and 59921 polypeptides, e.g., peptides having a non-wild type activity, e.g., an antagonist, agonist, or super agonist of a naturally occurring 59914 and 59921 polypeptides, e.g., naturally occurring 59914 and 59921 polypeptides. The method includes: altering the sequence of 59914 and 59921 polypeptides, e.g., altering the sequence, e.g., by substitution or deletion of one or more residues of a non-conserved region, a domain or residue disclosed herein, and testing the altered polypeptide for the desired activity.

In another aspect, the invention features a method of making a fragment or analog of 59914 and 59921 polypeptides which demonstrate biological activities of naturally occurring 59914 and 59921 polypeptides. The method includes: altering the sequence, e.g., by substitution or deletion of one or more residues, of 59914 and 59921 polypeptides, e.g., altering the sequence of a non-conserved region, or a domain or residue described herein, and testing the altered polypeptide for the desired activity.

### Anti-59914 and 59921 Antibodies

In another aspect, the invention provides an anti-59914 and 59921 antibody. The term "antibody" as used herein refers to an immunoglobulin molecule and immunologically active portions of immunoglobulin molecules, i.e., molecules that contain an antigen binding site which specifically binds (immunoreacts with) an antigen, such as 59914 and 59921 molecules. Examples of immunologically active portions of immunoglobulin molecules include scFV and dcFV fragments, Fab and F(ab')₂ fragments which can be generated by treating the antibody with an enzyme such as papain or pepsin, respectively.

The antibody can be a polyclonal, monoclonal, recombinant, e.g., a chimeric, humanized, fully human, non-human (*e.g*., murine, rat, rabbit, or goat), or single chain antibody. In a preferred embodiment it has effector function and can fix complement. The antibody can be coupled to a toxin or imaging agent.

The term "monoclonal antibody" or "monoclonal antibody composition", as used herein, refers to a population of antibody molecules that contain only one species of an antigen binding site capable of immunoreacting with a particular epitope of 59914 and 59921. A monoclonal antibody composition thus typically displays a single binding affinity for a particular 59914 and 59921 protein with which it immunoreacts.

Polyclonal anti-59914 and 59921 antibodies can be prepared as described above by immunizing a suitable subject with a 59914 and 59921 immunogen. The anti-59914 and 59921 antibody titer in the immunized subject can be monitored over time by standard techniques, such as with an enzyme linked immunosorbent assay (ELISA) using immobilized 59914 and 59921. If desired, the antibody molecules directed against 59914 and 59921 can be isolated from the mammal (e.g., from the blood) and further purified by well known techniques, such as protein A chromatography to obtain the IgG fraction. At an appropriate time after immunization, *e.g*., when the anti-59914 and 59921 antibody titers are highest, antibody-producing cells can be obtained from the subject and used to prepare monoclonal antibodies by standard techniques, such as the hybridoma technique originally described by Kohler and Milstein (1975) *Nature* 256:495-497) (see also, Brown et al. (1981) *J. Immunol.* 127:539-46; Brown et al. (1980) *J. Biol. Chem* .255:4980-83; Yeh et al. (1976) *Proc. Natl. Acad. Sci. USA* 76:2927-31; and Yeh et al. (1982) *Int. J. Cancer* 29:269-75), the more recent human B cell hybridoma technique (Kozbor et al. (1983) *Immunol Today* 4:72), the EBV-hybridoma technique (Cole et al. (1985), *Monoclonal* *Antibodies and Cancer Therapy*, Alan R. Liss, Inc., pp. 77-96) or trioma techniques. The technology for producing monoclonal antibody hybridomas is well known (see generally R. H. Kenneth, in *Monoclonal Antibodies: A New Dimension In Biological Analyses*, Plenum Publishing Corp., New York, New York (1980); E. A. Lerner (1981) *Yale J. Biol. Med*., 54:387-402; M. L. Gefter et al. (1977) *Somatic Cell Genet*. 3:231-36). Briefly, an immortal cell line (typically a myeloma) is fused to lymphocytes (typically splenocytes) from a mammal immunized with a 59914 and 59921 immunogen as described above, and the culture supernatants of the resulting hybridoma cells are screened to identify a hybridoma producing a monoclonal antibody that binds 59914 and 59921.

Any of the many well known protocols used for fusing lymphocytes and immortalized cell lines can be applied for the purpose of generating an anti-59914 and 59921 monoclonal antibody (see, e.g., G. Galfre et al. (1977) *Nature* 266:55052; Gefter et al. *Somatic Cell Genet*., cited *supra;* Lerner, *Yale J. Biol. Med*., cited *supra*; Kenneth, *Monoclonal Antibodies,* cited *supra*). Moreover, the ordinarily skilled worker will appreciate that there are many variations of such methods which also would be useful. Typically, the immortal cell line (*e.g.*, a myeloma cell line) is derived from the same mammalian species as the lymphocytes. For example, murine hybridomas can be made by fusing lymphocytes from a mouse immunized with an immunogenic preparation of the present invention with an immortalized mouse cell line. Preferred immortal cell lines are mouse myeloma cell lines that are sensitive to culture medium containing hypoxanthine, aminopterin and thymidine ("HAT medium"). Any of a number of myeloma cell lines can be used as a fusion partner according to standard techniques, *e.g*., the P3-NS1/1-Ag4-1, P3-x63-Ag8.653 or Sp2/O-Ag14 myeloma lines. These myeloma lines are available from ATCC. Typically, HAT-sensitive mouse myeloma cells are fused to mouse splenocytes using polyethylene glycol ("PEG"). Hybridoma cells resulting from the fusion are then selected using HAT medium, which kills unfused and unproductively fused myeloma cells (unfused splenocytes die after several days because they are not transformed). Hybridoma cells producing a monoclonal antibody of the invention are detected by screening the hybridoma culture supernatants for antibodies that bind 59914 and 59921, *e.g*., using a standard ELISA assay.

Alternative to preparing monoclonal antibody-secreting hybridomas, a monoclonal anti-59914 and 59921 antibody can be identified and isolated by screening a recombinant combinatorial immunoglobulin library (*e.g.*, an antibody phage display library) with 59914 and 59921 to thereby isolate immunoglobulin library members that bind 59914 and 59921. Kits for generating and screening phage display libraries are commercially available (*e.g*., the Pharmacia *Recombinant Phage Antibody System,* Catalog No. 27-9400-01; and the Stratagene *SurfZAP™ Phage Display Kit*, Catalog No. 240612). Additionally, examples of methods and reagents particularly amenable for use in generating and screening antibody display library can be found in, for example, Ladner et al. U.S. Patent No. 5,223,409; Kang et al. PCT International Publication No. WO 92/18619; Dower et al. PCT International Publication No. WO 91/17271; Winter et al. PCT International Publication WO 92/20791; Markland et al. PCT International Publication No. WO 92/15679; Breitling et al. PCT International Publication WO 93/01288; McCafferty et al. PCT International Publication No. WO 92/01047; Garrard et al. PCT International Publication No. WO 92/09690; Ladner et al. PCT International Publication No. WO 90/02809; Fuchs et al. (1991) *Bio*/*Technology* 9:1370-1372; Hay et al. (1992) *Hum. Antibod. Hybridomas* 3:81-85; Huse et al. (1989) *Science* 246:1275-1281; Griffiths et al. (1993) *EMBO J* 12:725-734; Hawkins et al. (1992) *J. Mol. Biol.* 226:889-896; Clarkson et al. (1991) *Nature* 352:624-628; Gram et al. (1992) *Proc. Natl. Acad. Sci. USA* 89:3576-3580; Garrad et al. (1991) *Bio*/*Technology* 9:1373-1377; Hoogenboom et al. (1991) *Nuc. Acid Res.* 19:4133-4137; Barbas et al. (1991) *Proc. Natl. Acad. Sci. USA* 88:7978-7982; and McCafferty et al. *Nature* (1990) 348:552-554.

Additionally, chimeric, humanized, and completely human antibodies are also within the scope of the invention. Chimeric, humanized, but most preferably, completely human antibodies are desirable for applications which include repeated administration, e.g., therapeutic treatment of human patients, and some diagnostic applications.

Chimeric and humanized monoclonal antibodies, comprising both human and non-human portions, can be made using standard recombinant DNA techniques. Such chimeric and humanized monoclonal antibodies can be produced by recombinant DNA techniques known in the art, for example using methods described in Robinson et al. International Application No. PCT/US86/02269; Akira, et al. European Patent Application 184,187; Taniguchi, M., European Patent Application 171,496; Morrison et al. European Patent Application 173,494; Neuberger et al. PCT International Publication No. WO 86/01533; Cabilly et al. U.S. Patent No. 4,816,567; Cabilly et al. European Patent Application 125,023; Better et al. (1988) *Science* 240:1041-1043; Liu et al. (1987) *Proc. Natl. Acad. Scl. USA* 84:3439-3443; Liu et al. (1987) *J. Immunol.* 139:3521-3526; Sun et al. (1987) *Proc. Natl. Acad. Sci. USA* 84:214-218; Nishimura et al. (1987) *Canc. Res.* 47:999-1005; Wood et al. (1985) *Nature* 314:446-449; and Shaw et al. (1988) *J. Natl. Cancer Inst.* 80:1553-1559); Morrison, S. L. (1985) *Science* 229:1202-1207; Oi et al. (1986) *BioTechniques* 4:214; Winter U.S. Patent 5,225,539; Jones et al. (1986) *Nature* 321:552-525; Verhoeyan et al. (1988) *Science* 239:1534; and Beidler et al. (1988) *J. Immunol.* 141:4053-4060.

Completely human antibodies are particularly desirable for therapeutic treatment of human patients. Such antibodies can be produced using transgenic mice that are incapable of expressing endogenous immunoglobulin heavy and light chains genes, but which can express human heavy and light chain genes. See, for example, Lonberg and Huszar (1995) *Int. Rev. Immunol*. *13*:65-93); and U.S. Patent Nos. 5,625,126; 5,633,425; 5,569,825; 5,661,016; and 5,545,806. In addition, companies such as Abgenix, Inc. (Fremont, CA) and Medarex, Inc. (Princeton, NJ), can be engaged to provide human antibodies directed against a selected antigen using technology similar to that described above.

Completely human antibodies that recognize a selected epitope can be generated using a technique referred to as "guided selection." In this approach a selected non-human monoclonal antibody, e.g., a murine antibody, is used to guide the selection of a completely human antibody recognizing the same epitope. This technology is described by Jespers et al. (1994) *Bio*/*Technology 12*:899-903).

Full-length 59914 and 59921 proteins, or antigenic peptide fragments of 59914 and 59921, can be used as an immunogen or can be used to identify anti-59914 and 59921 antibodies made with other immunogens, e.g., cells, membrane preparations, and the like. The antigenic peptides of 59914 and 59921 should include at least 8 amino acid residues of the amino acid sequence shown in SEQ ID NO:2 and SEQ ID NO:5 and encompass an epitope of 59914 and 59921, respectively. Preferably, the antigenic peptide includes at least 10 amino acid residues, more preferably at least 15 amino acid residues, even more preferably at least 20 amino acid residues, and most preferably at least 30 amino acid residues.

Fragments of 59914 and 59921 which include, e.g., residues 479-598 of SEQ ID NO:2 and 402-521 SEQ ID NO:5, can be used as immunogens to make an antibodies against the conserved choline transporter domain of the 59914 and 59921 proteins.

Antibodies reactive with, or specific or selective for, any of these regions, or other regions or domains described herein are provided.

In an alternative embodiment, the antibody fails to bind to an Fc receptor, e.g., it is a type which does not support Fc receptor binding or has been modified, e.g., by deletion or other mutation, such that is does not have a functional Fc receptor binding region.

Preferred epitopes encompassed by the antigenic peptide are regions of 59914 and 59921 which are located on the surface of the protein, *e.g.*, hydrophilic regions (depicted, e.g., in the hydropathy plot in *Figure* 1, as residues below the dashed horizontal line), as well as regions with high antigenicity. For example, an Emini surface probability analysis of the human 59914 and 59921 protein sequences can be used to identify the regions that have a particularly high probability of being localized to the surface of the 59914 and 59921 proteins, and are thus likely to constitute surface residues useful for targeting antibody production.

In a preferred embodiment the antibody binds an epitope on any domain or region on 59914 and 59921 proteins described herein.

The anti-59914 and 59921 antibody can be a single chain antibody. A single-chain antibody (scFV) may be engineered as described, for example, in Colcher, D. et al., (1999 ) *Ann. NY Acad. Sci.* 880: 263-80; and Reiter, Y., *Clin. Cancer Res*. 1996 Feb;2(2):245-52. The single chain antibody can be dimerized or multimerized to generate multivalent antibodies having specificities for different epitopes of the same target 59914 and 59921 protein.

Anti-59914 and 59921 antibodies (e.g., monoclonal antibodies) can be used to isolate 59914 and 59921, respectively, by standard techniques, such as affinity chromatography or immunoprecipitation. Moreover, an anti-59914 and 59921 antibody can be used to detect 59914 and 59921 protein, respectively, (e.g., in a cellular lysate or cell supernatant) in order to evaluate the abundance and pattern of expression of the protein. Anti-59914 and 59921 antibodies can be used diagnostically to monitor protein levels in tissue as part of a clinical testing procedure, e.g., to, for example, determine the efficacy of a given treatment regimen. Detection can be facilitated by coupling (i.e., physically linking) the antibody to a detectable substance (i.e., antibody labeling). Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### 1.2 Recombinant Expression Vectors, Host Cells and Genetically Engineered Cells

In another aspect, the invention includes, vectors, preferably expression vectors, containing a nucleic acid encoding a polypeptide described herein. As used herein, the term "vector" refers to a nucleic acid molecule capable of transporting another nucleic acid to which it has been linked and can include a plasmid, cosmid or viral vector. The vector can be capable of autonomous replication or it can integrate into a host DNA. Viral vectors include, e.g., replication defective retroviruses, adenoviruses and adeno-associated viruses.

A vector can include 59914 and 59921 nucleic acids in a form suitable for expression of the nucleic acids in a host cell. Preferably the recombinant expression vector includes one or more regulatory sequences operatively linked to the nucleic acid sequence to be expressed. The term "regulatory sequence" includes promoters, enhancers and other expression control elements (e.g., polyadenylation signals). Regulatory sequences include those which direct constitutive expression of a nucleotide sequence, as well as tissue-specific regulatory and/or inducible sequences. The design of the expression vector can depend on such factors as the choice of the host cell to be transformed, the level of expression of protein desired, and the like. The expression vectors of the invention can be introduced into host cells to thereby produce proteins or polypeptides, including fusion proteins or polypeptides, encoded by nucleic acids as described herein (e.g., 59914 and 59921 proteins, mutant forms of 59914 and 59921 proteins, fusion proteins, and the like).

The recombinant expression vectors of the invention can be designed for expression of 59914 and 59921 proteins in prokaryotic or eukaryotic cells. For example, polypeptides of the invention can be expressed in *E. coli*, insect cells (e.g., using baculovirus expression vectors), yeast cells or mammalian cells. Suitable host cells are discussed further in Goeddel, (1990) Gene *Expression Technology: Methods in Enzymology* 185, Academic Press, San Diego, CA . Alternatively, the recombinant expression vector can be transcribed and translated *in vitro*, for example using T7 promoter regulatory sequences and T7 polymerase.

Expression of proteins in prokaryotes is most often carried out in *E. coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin, and enterokinase. Typical fusion expression vectors include pGEX (Pharmacia Biotech, Inc; Smith, D.B. and Johnson, K.S. (1988) *Gene* 67:31-40), pMAL (New England Biolabs, Beverly MA) and pRIT5 (Pharmacia, Piscataway NJ) which fuse glutathione S-transferase (GST), maltose E binding protein, or protein A, respectively, to the target recombinant protein.

Purified fusion proteins can be used in 59914 and 59921 activity assays, (e.g., direct assays or competitive assays described in detail below), or to generate antibodies specific for 59914 and 59921 proteins. In a preferred embodiment, a fusion protein expressed in a retroviral expression vector of the present invention can be used to infect bone marrow cells which are subsequently transplanted into irradiated recipients. The pathology of the subject recipient is then examined after sufficient time has passed (e.g., six weeks).

To maximize recombinant protein expression in *E. coli* is to express the protein in a host bacteria with an impaired capacity to proteolytically cleave the recombinant protein (Gottesman, S., (1990) *Gene Expression Technology: Methods in Enzymology* 185, Academic Press, San Diego, California 119-128). Another strategy is to alter the nucleic acid sequence of the nucleic acid to be inserted into an expression vector so that the individual codons for each amino acid are those preferentially utilized in *E. coli* (Wada et al., (1992) *Nucleic Acids Res.* 20:2111-2118). Such alteration of nucleic acid sequences of the invention can be carried out by standard DNA synthesis techniques.

The 59914 and 59921 expression vectors can be yeast expression vectors, vectors for expression in insect cells, e.g., baculovirus expression vectors or vectors suitable for expression in mammalian cells.

When used in mammalian cells, the expression vector's control functions are often provided by viral regulatory elements. For example, commonly used promoters are derived from polyoma, Adenovirus 2, cytomegalovirus and Simian Virus 40.

In another embodiment, the recombinant mammalian expression vector is capable of directing expression of the nucleic acid preferentially in a particular cell type (e.g., tissue-specific regulatory elements are used to express the nucleic acid). Non-limiting examples of suitable tissue-specific promoters include the albumin promoter (liver-specific; Pinkert et al. (1987) *Genes Dev.* 1:268-277), lymphoid-specific promoters (Calame and Eaton (1988) *Adv. Immunol*. 43:235-275), in particular promoters of T cell receptors (Winoto and Baltimore (1989) *EMBO J.* 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *Proc. Natl. Acad. Sci. USA* 86:5473-5477), pancreas-specific promoters (Edlund et al. (1985) *Science* 230:912-916), and mammary gland-specific promoters (e.g., milk whey promoter; U.S. Patent No. 4,873,316 and European Application Publication No. 264,166). Developmentally-regulated promoters are also encompassed, for example, the murine hox promoters (Kessel and Gruss (1990) *Science* 249:374-379) and the α-fetoprotein promoter (Campes and Tilghman (1989) *Genes Dev*. 3:537-546).

The invention further provides a recombinant expression vector comprising a DNA molecule of the invention cloned into the expression vector in an antisense orientation. Regulatory sequences (e.g., viral promoters and/or enhancers) operatively linked to a nucleic acid cloned in the antisense orientation can be chosen which direct the constitutive, tissue specific or cell type specific expression of antisense RNA in a variety of cell types. The antisense expression vector can be in the form of a recombinant plasmid, phagemid or attenuated virus. For a discussion of the regulation of gene expression using antisense genes see Weintraub, H. et al., (1986) Antisense RNA as a molecular tool for genetic analysis, *Reviews - Trends in Genetics* 1:1.

Another aspect the invention provides a host cell which includes a nucleic acid molecule described herein, e.g., a 59914 and 59921 nucleic acid molecule within a recombinant expression vector or a 59914 and 59921 nucleic acid molecule containing sequences which allow it to homologously recombine into a specific site of the host cell's genome. The terms "host cell" and "recombinant host cell" are used interchangeably herein. Such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, 59914 and 59921 proteins can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into host cells via conventional transformation or transfection techniques. As used herein, the terms "transformation" and "transfection" are intended to refer to a variety of art-recognized techniques for introducing foreign nucleic acid (e.g., DNA) into a host cell, including calcium phosphate or calcium chloride co-precipitation, DEAE-dextran-mediated transfection, lipofection, or electroporation.

A host cell of the invention can be used to produce (i.e., express) 59914 and 59921 proteins. Accordingly, the invention further provides methods for producing 59914 and 59921 proteins using the host cells of the invention. In one embodiment, the method includes culturing the host cell of the invention (into which a recombinant expression vector encoding 59914 and 59921 proteins has been introduced) in a suitable medium such that 59914 and 59921 proteins is produced. In another embodiment, the method further includes isolating 59914 and 59921 proteins from the medium or the host cell.

In another aspect, the invention features a cell or a purified preparation of cells which includes 59914 and 59921 transgenes, or which otherwise misexpresses 59914 and 59921. The cell preparation can consist of human or nonhuman cells, e.g., rodent cells, e.g., mouse or rat cells, rabbit cells, or pig cells. In preferred embodiments, the cell, or cells, include 59914 and 59921 transgenes, e.g., a heterologous form of 59914 and 59921, e.g., a gene derived from humans (in the case of a non-human cell). The 59914 and 59921 transgene can be misexpressed, e.g., overexpressed or underexpressed. In other preferred embodiments, the cell, or cells, includes a gene which misexpresses an endogenous 59914 and 59921, e.g., a gene, the expression of which is disrupted, e.g., a knockout. Such cells can serve as a model for studying disorders which are related to mutated or misexpressed 59914 and 59921 alleles or for use in drug screening.

In another aspect, the invention features, a human cell, e.g., a hematopoietic stem cell, transformed with nucleic acid which encodes subject 59914 and 59921 polypeptides.

Also provided are cells, preferably human cells, e.g., human hematopoietic or fibroblast cells, in which endogenous 59914 and 59921 genes are under the control of a regulatory sequence that does not normally control the expression of the endogenous 59914 and 59921 genes. The expression characteristics of an endogenous gene within a cell, e.g., a cell line or microorganism, can be modified by inserting a heterologous DNA regulatory element into the genome of the cell such that the inserted regulatory element is operably linked to the endogenous 59914 and 59921 genes. For example, an endogenous 59914 and 59921 genes which are "transcriptionally silent," e.g., not normally expressed, or expressed only at very low levels, may be activated by inserting a regulatory element which is capable of promoting the expression of a normally expressed gene product in that cell. Techniques such as targeted homologous recombinations, can be used to insert the heterologous DNA as described in, e.g., Chappel, US 5,272,071; WO 91/06667, published in May 16, 1991.

### 1.3 Transgenic Animals

The invention provides non-human transgenic animals. Such animals are useful for studying the function and/or activity of 59914 and 59921 proteins and for identifying and/or evaluating modulators of 59914 and 59921 activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, and the like. A transgene is exogenous DNA or a rearrangement, e.g., a deletion of endogenous chromosomal DNA, which preferably is integrated into or occurs in the genome of the cells of a transgenic animal. A transgene can direct the expression of an encoded gene product in one or more cell types or tissues of the transgenic animal, other transgenes, e.g., a knockout, reduce expression. Thus, a transgenic animal can be one in which endogenous 59914 and 59921 genes have been altered by, e.g., by homologous recombination between the endogenous genes and exogenous DNA molecules introduced into a cell of the animal, e.g., an embryonic cell of the animal, prior to development of the animal.

Intronic sequences and polyadenylation signals can also be included in the transgene to increase the efficiency of expression of the transgene. A tissue-specific regulatory sequence(s) can be operably linked to a transgene of the invention to direct expression of 59914 and 59921 proteins to particular cells. A transgenic founder animal can be identified based upon the presence of a 59914 and 59921 transgene in its genome and/or expression of 59914 and 59921 mRNA in tissues or cells of the animals. A transgenic founder animal can then be used to breed additional animals carrying the transgene. Moreover, transgenic animals carrying a transgene encoding 59914 and 59921 proteins can further be bred to other transgenic animals carrying other transgenes.

59914 and 59921 proteins or polypeptides can be expressed in transgenic animals or plants, e.g., a nucleic acid encoding the protein or polypeptide can be introduced into the genome of an animal. In preferred embodiments the nucleic acid is placed under the control of a tissue specific promoter, e.g., a milk or egg specific promoter, and recovered from the milk or eggs produced by the animal. Suitable animals are mice, pigs, cows, goats, and sheep.

The invention also includes a population of cells from a transgenic animal, as discussed, e.g., herein.

### 1.4 Uses

The nucleic acid molecules, proteins, protein homologues, and antibodies described herein can be used in one or more of the following methods: a) screening assays; b) predictive medicine (e.g., diagnostic assays, prognostic assays, monitoring clinical trials, and pharmacogenetics); and c) methods of treatment (e.g., therapeutic and prophylactic).

The isolated nucleic acid molecules of the invention can be used, for example, to express 59914 and 59921 proteins (e.g., via a recombinant expression vector in a host cell in gene therapy applications), to detect 59914 and 59921 mRNA (e.g., in a biological sample) or a genetic alteration in 59914 and 59921 genes, and to modulate 59914 and 59921 activity, as described further below. The 59914 and 59921 proteins can be used to treat disorders characterized by insufficient or excessive production of a 59914 and 59921 substrate or production of 59914 and 59921 inhibitors. In addition, the 59914 and 59921 proteins can be used to screen for naturally occurring 59914 and 59921 substrates, to screen for drugs or compounds which modulate 59914 and 59921 activity, as well as to treat disorders characterized by insufficient or excessive production of 59914 and 59921 protein or production of 59914 and 59921 protein forms which have decreased, aberrant or unwanted activity compared to 59914 and 59921 wild type protein. Moreover, the anti-59914 and 59921 antibodies of the invention can be used to detect and isolate 59914 and 59921 proteins, regulate the bioavailability of 59914 and 59921 proteins, and modulate 59914 and 59921 activity.

A method of evaluating a compound for the ability to interact with, e.g., bind, a subject 59914 and 59921 polypeptide is provided. The method includes: contacting the compound with the subject 59914 and 59921 polypeptide; and evaluating ability of the compound to interact with, e.g., to bind or form a complex with the subject 59914 and 59921 polypeptide. This method can be performed *in vitro,* e.g., in a cell free system, or *in vivo,* e.g., in a two-hybrid interaction trap assay. This method can be used to identify naturally occurring molecules which interact with subject 59914 and 59921 polypeptide. It can also be used to find natural or synthetic inhibitors of subject 59914 and 59921 polypeptide. Screening methods are discussed in more detail herein.

### 1.5 Screening Assays:

The invention provides methods (also referred to herein as "screening assays") for identifying modulators, i.e., candidate or test compounds or agents (e.g., proteins, peptides, peptidomimetics, peptoids, small molecules or other drugs) which bind to 59914 and 59921 proteins, have a stimulatory or inhibitory effect on, for example, 59914 and 59921 expression or 59914 and 59921 activity, or have a stimulatory or inhibitory effect on, for example, the expression or activity of a 59914 and 59921 substrate. Compounds thus identified can be used to modulate the activity of target gene products (e.g., 59914 and 59921 genes) in a therapeutic protocol, to elaborate the biological function of the target gene product, or to identify compounds that disrupt normal target gene interactions.

In one embodiment, the invention provides assays for screening candidate or test compounds which are substrates of 59914 and 59921 proteins or polypeptides or a biologically active portion thereof. In another embodiment, the invention provides assays for screening candidate or test compounds which bind to or modulate the activity of 59914 and 59921 proteins or polypeptides or a biologically active portion thereof.

The test compounds of the present invention can be obtained using any of the numerous approaches in combinatorial library methods known in the art, including: biological libraries; peptoid libraries (libraries of molecules having the functionalities of peptides, but with a novel, non-peptide backbone which are resistant to enzymatic degradation but which nevertheless remain bioactive; see, e.g., Zuckermann, R.N. et al. (1994) *J. Med. Chem*. 37:2678-85); spatially addressable parallel solid phase or solution phase libraries; synthetic library methods requiring deconvolution; the 'one-bead one-compound' library method; and synthetic library methods using affinity chromatography selection. The biological library and peptoid library approaches are limited to peptide libraries, while the other four approaches are applicable to peptide, non-peptide oligomer or small molecule libraries of compounds (Lam, K.S. (1997) *Anticancer Drug Des*. 12:145).

Examples of methods for the synthesis of molecular libraries can be found in the art, for example in: DeWitt et al. (1993) *Proc. Natl. Acad. Sci. U.S.A*. 90:6909; Erb et al. (1994) *Proc. Natl. Acad. Sci. USA* 91:11422; Zuckermann et al. (1994). *J. Med. Chem*. 37:2678; Cho et al. (1993) *Science* 261:1303; Carrell et al. (1994) *Angew. Chem. Int. Ed. Engl*. 33:2059; Carell et al. (1994) *Angew. Chem. Int. Ed. Engl*. 33:2061; and in Gallop et al. (1994) *J. Med. Chem*. 37:1233.Libraries of compounds may be presented in solution (e.g., Houghten (1992) *Biotechniques* 13:412-421), or on beads (Lam (1991) *Nature* 354:82-84), chips (Fodor (1993) *Nature* 364:555-556), bacteria (Ladner, USP 5,223,409), spores (Ladner USP '409), plasmids (Cull et al. (1992) *Proc Natl Acad Sci USA* 89:1865-1869) or on phage (Scott and Smith (1990) *Science* 249:386-390; Devlin (1990) *Science* 249:404-406; Cwirla et al. (1990) *Proc. Natl. Acad. Sci.* 87:6378-6382; Felici (1991) *J. Mol. Biol.* 222:301-310; Ladner *supra*.).

In one embodiment, an assay is a cell-based assay in which a cell which expresses 59914 and 59921 proteins or biologically active portions thereof are contacted with a test compound, and the ability of the test compound to modulate 59914 and 59921 activity is determined. Determining the ability of the test compound to modulate 59914 and 59921 activity can be accomplished by monitoring, for example, (i) the interaction of 59914 and 59921 proteins with a 59914 and 59921 target molecule; (ii) the interaction of 59914 and 59921 proteins with a 59914 and 59921 target molecule, wherein the 59914 and 59921 target is a choline or choline metabolite (or compound of which choline is a component or precursor) substrate, e.g., choline metabolites or synthesized compounds which 59914 and 59921 proteins can transport across membranes. The cell, for example, can be of mammalian origin, e.g., human.

The ability of the test compound to modulate 59914 and 59921 binding to a compound, e.g., a 59914 and 59921 substrate, or to bind to 59914 and 59921 can also be evaluated. This can be accomplished, for example, by coupling the compound, e.g., the substrate, with a radioisotope or enzymatic label such that binding of the compound, e.g., the substrate, to 59914 and 59921 can be determined by detecting the labeled compound, e.g., substrate, in a complex. Alternatively, 59914 and 59921 could be coupled with a radioisotope or enzymatic label to monitor the ability of a test compound to modulate 59914 and 59921 binding to a 59914 and 59921 substrate in a complex. For example, compounds (e.g., 59914 and 59921 substrates) can be labeled with ¹²⁵I, ³⁵S, ¹⁴C, or ³H, either directly or indirectly, and the radioisotope detected by direct counting of radioemmission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

The ability of a compound (e.g., a 59914 and 59921 substrate) to interact with 59914 and 59921, with or without the labeling of any of the interactants, can be evaluated. For example, a microphysiometer can be used to detect the interaction of a compound with 59914 and 59921 without the labeling of either the compound or the 59914 and 59921. McConnell, H. M. et al. (1992) Science 257:1906-1912. As used herein, a "microphysiometer" (e.g., Cytosensor) is an analytical instrument that measures the rate at which a cell acidifies its environment using a light-addressable potentiometric sensor (LAPS). Changes in this acidification rate can be used as an indicator of the interaction between a compound and 59914 and 59921.

In yet another embodiment, a cell-free assay is provided in which 59914 and 59921 proteins, or biologically active portion thereof, is contacted with a test compound and the ability of the test compound to bind to the 59914 and 59921 protein, or biologically active portion thereof, is evaluated. Preferred biologically active portions of the 59914 and 59921 proteins to be used in assays of the present invention include fragments which participate in interactions with non-59914 and 59921 molecules, e.g., fragments with high surface probability scores.

Soluble and/or membrane-bound forms of isolated proteins (e.g., 59914 and 59921 proteins, or biologically active portions thereof) can be used in the cell-free assays of the invention. When membrane-bound forms of the protein are used, it may be desirable to utilize a solubilizing agent. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit®, Isotridecypoly(ethylene glycol ether)ₙ, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl=N,N-dimethyl-3-ammonio-1-propane sulfonate.

Cell-free assays involve preparing a reaction mixture of the target gene protein and the test compound under conditions and for a time sufficient to allow the two components to interact and bind, thus forming a complex that can be removed and/or detected.

The interaction between two molecules can also be detected, e.g., using fluorescence energy transfer (FET) (see, for example, Lakowicz et al., U.S. Patent No. 5,631,169; Stavrianopoulos, et al., U.S. Patent No. 4,868,103). A fluorophore label on the first, 'donor' molecule is selected such that its emitted fluorescent energy will be absorbed by a fluorescent label on a second, 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, the spatial relationship between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determining the ability of the 59914 and 59921 protein to bind to a target molecule can be accomplished using real-time Biomolecular Interaction Analysis (BIA) (see, e.g., Sjolander, S. and Urbaniczky, C. (1991) *Anal. Chem*. 63:2338-2345 and Szabo et al. (1995) *Curr. Opin. Struct. Biol*. 5:699-705). "Surface plasmon resonance" or "BIA" detects biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

In one embodiment, the target gene product or the test substance is anchored onto a solid phase. The target gene product/test compound complexes anchored on the solid phase can be detected at the end of the reaction. Preferably, the target gene product can be anchored onto a solid surface, and the test compound, (which is not anchored), can be labeled, either directly or indirectly, with detectable labels discussed herein.

It may be desirable to immobilize 59914 and 59921, an anti-59914 and 59921 antibody, or a 59914 and 59921 target molecule to facilitate separation of complexed from uncomplexed forms of one or both of the proteins, as well as to accommodate automation of the assay. Binding of a test compound to 59914 and 59921 proteins, or interaction of 59914 and 59921 proteins with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that allows one or both of the proteins to be bound to a matrix. For example, glutathione-S-transferase/59914 and 59921 fusion proteins or glutathione-S-transferase/target fusion proteins can be adsorbed onto glutathione sepharose beads (Sigma Chemical, St. Louis, MO) or glutathione derivatized microtiter plates, which are then combined with the test compound or the test compound and either the non-adsorbed target protein or 59914 and 59921 protein, and the mixture incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described above. Alternatively, the complexes can be dissociated from the matrix, and the level of 59914 and 59921 binding or activity determined using standard techniques.

Other techniques for immobilizing either 59914 and 59921 proteins or a target molecule on matrices include using conjugation of biotin and streptavidin. Biotinylated 59914 and 59921 protein or target molecules can be prepared from biotin-NHS (N-hydroxy-succinimide) using techniques known in the art (e.g., biotinylation kit, Pierce Chemicals, Rockford, IL), and immobilized in the wells of streptavidin-coated 96 well plates (Pierce Chemical).

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously non-immobilized component is pre-labeled, the detection of label immobilized on the surface indicates that complexes were formed. Where the previously non-immobilized component is not pre-labeled, an indirect label can be used to detect complexes anchored on the surface; e.g., using a labeled antibody specific for the immobilized component (the antibody, in turn, can be directly labeled or indirectly labeled with, e.g., a labeled anti-Ig antibody).

In one embodiment, this assay is performed utilizing antibodies reactive with 59914 and 59921 protein or target molecules but which do not interfere with binding of the 59914 and 59921 protein to its target molecule. Such antibodies can be derivatized to the wells of the plate, and unbound target or 59914 and 59921 protein trapped in the wells by antibody conjugation. Methods for detecting such complexes, in addition to those described above for the GST-immobilized complexes, include immunodetection of complexes using antibodies reactive with the 59914 and 59921 protein or target molecule, as well as enzyme-linked assays which rely on detecting an enzymatic activity associated with the 59914 and 59921 protein or target molecule.

Alternatively, cell-free assays can be conducted in a liquid phase. In such an assay, the reaction products are separated from unreacted components, by any of a number of standard techniques, including but not limited to: differential centrifugation (see, for example, Rivas, G., and Minton, A.P., (1993) *Trends Biochem Sci* 18:284-7); chromatography (gel filtration chromatography, ion-exchange chromatography); electrophoresis (see, e.g., Ausubel, F. et al., eds. Current Protocols in Molecular Biology 1999, J. Wiley: New York.); and immunoprecipitation (see, for example, Ausubel, F. et al., eds. (1999) *Current Protocols in Molecular Biology*, J. Wiley: New York). Such resins and chromatographic techniques are known to one skilled in the art (see, e.g., Heegaard, N.H., (1998) *J Mol Recognit* 11:141-8; Hage, D.S., and Tweed, S.A. (1997) *J Chromatogr B Biomed Sci Appl.* 699:499-525). Further, fluorescence energy transfer may also be conveniently utilized, as described herein, to detect binding without further purification of the complex from solution.

In a preferred embodiment, the assay includes contacting the 59914 and 59921 protein, or biologically active portion thereof, with a known compound which binds 59914 and 59921 to form an assay mixture, contacting the assay mixture with a test compound, and determining the ability of the test compound to interact with 59914 and 59921 proteins, wherein determining the ability of the test compound to interact with 59914 and 59921 proteins includes determining the ability of the test compound to preferentially bind to 59914 and 59921, or biologically active portion thereof, or to modulate the activity of a target molecule, as compared to the known compound.

The target gene products of the invention can, *in vivo*, interact with one or more cellular or extracellular macromolecules, such as proteins. For the purposes of this discussion, such cellular and extracellular macromolecules are referred to herein as "binding partners." Compounds that disrupt such interactions can be useful in regulating the activity of the target gene product. Such compounds can include, but are not limited to, molecules such as antibodies, peptides, and small molecules. The preferred target genes/products for use in this embodiment are the 59914 and 59921 genes herein identified. In an alternative embodiment, the invention provides methods for determining the ability of the test compound to modulate the activity of 59914 and 59921 proteins through modulation of the activity of a downstream effector of a 59914 and 59921 target molecule. For example, the activity of the effector molecule on an appropriate target can be determined, or the binding of the effector to an appropriate target can be determined, as previously described.

To identify compounds that interfere with the interaction between the target gene product and its cellular or extracellular binding partner(s), a reaction mixture containing the target gene product and the binding partner is prepared, under conditions and for a time sufficient, to allow the two products to form complex. In order to test an inhibitory agent, the reaction mixture is provided in the presence and absence of the test compound. The test compound can be initially included in the reaction mixture, or can be added at a time subsequent to the addition of the target gene and its cellular or extracellular binding partner. Control reaction mixtures are incubated without the test compound or with a placebo. The formation of any complexes between the target gene product and the cellular or extracellular binding partner is then detected. The formation of a complex in the control reaction, but not in the reaction mixture containing the test compound, indicates that the compound interferes with the interaction of the target gene product and the interactive binding partner. Additionally, complex formation within reaction mixtures containing the test compound and normal target gene product can also be compared to complex formation within reaction mixtures containing the test compound and mutant target gene product. This comparison can be important in those cases wherein it is desirable to identify compounds that disrupt interactions of mutant but not normal target gene products.

These assays can be conducted in a heterogeneous or homogeneous format. Heterogeneous assays involve anchoring either the target gene product or the binding partner onto a solid phase, and detecting complexes anchored on the solid phase at the end of the reaction. In homogeneous assays, the entire reaction is carried out in a liquid phase. In either approach, the order of addition of reactants can be varied to obtain different information about the compounds being tested. For example, test compounds that interfere with the interaction between the target gene products and the binding partners, e.g., by competition, can be identified by conducting the reaction in the presence of the test substance. Alternatively, test compounds that disrupt preformed complexes, e.g., compounds with higher binding constants that displace one of the components from the complex, can be tested by adding the test compound to the reaction mixture after complexes have been formed. The various formats are briefly described herein.

In a heterogeneous assay system, either the target gene product or the interactive cellular or extracellular binding partner, is anchored onto a solid surface (e.g., a microtiter plate), while the non-anchored species is labeled, either directly or indirectly. The anchored species can be immobilized by noncovalent or covalent attachments. Alternatively, an immobilized antibody specific for the species to be anchored can be used to anchor the species to the solid surface.

In order to conduct the assay, the partner of the immobilized species is exposed to the coated surface with or without the test compound. After the reaction is complete, unreacted components are removed (e.g., by washing) and any complexes formed will remain immobilized on the solid surface. Where the non-immobilized species is pre-labeled, the detection of label immobilized on the surface indicates that complexes were formed. Where the non-immobilized species is not pre-labeled, an indirect label can be used to detect complexes anchored on the surface; e.g., using a labeled antibody specific for the initially non-immobilized species (the antibody, in turn, can be directly labeled or indirectly labeled with, e.g., a labeled anti-Ig antibody). Depending upon the order of addition of reaction components, test compounds that inhibit complex formation or that disrupt preformed complexes can be detected.

Alternatively, the reaction can be conducted in a liquid phase in the presence or absence of the test compound, the reaction products separated from unreacted components, and complexes detected; e.g., using an immobilized antibody specific for one of the binding components to anchor any complexes formed in solution, and a labeled antibody specific for the other partner to detect anchored complexes. Again, depending upon the order of addition of reactants to the liquid phase, test compounds that inhibit complex or that disrupt preformed complexes can be identified.

In an alternate embodiment of the invention, a homogeneous assay can be used. For example, a preformed complex of the target gene product and the interactive cellular or extracellular binding partner product is prepared in that either the target gene products or their binding partners are labeled, but the signal generated by the label is quenched due to complex formation (see, e.g., U.S. Patent No. 4,109,496 that utilizes this approach for immunoassays). The addition of a test substance that competes with and displaces one of the species from the preformed complex will result in the generation of a signal above background. In this way, test substances that disrupt target gene product-binding partner interaction can be identified.

In yet another aspect, the 59914 and 59921 proteins can be used as "bait proteins" in a two-hybrid assay or three-hybrid assay (see, e.g., U.S. Patent No. 5,283,317; Zervos et al. (1993) *Cell* 72:223-232; Madura et al. (1993) *J. Biol. Chem*. 268:12046-12054; Bartel et al. (1993) *Biotechniques* 14:920-924; Iwabuchi et al. (1993) *Oncogene* 8:1693-1696; and Brent WO94/10300), to identify other proteins, which bind to or interact with 59914 and 59921 ("59914 and 59921-binding proteins" or "59914 and 59921-bp") and are involved in 59914 and 59921 activity. Such 59914 and 59921-bps can be activators or inhibitors of signals by the 59914 and 59921 proteins or 59914 and 59921 targets as, for example, downstream elements of a 59914 and 59921-mediated signaling pathway.

The two-hybrid system is based on the modular nature of most transcription factors, which consist of separable DNA-binding and activation domains. Briefly, the assay utilizes two different DNA constructs. In one construct, the gene that codes for 59914 and 59921 proteins is fused to a gene encoding the DNA binding domain of a known transcription factor (e.g., GAL-4). In the other construct, a DNA sequence, from a library of DNA sequences, that encodes an unidentified protein ("prey" or "sample") is fused to a gene that codes for the activation domain of the known transcription factor. (Alternatively the: 59914 and 59921 protein can be the fused to the activator domain.) If the "bait" and the "prey" proteins are able to interact, *in vivo,* forming a 59914 and 59921-dependent complex, the DNA-binding and activation domains of the transcription factor are brought into close proximity. This proximity allows transcription of a reporter gene (e.g., lacZ) which is operably linked to a transcriptional regulatory site responsive to the transcription factor. Expression of the reporter gene can be detected and cell colonies containing the functional transcription factor can be isolated and used to obtain the cloned gene which encodes the protein which interacts with the 59914 and 59921 protein.

In another embodiment, modulators of 59914 and 59921 expression are identified. For example, a cell or cell free mixture is contacted with a candidate compound and the expression of 59914 and 59921 mRNA or protein evaluated relative to the level of expression of 59914 and 59921 mRNA or protein in the absence of the candidate compound. When expression of 59914 and 59921 mRNA or protein is greater in the presence of the candidate compound than in its absence, the candidate compound is identified as a stimulator of 59914 and 59921 mRNA or protein expression. Alternatively, when expression of 59914 and 59921 mRNA or protein is less (statistically significantly less) in the presence of the candidate compound than in its absence, the candidate compound is identified as an inhibitor of 59914 and 59921 mRNA or protein expression. The level of 59914 and 59921 mRNA or protein expression can be determined by methods described herein for detecting 59914 and 59921 mRNA or protein.

In another aspect, the invention pertains to a combination of two or more of the assays described herein. For example, a modulating agent can be identified using a cell-based or a cell free assay, and the ability of the agent to modulate the activity of 59914 and 59921 proteins can be confirmed *in vivo* in an animal model.

This invention further pertains to novel agents identified by the above-described screening assays. Accordingly, it is within the scope of this invention to further use an agent identified as described herein (e.g., a 59914 and 59921 modulating agent, an anti-sense 59914 and 59921 nucleic acid molecule, a 59914 and 59921-specific antibody, or a 59914 and 59921-binding partner) in an appropriate animal model to determine the efficacy, toxicity, side effects, or mechanism of action, of treatment with such an agent. Furthermore, novel agents identified by the above-described screening assays can be used for treatments as described herein.

### 1.6 Detection Assays

Portions or fragments of the nucleic acid sequences identified herein can be used as polynucleotide reagents. For example, these sequences can be used to: (i) map their respective genes on a chromosome e.g., to locate gene regions associated with genetic disease or to associate 59914 and 59921 with a disease; (ii) identify an individual from a minute biological sample (tissue typing); and (iii) aid in forensic identification of a biological sample. These applications are described in the subsections below.

### 1.7 Chromosome Mapping

The 59914 and 59921 nucleotide sequences or portions thereof can be used to map the location of the 59914 and 59921 genes on a chromosome. This process is called chromosome mapping. Chromosome mapping is useful in correlating the 59914 and 59921 sequences with genes associated with disease. For example, in one embodiment, 59921 maps to chromosomal position 1p21.3-22.3, based on at least several regions of homology to a clone in the art known to map to chromosomal position 1p21.3-22.3 (clone RP4-639P13, Genbank accession number AL359554), as described herein. Among the genes found in this region is a cholinergic receptor (Online Mendelian Inheritance in Man (OMIM) Accession No. 118507 (see http://www.ncbi.nlm.nih.gov/entrez/dispomim.cgi?id=118507), and among the disorders associated with this region are, myoadenylate deaminase deficiency, epilepsy, Waardenburg syndrome, type 2B, stickler syndrome type II, glycogen storage disease IIIa, and osteoporosis (autosomal dominant, type II), which 59914 and 59921 nucleotides, amino acids, and modulators thereof can be used to treat.

Briefly, 59914 and 59921 genes can be mapped to chromosomes by preparing PCR primers (preferably 15-25 bp in length) from the 59914 and 59921 nucleotide sequences. These primers can then be used for PCR screening of somatic cell hybrids containing individual human chromosomes. Only those hybrids containing the human gene corresponding to the 59914 and 59921 sequences will yield an amplified fragment.

A panel of somatic cell hybrids in which each cell line contains either a single human chromosome or a small number of human chromosomes, and a full set of mouse chromosomes, can allow easy mapping of individual genes to specific human chromosomes. (D'Eustachio P. et al. (1983) *Science* 220:919-924).

Other mapping strategies e.g., *in situ* hybridization (described in Fan, Y. et al. (1990) *Proc. Natl. Acad. Sci. USA*, 87:6223-27), pre-screening with labeled flow-sorted chromosomes, and pre-selection by hybridization to chromosome specific cDNA libraries can be used to map 59914 and 59921 to a chromosomal location.

Fluorescence *in situ* hybridization (FISH) of a DNA sequence to a metaphase chromosomal spread can further be used to provide a precise chromosomal location in one step. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases will suffice to get good results at a reasonable amount of time. For a review of this technique, see Verma et al., Human Chromosomes: A Manual of Basic Techniques ((1988) Pergamon Press, New York).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. (Such data are found, for example, in V. McKusick, Mendelian Inheritance in Man, available on-line through Johns Hopkins University Welch Medical Library). The relationship between a gene and a disease, mapped to the same chromosomal region, can then be identified through linkage analysis (co-inheritance of physically adjacent genes), described in, for example, Egeland, J. et al. (1987) *Nature,* 325:783-787.

Moreover, differences in the DNA sequences between individuals affected and unaffected with a disease associated with the 59914 and 59921 gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### 1.8 Tissue Typing

59914 and 59921 sequences can be used to identify individuals from biological samples using, e.g., restriction fragment length polymorphism (RFLP). In this technique, an individual's genomic DNA is digested with one or more restriction enzymes, the fragments separated, e.g., in a Southern blot, and probed to yield bands for identification. The sequences of the present invention are useful as additional DNA markers for RFLP (described in U.S. Patent 5,272,057).

Furthermore, the sequences of the present invention can also be used to determine the actual base-by-base DNA sequence of selected portions of an individual's genome. Thus, the 59914 and 59921 nucleotide sequences described herein can be used to prepare two PCR primers from the 5' and 3' ends of the sequences. These primers can then be used to amplify an individual's DNA and subsequently sequence it. Panels of corresponding DNA sequences from individuals, prepared in this manner, can provide unique individual identifications, as each individual will have a unique set of such DNA sequences due to allelic differences.

Allelic variation occurs to some degree in the coding regions of these sequences, and to a greater degree in the noncoding regions. Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences of SEQ ID NO:1 and SEQ ID NO:4 can provide positive individual identification with a panel of perhaps 10 to 1,000 primers which each yield a noncoding amplified sequence of 100 bases. If coding sequences, such as those in SEQ ID NO:3 and SEQ ID NO:6 are used, a more appropriate number of primers for positive individual identification would be 500-2,000.

If a panel of reagents from 59914 and 59921 nucleotide sequences described herein is used to generate a unique identification database for an individual, those same reagents can later be used to identify tissue from that individual. Using the unique identification database, positive identification of the individual, living or dead, can be made from extremely small tissue samples.

### 1.9 Use of Partial 59914 and 59921 Sequences in Forensic Biology

DNA-based identification techniques can also be used in forensic biology. To make such an identification, PCR technology can be used to amplify DNA sequences taken from very small biological samples such as tissues, e.g., hair or skin, or body fluids, e.g., blood, saliva, or semen found at a crime scene. The amplified sequence can then be compared to a standard, thereby allowing identification of the origin of the biological sample.

The sequences of the present invention can be used to provide polynucleotide reagents, e.g., PCR primers, targeted to specific loci in the human genome, which can enhance the reliability of DNA-based forensic identifications by, for example, providing another "identification marker" (i.e. another DNA sequence that is unique to a particular individual). As mentioned above, actual base sequence information can be used for identification as an accurate alternative to patterns formed by restriction enzyme generated fragments. Sequences targeted to noncoding regions of SEQ ID NO:1 and SEQ ID NO:4 (e.g., fragments derived from the noncoding regions of SEQ ID NO:1 and SEQ ID NO:4 having a length of at least 20 bases, preferably at least 30 bases) are particularly appropriate for this use.

The 59914 and 59921 nucleotide sequences described herein can further be used to provide polynucleotide reagents, e.g., labeled or labelable probes which can be used in, for example, an *in situ* hybridization technique, to identify a specific tissue. This can be very useful in cases where a forensic pathologist is presented with a tissue of unknown origin. Panels of such 59914 and 59921 probes can be used to identify tissue by species and/or by organ type.

In a similar fashion, these reagents, e.g., 59914 and 59921 primers or probes can be used to screen tissue culture for contamination (i.e. screen for the presence of a mixture of different types of cells in a culture).

### 1.10 Predictive Medicine

The present invention also pertains to the field of predictive medicine in which diagnostic assays, prognostic assays, and monitoring clinical trials are used for prognostic (predictive) purposes to thereby treat an individual.

Generally, the invention provides, a method of determining if a subject is at risk for a disorder related to a lesion in or the misexpression of a gene which encodes 59914 and 59921.

Such disorders include, e.g., a disorder associated with the misexpression of 59914 and 59921 genes.

The method includes one or more of the following:
detecting, in a tissue of the subject, the presence or absence of a mutation which affects the expression of the 59914 and 59921 genes, or detecting the presence or absence of a mutation in a region which controls the expression of the genes, e.g., a mutation in the 5' control region;
detecting, in a tissue of the subject, the presence or absence of a mutation which alters the structure of the 59914 and 59921 genes;
detecting, in a tissue of the subject, the misexpression of the 59914 and 59921 genes, at the mRNA level, e.g., detecting a non-wild type level of a mRNA; or
detecting, in a tissue of the subject, the misexpression of the genes, at the protein level, e.g., detecting a non-wild type level of a 59914 and 59921 polypeptides.

In preferred embodiments the method includes: ascertaining the existence of at least one of: a deletion of one or more nucleotides from the 59914 and 59921 genes; an insertion of one or more nucleotides into the gene, a point mutation, e.g., a substitution of one or more nucleotides of the gene, a gross chromosomal rearrangement of the gene, e.g., a translocation, inversion, or deletion.

For example, detecting the genetic lesion can include: (i) providing a probe/primer including an oligonucleotide containing a region of nucleotide sequence which hybridizes to a sense or antisense sequence from SEQ ID NO:1 and SEQ ID NO:4, or naturally occurring mutants thereof or 5' or 3' flanking sequences naturally associated with the 59914 and 59921 genes; (ii) exposing the probe/primer to nucleic acid of the tissue; and detecting, by hybridization, e.g., *in situ* hybridization, of the probe/primer to the nucleic acid, the presence or absence of the genetic lesion.

In preferred embodiments detecting the misexpression includes ascertaining the existence of at least one of: an alteration in the level of a messenger RNA transcript of the 59914 and 59921 genes; the presence of a non-wild type splicing pattern of a messenger RNA transcript of the gene; or a non-wild type level of 59914 and 59921.

Methods of the invention can be used prenatally or to determine if a subject's offspring will be at risk for a disorder.

In preferred embodiments the method includes determining the structure of 59914 and 59921 genes, an abnormal structure being indicative of risk for the disorder.

In preferred embodiments the method includes contacting a sample from the subject with an antibody to the 59914 and 59921 proteins or nucleic acids, which hybridizes specifically with the genes. These and other embodiments are discussed below.

### 1.11 Diagnostic and Prognostic Assays

The presence, level, or absence of 59914 and 59921 proteins or nucleic acids in a biological sample can be evaluated by obtaining a biological sample from a test subject and contacting the biological sample with a compound or an agent capable of detecting 59914 and 59921 proteins or nucleic acids (e.g., mRNA, genomic DNA) that encodes 59914 and 59921 proteins such that the presence of 59914 and 59921 proteins or nucleic acids are detected in the biological sample. The term "biological sample" includes tissues, cells and biological fluids isolated from a subject, as well as tissues, cells and fluids present within a subject. A preferred biological sample is serum. The level of expression of the 59914 and 59921 genes can be measured in a number of ways, including, but not limited to: measuring the mRNA encoded by the 59914 and 59921 genes; measuring the amount of protein encoded by the 59914 and 59921 genes; or measuring the activity of the protein encoded by the 59914 and 59921 genes.

The level of mRNA corresponding to 59914 and 59921 genes in a cell can be determined both by *in situ* and by *in vitro* formats.

The isolated mRNA can be used in hybridization or amplification assays that include, but are not limited to, Southern or Northern analyses, polymerase chain reaction analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, for example, full-length 59914 and 59921 nucleic acids, such as the nucleic acid of SEQ ID NO:1 and SEQ ID NO:4, or a portion thereof, such as an oligonucleotide of at least 7, 15, 30, 50, 100, 250 or 500 nucleotides in length and sufficient to specifically hybridize under stringent conditions to 59914 and 59921 mRNA or genomic DNA. Other suitable probes for use in the diagnostic assays are described herein.

In one format, mRNA (or cDNA) is immobilized on a surface and contacted with the probes, for example by running the isolated mRNA on an agarose gel and transferring the mRNA from the gel to a membrane, such as nitrocellulose. In an alternative format, the probes are immobilized on a surface and the mRNA (or cDNA) is contacted with the probes, for example, in a two-dimensional gene chip array. A skilled artisan can adapt known mRNA detection methods for use in detecting the level of mRNA encoded by the 59914 and 59921 genes.

The level of mRNA in a sample that is encoded by one of 59914 and 59921 can be evaluated with nucleic acid amplification, e.g., by rtPCR (Mullis (1987) U.S. Patent No. 4,683,202), ligase chain reaction (Barany (1991) *Proc. Natl. Acad. Sci. USA* 88:189-193), self sustained sequence replication (Guatelli et al., (1990) *Proc. Natl. Acad. Sci. USA* 87:1874-1878), transcriptional amplification system (Kwoh et al., (1989), *Proc. Natl. Acad. Sci. USA* 86:1173-1177), Q-Beta Replicase (Lizardi et al., (1988) *Bio*/*Technology* 6:1197), rolling circle replication (Lizardi et al., U.S. Patent No. 5,854,033) or any other nucleic acid amplification method, followed by the detection of the amplified molecules using techniques known in the art. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, a cell or tissue sample can be prepared/processed and immobilized on a support, typically a glass slide, and then contacted with a probe that can hybridize to mRNA that encodes the 59914 and 59921 gene being analyzed.

In another embodiment, the methods further contacting a control sample with a compound or agent capable of detecting 59914 and 59921 mRNA, or genomic DNA, and comparing the presence of 59914 and 59921 mRNA or genomic DNA in the control sample with the presence of 59914 and 59921 mRNA or genomic DNA in the test sample.

A variety of methods can be used to determine the level of protein encoded by 59914 and 59921. In general, these methods include contacting an agent that selectively binds to the protein, such as an antibody with a sample, to evaluate the level of protein in the sample. In a preferred embodiment, the antibody bears a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling (i.e., physically linking) a detectable substance to the probe or antibody, as well as indirect labeling of the probe or antibody by reactivity with a detectable substance. Examples of detectable substances are provided herein.

The detection methods can be used to detect 59914 and 59921 proteins in a biological sample *in vitro*, as well as *in vivo*. *In vitro* techniques for detection of 59914 and 59921 proteins include enzyme linked immunosorbent assays (ELISAs), immunoprecipitations, immunofluorescence, enzyme immunoassay (EIA), radioimmunoassay (RIA), and Western blot analysis. *In vivo* techniques for detection of 59914 and 59921 proteins include introducing into a subject a labeled anti-59914 and 59921 antibody. For example, the antibody can be labeled with a radioactive marker whose presence and location in a subject can be detected by standard imaging techniques.

In another embodiment, the methods further include contacting the control sample with a compound or agent capable of detecting 59914 and 59921 proteins, and comparing the presence of 59914 and 59921 proteins in the control sample with the presence of 59914 and 59921 proteins in the test sample.

The invention also includes kits for detecting the presence of 59914 and 59921 in a biological sample. For example, the kit can include a compound or agent capable of detecting 59914 and 59921 proteins or mRNA in a biological sample; and a standard. The compound or agent can be packaged in a suitable container. The kit can further comprise instructions for using the kit to detect 59914 and 59921 proteins or nucleic acids.

For antibody-based kits, the kit can include: (1) a first antibody (e.g., attached to a solid support) which binds to a polypeptide corresponding to a marker of the invention; and, optionally, (2) a second, different antibody which binds to either the polypeptide or the first antibody and is conjugated to a detectable agent.

For oligonucleotide-based kits, the kit can include: (1) an oligonucleotide, e.g., a detectably labeled oligonucleotide, which hybridizes to a nucleic acid sequence encoding a polypeptide corresponding to a marker of the invention, or (2) a pair of primers useful for amplifying a nucleic acid molecule corresponding to a marker of the invention. The kit can also includes a buffering agent, a preservative, or a protein stabilizing agent. The kit can also includes components necessary for detecting the detectable agent (e.g., an enzyme or a substrate). The kit can also contain a control sample or a series of control samples which can be assayed and compared to the test sample contained. Each component of the kit can be enclosed within an individual container and all of the various containers can be within a single package, along with instructions for interpreting the results of the assays performed using the kit.

The diagnostic methods described herein can identify subjects having, or at risk of developing, a disease or disorder associated with misexpressed or aberrant or unwanted 59914 and 59921 expression or activity. As used interchangeably herein, the terms "unwanted" and "undesirable" include an unwanted phenomenon involved in a biological response such as pain or deregulated cell proliferation.

In one embodiment, a disease or disorder associated with aberrant or unwanted 59914 and 59921 expression or activity is identified. A test sample is obtained from a subject and 59914 and 59921 proteins or nucleic acids (e.g., mRNA or genomic DNA) is evaluated, wherein the level, e.g., the presence or absence, of 59914 and 59921 proteins or nucleic acid is diagnostic for a subject having or at risk of developing a disease or disorder associated with aberrant or unwanted 59914 and 59921 expression or activity. As used herein, a "test sample" refers to a biological sample obtained from a subject of interest, including a biological fluid (e.g., serum), cell sample, or tissue.

The prognostic assays described herein can be used to determine whether a subject can be administered an agent (e.g., an agonist, antagonist, peptidomimetic, protein, peptide, nucleic acid, small molecule, or other drug candidate) to treat a disease or disorder associated with aberrant or unwanted 59914 and 59921 expression or activity. For example, such methods can be used to determine whether a subject can be effectively treated with an agent for a cellular proliferative and/or differentiative disorder, a hormonal disorder, an immune or inflammatory disorder, a neurological disorder, a cardiovascular disorder, a blood vessel disorder, or a platelet disorder.

The methods of the invention can also be used to detect genetic alterations in 59914 and 59921 genes, thereby determining if a subject with the altered gene is at risk for a disorder characterized by misregulation in 59914 and 59921 protein activity or nucleic acid expression, such as a cellular proliferative and/or differentiative disorder, a hormonal disorder, an immune or inflammatory disorder, a neurological disorder, a cardiovascular disorder, a blood vessel disorder, or a platelet disorder. In preferred embodiments, the methods include detecting, in a sample from the subject, the presence or absence of a genetic alteration characterized by at least one of an alteration affecting the integrity of a gene encoding 59914 and 59921 proteins, or the misexpression of the 59914 and 59921 genes. For example, such genetic alterations can be detected by ascertaining the existence of at least one of 1) a deletion of one or more nucleotides from 59914 and 59921 genes; 2) an addition of one or more nucleotides to 59914 and 59921 genes; 3) a substitution of one or more nucleotides of 59914 and 59921 genes, 4) a chromosomal rearrangement of 59914 and 59921 genes; 5) an alteration in the level of a messenger RNA transcript of 59914 and 59921 genes, 6) aberrant modification of 59914 and 59921 genes, such as of the methylation pattern of the genomic DNA, 7) the presence of a non-wild type splicing pattern of a messenger RNA transcript of 59914 and 59921 genes, 8) a non-wild type level of 59914 and 59921 proteins, 9) allelic loss of 59914 and 59921 genes, and 10) inappropriate post-translational modification of 59914 and 59921 proteins.

An alteration can be detected without a probe/primer in a polymerase chain reaction, such as anchor PCR or RACE PCR, or, alternatively, in a ligation chain reaction (LCR), the latter of which can be particularly useful for detecting point mutations in the 59914 and 59921 gene. This method can include the steps of collecting a sample of cells from a subject, isolating nucleic acid (e.g., genomic, mRNA or both) from the sample, contacting the nucleic acid sample with one or more primers which specifically hybridize to 59914 and 59921 genes under conditions such that hybridization and amplification of the 59914 and 59921 gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein. Alternatively, other amplification methods described herein or known in the art can be used.

In another embodiment, mutations in 59914 and 59921 genes from a sample cell can be identified by detecting alterations in restriction enzyme cleavage patterns. For example, sample and control DNA is isolated, amplified (optionally), digested with one or more restriction endonucleases, and fragment length sizes are determined, e.g., by gel electrophoresis and compared. Differences in fragment length sizes between sample and control DNA indicates mutations in the sample DNA. Moreover, the use of sequence specific ribozymes (see, for example, U.S. Patent No. 5,498,531) can be used to score for the presence of specific mutations by development or loss of a ribozyme cleavage site.

In other embodiments, genetic mutations in 59914 and 59921 can be identified by hybridizing a sample and control nucleic acids, e.g., DNA or RNA, two dimensional arrays, e.g., chip based arrays. Such arrays include a plurality of addresses, each of which is positionally distinguishable from the other. A different probe is located at each address of the plurality. The arrays can have a high density of addresses, e.g., can contain hundreds or thousands of oligonucleotides probes (Cronin, M.T. et al. (1996) *Human Mutation* 7: 244-255; Kozal, M.J. et al. (1996) *Nature Medicine* 2: 753-759). For example, genetic mutations in 59914 and 59921 can be identified in two dimensional arrays containing light-generated DNA probes as described in Cronin, M.T. et al. *supra*. Briefly, a first hybridization array of probes can be used to scan through long stretches of DNA in a sample and control to identify base changes between the sequences by making linear arrays of sequential overlapping probes. This step allows the identification of point mutations. This step is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the 59914 and 59921 gene and detect mutations by comparing the sequence of the sample 59914 and 59921 with the corresponding wild-type (control) sequence. Automated sequencing procedures can be utilized when performing the diagnostic assays ((1995) *Biotechniques* 19:448), including sequencing by mass spectrometry.

Other methods for detecting mutations in the 59914 and 59921 gene include methods in which protection from cleavage agents is used to detect mismatched bases in RNA/RNA or RNA/DNA heteroduplexes (Myers et al. (1985) *Science* 230:1242; Cotton et al. (1988) *Proc. Natl Acad Sci USA* 85:4397; Saleeba et al. (1992) *Methods Enzymol*. 217:286-295).

In still another embodiment, the mismatch cleavage reaction employs one or more proteins that recognize mismatched base pairs in double-stranded DNA (so called "DNA mismatch repair" enzymes) in defined systems for detecting and mapping point mutations in 59914 and 59921 cDNAs obtained from samples of cells. For example, the mutY enzyme of *E. coli* cleaves A at G/A mismatches and the thymidine DNA glycosylase from HeLa cells cleaves T at G/T mismatches (Hsu et al. (1994) *Carcinogenesis* 15:1657-1662; U.S. Patent No. 5,459,039).

In other embodiments, alterations in electrophoretic mobility will be used to identify mutations in 59914 and 59921 genes. For example, single strand conformation polymorphism (SSCP) may be used to detect differences in electrophoretic mobility between mutant and wild type nucleic acids (Orita et al. (1989) Proc *Natl. Acad. Sci USA*: 86:2766, see also Cotton (1993) *Mutat. Res.* 285:125-144; and Hayashi (1992) Genet. *Anal. Tech. Appl.* 9:73-79). Single-stranded DNA fragments of sample and control 59914 and 59921 nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In a preferred embodiment, the subject method utilizes heteroduplex analysis to separate double stranded heteroduplex molecules on the basis of changes in electrophoretic mobility (Keen et al. (1991) *Trends Genet* 7:5).

In yet another embodiment, the movement of mutant or wild-type fragments in polyacrylamide gels containing a gradient of denaturant is assayed using denaturing gradient gel electrophoresis (DGGE) (Myers et al. (1985) *Nature* 313:495). When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA (Rosenbaum and Reissner (1987) *Biophys Chem* 265:12753).

Examples of other techniques for detecting point mutations include, but are not limited to, selective oligonucleotide hybridization, selective amplification, or selective primer extension (Saiki et al. (1986) *Nature* 324:163); Saiki et al. (1989) *Proc. Natl Acad. Sci USA* 86:6230).

Alternatively, allele specific amplification technology which depends on selective PCR amplification may be used in conjunction with the instant invention. Oligonucleotides used as primers for specific amplification may carry the mutation of interest in the center of the molecule (so that amplification depends on differential hybridization) (Gibbs et al. (1989) *Nucleic Acids Res.* 17:2437-2448) or at the extreme 3' end of one primer where, under appropriate conditions, mismatch can prevent, or reduce polymerase extension (Prossner (1993) *Tibtech* 11:238). In addition it may be desirable to introduce a novel restriction site in the region of the mutation to create cleavage-based detection (Gasparini et al. (1992) *Mol. Cell Probes* 6:1). It is anticipated that in certain embodiments amplification may also be performed using Taq ligase for amplification (Barany (1991) *Proc. Natl. Acad. Sci USA* 88:189). In such cases, ligation will occur only if there is a perfect match at the 3' end of the 5' sequence making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, *e.g*., in clinical settings to diagnose patients exhibiting symptoms or family history of a disease or illness involving 59914 and 59921 genes.

### Use of 59914 and 59921 Molecules as Surrogate Markers

The 59914 and 59921 molecules of the invention are also useful as markers of disorders or disease states, as markers for precursors of disease states, as markers for predisposition of disease states, as markers of drug activity, or as markers of the pharmacogenomic profile of a subject. Using the methods described herein, the presence, absence and/or quantity of the 59914 and 59921 molecules of the invention may be detected, and may be correlated with one or more biological states *in vivo.* For example, the 59914 and 59921 molecules of the invention may serve as surrogate markers for one or more disorders or disease states or for conditions leading up to disease states. As used herein, a "surrogate marker" is an objective biochemical marker which correlates with the absence or presence of a disease or disorder, or with the progression of a disease or disorder (e.g., with the presence or absence of a tumor). The presence or quantity of such markers is independent of the disease. Therefore, these markers may serve to indicate whether a particular course of treatment is effective in lessening a disease state or disorder. Surrogate markers are of particular use when the presence or extent of a disease state or disorder is difficult to assess through standard methodologies (e.g., early stage tumors), or when an assessment of disease progression is desired before a potentially dangerous clinical endpoint is reached (e.g., an assessment of cardiovascular disease may be made using cholesterol levels as a surrogate marker, and an analysis of HIV infection may be made using HIV RNA levels as a surrogate marker, well in advance of the undesirable clinical outcomes of myocardial infarction or fully-developed AIDS). Examples of the use of surrogate markers in the art include: Koomen et al. (2000) J. Mass. Spectrom. 35: 258-264; and James (1994) *AIDS Treatment News Archive* 209.

The 59914 and 59921 molecules of the invention are also useful as pharmacodynamic markers. As used herein, a "pharmacodynamic marker" is an objective biochemical marker which correlates specifically with drug effects. The presence or quantity of a pharmacodynamic marker is not related to the disease state or disorder for which the drug is being administered; therefore, the presence or quantity of the marker is indicative of the presence or activity of the drug in a subject. For example, a pharmacodynamic marker may be indicative of the concentration of the drug in a biological tissue, in that the marker is either expressed or transcribed or not expressed or transcribed in that tissue in relationship to the level of the drug. In this fashion, the distribution or uptake of the drug may be monitored by the pharmacodynamic marker. Similarly, the presence or quantity of the pharmacodynamic marker may be related to the presence or quantity of the metabolic product of a drug, such that the presence or quantity of the marker is indicative of the relative breakdown rate of the drug in vivo. Pharmacodynamic markers are of particular use in increasing the sensitivity of detection of drug effects, particularly when the drug is administered in low doses. Since even a small amount of a drug may be sufficient to activate multiple rounds of marker (e.g., a 59914 and 59921 marker) transcription or expression, the amplified marker may be in a quantity which is more readily detectable than the drug itself. Also, the marker may be more easily detected due to the nature of the marker itself; for example, using the methods described herein, anti-59914 and 59921 antibodies may be employed in an immune-based detection system for 59914 and 59921 proteins marker, or 59914 and 59921-specific radiolabeled probes may be used to detect 59914 and 59921 mRNA marker. Furthermore, the use of a pharmacodynamic marker may offer mechanism-based prediction of risk due to drug treatment beyond the range of possible direct observations. Examples of the use of pharmacodynamic markers in the art include: Matsuda et al. US 6,033,862; Hattis et al. (1991) Env. Health Perspect. 90: 229-238; Schentag (1999) *Am. J. Health-Syst. Pharm*. 56 Suppl. 3: S21-S24; and Nicolau (1999) *Am, J. Health-Syst. Pharm*. 56 Suppl. 3: S16-S20.

The 59914 and 59921 molecules of the invention are also useful as pharmacogenomic markers. As used herein, a "pharmacogenomic marker" is an objective biochemical marker which correlates with a specific clinical drug response or susceptibility in a subject (see, e.g., McLeod et al. (1999) *Eur. J. Cancer* 35:1650-1652). The presence or quantity of the pharmacogenomic marker is related to the predicted response of the subject to a specific drug or class of drugs prior to administration of the drug. By assessing the presence or quantity of one or more pharmacogenomic markers in a subject, a drug therapy which is most appropriate for the subject, or which is predicted to have a greater degree of success, may be selected. For example, based on the presence or quantity of RNA, or protein (e.g., 59914 and 59921 protein or RNA) for specific tumor markers in a subject, a drug or course of treatment may be selected that is optimized for the treatment of the specific tumor likely to be present in the subject. Similarly, the presence or absence of a specific sequence mutation in 59914 and 59921 DNA may correlate 59914 and 59921 drug response. The use of pharmacogenomic markers therefore permits the application of the most appropriate treatment for each subject without having to administer the therapy.

### 1.12 Pharmaceutical Compositions

The nucleic acid and polypeptides, fragments thereof, as well as anti-59914 and 59921 antibodies and small molecule modulators of 59914 and 59921 molecules (also referred to herein as "active compounds") of the invention can be incorporated into pharmaceutical compositions. Such compositions typically include the nucleic acid molecule, protein, or antibody and a pharmaceutically acceptable carrier. As used herein, a "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany NJ) or phosphate buffered saline (PBS). In all cases, the composition must be sterile and should be fluid to the extent that easy syringability exists. It should be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the active compound can be incorporated with excipients and used in the form of tablets, troches, or capsules, e.g., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation (Palo Alto CA) and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier.

Toxicity and therapeutic efficacy of such compounds can be determined by standard pharmaceutical procedures in cell cultures or experimental animals, *e.g*., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds which exhibit high therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

As defined herein, a therapeutically effective amount of protein or polypeptide (i.e., an effective dosage) ranges from about 0.001 to 30 mg/kg body weight, preferably about 0.01 to 25 mg/kg body weight, more preferably about 0.1 to 20 mg/kg body weight, and even more preferably about 1 to 10 mg/kg, 2 to 9 mg/kg, 3 to 8 mg/kg, 4 to 7 mg/kg, or 5 to 6 mg/kg body weight. The protein or polypeptide can be administered one time per week for between about 1 to 10 weeks, preferably between 2 to 8 weeks, more preferably between about 3 to 7 weeks, and even more preferably for about 4, 5, or 6 weeks. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a protein, polypeptide, or antibody can include a single treatment or, preferably, can include a series of treatments.

For antibodies, the preferred dosage is 0.1 mg/kg of body weight (generally 10 mg/kg to 20 mg/kg). If the antibody is to act in the brain, a dosage of 50 mg/kg to 100 mg/kg is usually appropriate. Generally, partially human antibodies and fully human antibodies have a longer half-life within the human body than other antibodies. Accordingly, lower dosages and less frequent administration is often possible. Modifications such as lipidation can be used to stabilize antibodies and to enhance uptake and tissue penetration (e.g., into the brain). A method for lipidation of antibodies is described by Cruikshank et al. ((1997) *J. Acquired Immune Deficiency Syndromes and Human Retrovirology* 14:193).

The present invention encompasses agents which modulate expression or activity. An agent may, for example, be a small molecule. For example, such small molecules include, but are not limited to, peptides, peptidomimetics (e.g., peptoids), amino acids, amino acid analogs, polynucleotides, polynucleotide analogs, nucleotides, nucleotide analogs, organic or inorganic compounds (i.e.,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

Exemplary doses include milligram or microgram amounts of the small molecule per kilogram of subject or sample weight (e.g., about 1 microgram per kilogram to about 500 milligrams per kilogram, about 100 micrograms per kilogram to about 5 milligrams per kilogram, or about 1 microgram per kilogram to about 50 micrograms per kilogram. It is furthermore understood that appropriate doses of a small molecule depend upon the potency of the small molecule with respect to the expression or activity to be modulated. When one or more of these small molecules is to be administered to an animal (e.g., a human) in order to modulate expression or activity of a polypeptide or nucleic acid of the invention, a physician, veterinarian, or researcher may, for example, prescribe a relatively low dose at first, subsequently increasing the dose until an appropriate response is obtained. In addition, it is understood that the specific dose level for any particular animal subject will depend upon a variety of factors including the activity of the specific compound employed, the age, body weight, general health, gender, and diet of the subject, the time of administration, the route of administration, the rate of excretion, any drug combination, and the degree of expression or activity to be modulated.

An antibody (or fragment thereof) may be conjugated to a therapeutic moiety such as a cytotoxin, a therapeutic agent or a radioactive agent (e.g., a radioactive metal ion). A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cisdichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

The conjugates of the invention can be used for modifying a given biological response, the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating a therapeutic moiety to an antibody are well known (see, e.g., Arnon et al., 1985, "Monoclonal Antibodies For Immunotargeting Of Drugs In Cancer Therapy", in Monoclonal Antibodies And Cancer Therapy, Reisfeld et al., Eds., Alan R. Liss, Inc. pp. 243-256; Hellstrom et al., 1987, "Antibodies For Drug Delivery", in Controlled Drug Delivery, 2nd ed., Robinson et al., Eds., Marcel Dekker, Inc., pp. 623-653; Thorpe, 1985, "Antibody Carriers Of Cytotoxic Agents In Cancer Therapy: A Review", in Monoclonal Antibodies '84: Biological And Clinical Applications, Pinchera et al., Eds., pp. 475-506; "Analysis, Results, And Future Prospective Of The Therapeutic Use Of Radiolabeled Antibody In Cancer Therapy", in Monoclonal Antibodies For Cancer Detection And Therapy, Baldwin et al., Eds., Academic Press, pp. 303-316, 1985; and Thorpe et al., 1982, Immunol. Rev., 62:119-158). Alternatively, an antibody can be conjugated to a second antibody to form an antibody heteroconjugate as described by Segal in U.S. Patent No. 4,676,980.

The nucleic acid molecules of the invention can be inserted into vectors and used as gene therapy vectors. Gene therapy vectors can be delivered to a subject by, for example, intravenous injection, local administration (see U.S. Patent 5,328,470) or by stereotactic injection (see e.g., Chen et al. (1994) *Proc. Natl. Acad. Sci. USA* 91:3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g., retroviral vectors, the pharmaceutical preparation can include one or more cells which produce the gene delivery system.

The pharmaceutical compositions can be included in a container, pack, or dispenser together with instructions for administration.

### 1.13 Methods of Treatment

The present invention provides for both prophylactic and therapeutic methods of treating a subject at risk of (or susceptible to) a disorder or having a disorder associated with aberrant or undesirable 59914 and 59921 expression or activity. With regard to both prophylactic and therapeutic methods of treatment, such treatments may be specifically tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and commercially available. More specifically, the term refers the study of how a patient's genes determine his or her response to a drug (e.g., a patient's "drug response phenotype", or "drug response genotype".) Thus, another aspect of the invention provides methods for tailoring an individual's prophylactic or therapeutic treatment with either the 59914 and 59921 molecules of the present invention or 59914 and 59921 modulators according to that individual's drug response genotype. Pharmacogenomics allows a clinician or physician to target prophylactic or therapeutic treatments to patients who will most benefit from the treatment and to identify patients who will experience toxic drug-related side effects.

"Treatment", as used herein, is defined as the application or administration of a therapeutic agent to a patient, or application or administration of a therapeutic agent to an isolated tissue or cell line from a patient, who has a disease, a symptom of disease or a predisposition toward a disease, with the purpose to cure, heal, alleviate, relieve, alter, remedy, ameliorate, palliate, improve or affect the disease, the symptoms of disease or the predisposition toward disease. A therapeutic agent includes, but is not limited to, small molecules, peptides, antibodies, ribozymes and antisense oligonucleotides.

In one aspect, the invention provides a method for preventing in a subject, a disease or condition associated with an aberrant or undesirable 59914 and 59921 expression or activity, by administering to the subject a 59914 and 59921 molecule or an agent which modulates 59914 and 59921 expression or at least one 59914 and 59921 activity. Subjects at risk for a disease which is caused or contributed to by aberrant or undesirable 59914 and 59921 expression or activity can be identified by, for example, any or a combination of diagnostic or prognostic assays as described herein. Administration of a prophylactic agent can occur prior to the manifestation of symptoms characteristic of the 59914 and 59921 aberrance, such that a disease or disorder is prevented or, alternatively, delayed in its progression. Depending on the type of 59914 and 59921 aberrance, for example, a 59914 and 59921 molecule (e.g., a 59914 and 59921 nucleic acid molecule or 59914 and 59921 proteins or polypeptide, or a fragment thereof, as described herein), or 59914 and 59921 agonist or 59914 and 59921 antagonist agent can be used for treating the subject. The appropriate agent can be determined based on screening assays described herein.

It is possible that some 59914 and 59921 disorders can be caused, at least in part, by an abnormal level of gene product, or by the presence of a gene product exhibiting abnormal activity. As such, the reduction in the level and/or activity of such gene products would bring about the amelioration of disorder symptoms.

As discussed, successful treatment of 59914 and 59921 disorders can be brought about by techniques that serve to inhibit the expression or activity of 59914 and 59921 target gene products. For example, compounds, e.g., an agent identified using an assays described above, that proves to exhibit negative modulatory activity, can be used in accordance with the invention to prevent and/or ameliorate symptoms of 59914 and 59921 disorders. Such molecules can include, but are not limited to peptides, phosphopeptides, small organic or inorganic molecules, or antibodies (including, for example, polyclonal, monoclonal, humanized, human, anti-idiotypic, chimeric or single chain antibodies, and Fab, F(ab')₂ and Fab expression library fragments, scFV molecules, and epitope-binding fragments thereof).

Further, antisense and ribozyme molecules that inhibit expression of the target gene can also be used in accordance with the invention to reduce the level of target gene expression, thus effectively reducing the level of target gene activity. Still further, triple helix molecules can be utilized in reducing the level of target gene activity. Antisense, ribozyme and triple helix molecules are discussed above.

It is possible that the use of antisense, ribozyme, and/or triple helix molecules to reduce or inhibit mutant gene expression can also reduce or inhibit the transcription (triple helix) and/or translation (antisense, ribozyme) of mRNA produced by normal target gene alleles, such that the concentration of normal target gene product present can be lower than is necessary for a normal phenotype. In such cases, nucleic acid molecules that encode and express target gene polypeptides exhibiting normal target gene activity can be introduced into cells via gene therapy method. Alternatively, in instances in that the target gene encodes an extracellular protein, it can be preferable to coadminister normal target gene protein into the cell or tissue in order to maintain the requisite level of cellular or tissue target gene activity.

Another method by which nucleic acid molecules may be utilized in treating or preventing a disease characterized by 59914 and 59921 expression is through the use of aptamer molecules specific for 59914 and 59921 protein. Aptamers are nucleic acid molecules having a tertiary structure which permits them to specifically bind to protein ligands (see, e.g., Osborne, et al. (1997) *Curr. Opin. Chem. Biol.* 1(1):5-9; and Patel, D.J. (1997) *Curr. Opin. Chem. Biol*. 1(1):32-46). Since nucleic acid molecules may in many cases be more conveniently introduced into target cells than therapeutic protein molecules may be, aptamers offer a method by which 59914 and 59921 protein activity may be specifically decreased without the introduction of drugs or other molecules which may have pluripotent effects.

Antibodies can be generated that are both specific for target gene product and that reduce target gene product activity. Such antibodies may, therefore, by administered in instances whereby negative modulatory techniques are appropriate for the treatment of 59914 and 59921 disorders. For a description of antibodies, see the Antibody section above.

In circumstances wherein injection of an animal or a human subject with 59914 and 59921 proteins or epitope for stimulating antibody production is harmful to the subject, it is possible to generate an immune response against 59914 and 59921 through the use of anti-idiotypic antibodies (see, for example, Herlyn, D. (1999) *Ann. Med.* 31(1):66-78; and Bhattacharya-Chatterjee, M., and Foon, K.A. (1998) *Cancer Treat. Res.* 94:51-68). If an anti-idiotypic antibody is introduced into a mammal or human subject, it should stimulate the production of anti-anti-idiotypic antibodies, which should be specific to the 59914 and 59921 protein. Vaccines directed to a disease characterized by 59914 and 59921 expression may also be generated in this fashion.

In instances where the target antigen is intracellular and whole antibodies are used, internalizing antibodies may be preferred. Lipofectin or liposomes can be used to deliver the antibody or a fragment of the Fab region that binds to the target antigen into cells. Where fragments of the antibody are used, the smallest inhibitory fragment that binds to the target antigen is preferred. For example, peptides having an amino acid sequence corresponding to the Fv region of the antibody can be used. Alternatively, single chain neutralizing antibodies that bind to intracellular target antigens can also be administered. Such single chain antibodies can be administered, for example, by expressing nucleotide sequences encoding single-chain antibodies within the target cell population (see e.g., Marasco et al. (1993) *Proc. Natl. Acad. Sci. USA* 90:7889-7893).

The identified compounds that inhibit target gene expression, synthesis and/or activity can be administered to a patient at therapeutically effective doses to prevent, treat or ameliorate 59914 and 59921 disorders. A therapeutically effective dose refers to that amount of the compound sufficient to result in amelioration of symptoms of the disorders.

Toxicity and therapeutic efficacy of such compounds can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Compounds that exhibit large therapeutic indices are preferred. While compounds that exhibit toxic side effects can be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED₅₀ with little or no toxicity. The dosage can vary within this range depending upon the dosage form employed and the route of administration utilized. For any compound used in the method of the invention, the therapeutically effective dose can be estimated initially from cell culture assays. A dose can be formulated in animal models to achieve a circulating plasma concentration range that includes the IC₅₀ (i.e., the concentration of the test compound that achieves a half-maximal inhibition of symptoms) as determined in cell culture. Such information can be used to more accurately determine useful doses in humans. Levels in plasma can be measured, for example, by high performance liquid chromatography.

Another measurement which can be used to determine the effective dose for an individual is to directly assay levels of "free" and "bound" compound in the serum of the test subject. Such assays may utilize antibody mimics and/or "biosensors" that have been created through molecular imprinting techniques. The compound which is able to modulate 59914 and 59921 activity is used as a template, or "imprinting molecule", to spatially organize polymerizable monomers prior to their polymerization with catalytic reagents. The subsequent removal of the imprinted molecule leaves a polymer matrix which contains a repeated "negative image" of the compound and is able to selectively rebind the molecule under biological assay conditions. A detailed review of this technique is found in Ansell, R. J. et al. (1996) *Current Opinion in Biotechnology* 7:89-94 and in Shea, K.J. (1994) *Trends in Polymer Science* 2:166-173. Such "imprinted" affinity matrixes are amenable to ligand-binding assays, whereby the immobilized monoclonal antibody component is replaced by an appropriately imprinted matrix. An example of the use of such matrices in this way can be seen in Vlatakis, G. et al., (1993) *Nature* 361:645-647. Through the use of isotope-labeling, the "free" concentration of compound which modulates the expression or activity of 59914 and 59921 can be readily monitored and used in calculations of IC₅₀.

Such "imprinted" affinity matrices can also be designed to include fluorescent groups whose photon-emitting properties measurably change upon local and selective binding of target compound. These changes can be readily assayed in real time using appropriate fiberoptic devices, in turn allowing the dose in a test subject to be quickly optimized based on its individual IC₅₀. A rudimentary example of such a "biosensor" is discussed in Kriz, D. et al. (1995) *Analytical Chemistry* 67:2142-2144.

Another aspect of the invention pertains to methods of modulating 59914 and 59921 expression or activity for therapeutic purposes. Accordingly, in an exemplary embodiment, the modulatory method of the invention involves contacting a cell with a 59914 and 59921 molecule (e.g., a 59914 and 59921 nucleic acid molecule or 59914 and 59921 protein or polypeptide, or a fragment thereof, as described herein) or an agent that modulates one or more of the activities of the 59914 and 59921 protein activity associated with the cell. An agent that modulates 59914 and 59921 protein activity can be an agent as described herein, such as a nucleic acid or a protein, a naturally-occurring target molecule of 59914 and 59921 proteins (*e.g*., a 59914 and 59921 substrate, ligand, or receptor), an anti-59914 and 59921 antibody, a 59914 and 59921 agonist or antagonist, a peptidomimetic of a 59914 and 59921 agonist or antagonist, or other small molecule.

In one embodiment, the agent stimulates one or more 59914 and 59921 activities. Examples of such stimulatory agents include active 59914 and 59921 proteins and nucleic acid molecules encoding 59914 and 59921 proteins or polypeptide, or a fragment thereof. In another embodiment, the agent inhibits one or more 59914 and 59921 activities. Examples of such inhibitory agents include antisense 59914 and 59921 nucleic acid molecules, anti-59914 and 59921 antibodies, and 59914 and 59921 inhibitors. These modulatory methods can be performed *in vitro* (e.g., by culturing the cell with the agent) or, alternatively, *in vivo* (e.g., by administering the agent to a subject), or *in situ*. As such, the present invention provides methods of treating an individual afflicted with a disease or disorder characterized by aberrant or unwanted expression or activity of 59914 and 59921 proteins or nucleic acid molecule. In one embodiment, the method involves administering an agent (e.g., an agent identified by a screening assay described herein), or combination of agents that modulates (e.g., upregulates or downregulates) 59914 and 59921 expression or activity. In another embodiment, the method involves administering 59914 and 59921 proteins or nucleic acid molecule as therapy to compensate for reduced, aberrant, or undesirable 59914 and 59921 expression or activity.

Stimulation of 59914 and 59921 expression or activity is desirable in situations in which 59914 and 59921 expression or activity is abnormally downregulated and/or in which increased 59914 and 59921 expression or activity is likely to have a beneficial effect. Likewise, inhibition of 59914 and 59921 expression or activity is desirable in situations in which 59914 and 59921 expression or activity is abnormally upregulated and/or in which decreased 59914 and 59921 expression or activity is likely to have a beneficial effect.

The 59914 and 59921 molecules can act as novel diagnostic targets and therapeutic agents for controlling one or more of cellular proliferative and/or differentiative disorders, hormonal disorders, immune and inflammatory disorders, neurological disorders, cardiovascular disorders, blood vessel disorders, and platelet disorders, as described above, as well as disorders associated with bone metabolism, viral diseases, and pain and metabolic disorders.

Aberrant expression and/or activity of 59914 and 59921 molecules may mediate disorders associated with bone metabolism. "Bone metabolism" refers to direct or indirect effects in the formation or degeneration of bone structures, e.g., bone formation, bone resorption, etc., which may ultimately affect the concentrations in serum of calcium and phosphate. This term also includes activities mediated by 59914 and 59921 molecules effects in bone cells, e.g. osteoclasts and osteoblasts, that may in turn result in bone formation and degeneration. For example, 59914 and 59921 molecules may support different activities of bone resorbing osteoclasts such as the stimulation of differentiation of monocytes and mononuclear phagocytes into osteoclasts. Accordingly, 59914 and 59921 molecules that modulate the production of bone cells can influence bone formation and degeneration, and thus may be used to treat bone disorders. Examples of such disorders include, but are not limited to, osteoporosis, osteodystrophy, osteomalacia, rickets, osteitis fibrosa cystica, renal osteodystrophy, osteosclerosis, anti-convulsant treatment, osteopenia, fibrogenesis-imperfecta ossium, secondary hyperparathyrodism, hypoparathyroidism, hyperparathyroidism, cirrhosis, obstructive jaundice, drug induced metabolism, medullary carcinoma, chronic renal disease, rickets, sarcoidosis, glucocorticoid antagonism, malabsorption syndrome, steatorrhea, tropical sprue, idiopathic hypercalcemia and milk fever.

Additionally, 59914 and 59921 molecules may play an important role in the etiology of certain viral diseases, including but not limited to, Hepatitis B, Hepatitis C and Herpes Simplex Virus (HSV). Modulators of 59914 and 59921activity can be used to control viral diseases. The modulators can be used in the treatment and/or diagnosis of viral infected tissue or virus-associated tissue fibrosis, especially liver and liver fibrosis. Also, 59914 and 59921 modulators can be used in the treatment and/or diagnosis of virus-associated carcinomas, especially hepatocellular cancers.

Additionally, 59914 and 59921 may play an important role in the regulation of metabolism or pain disorders. Diseases of metabolic imbalance include, but are not limited to, obesity, anorexia nervosa, bullemia, cachexia, lipid disorders, and diabetes. Examples of pain disorders include, but are not limited to, pain response elicited during various forms of tissue injury, e.g., inflammation, infection, and ischemia, usually referred to as hyperalgesia (described in, for example, Fields, H.L., (1987) *Pain,* New York:McGraw-Hill); pain associated with muscoloskeletal disorders, e.g., joint pain; tooth pain; headaches; pain associated with surgery pain related to irritable bowel syndrome; and chest pain.

### 1.14 Pharmacogenomics

The 59914 and 59921 molecules of the present invention, as well as agents, and modulators which have a stimulatory or inhibitory effect on a 59914 and 59921 activity (e.g., 59914 and 59921 gene expression) as identified by a screening assay described herein can be administered to individuals to treat (prophylactically or therapeutically) 59914 and 59921 associated disorders (e.g., cellular proliferative and/or differentiative disorders, hormonal disorders, immune and inflammatory disorders, neurological disorders, cardiovascular disorders, blood vessel disorders, and platelet disorders) associated with aberrant or undesirable 59914 and 59921 activity. In conjunction with such treatment, pharmacogenomics (i.e., the study of the relationship between an individual's genotype and that individual's response to a foreign compound or drug) may be considered. Differences in metabolism of therapeutics can lead to severe toxicity or therapeutic failure by altering the relation between dose and blood concentration of the pharmacologically active drug. Thus, a physician or clinician may consider applying knowledge obtained in relevant pharmacogenomics studies in determining whether to administer a 59914 and 59921 molecule or 59914 and 59921 modulator as well as tailoring the dosage and/or therapeutic regimen of treatment with a 59914 and 59921 molecule or 59914 and 59921 modulator.

Pharmacogenomics deals with clinically significant hereditary variations in the response to drugs due to altered drug disposition and abnormal action in affected persons. See, for example, Eichelbaum, M. et al. (1996) *Clin. Exp. Pharmacol. Physiol*. 23:983-985 and Linder, M.W. et al. (1997) *Clin. Chem.* 43:254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare genetic defects or as naturally-occurring polymorphisms. For example, glucose-6-phosphate dehydrogenase deficiency (G6PD) is a common inherited enzymopathy in which the main clinical complication is haemolysis after ingestion of oxidant drugs (anti-malarials, sulfonamides, analgesics, nitrofurans) and consumption of fava beans.

One pharmacogenomics approach to identifying genes that predict drug response, known as "a genome-wide association", relies primarily on a high-resolution map of the human genome consisting of already known gene-related markers (e.g., a "bi-allelic" gene marker map which consists of 60,000-100,000 polymorphic or variable sites on the human genome, each of which has two variants.) Such a high-resolution genetic map can be compared to a map of the genome of each of a statistically significant number of patients taking part in a Phase II/III drug trial to identify markers associated with a particular observed drug response or side effect. Alternatively, such a high resolution map can be generated from a combination of some ten-million known single nucleotide polymorphisms (SNPs) in the human genome. As used herein, a "SNP" is a common alteration that occurs in a single nucleotide base in a stretch of DNA. For example, a SNP may occur once per every 1000 bases of DNA. A SNP may be involved in a disease process, however, the vast majority may not be disease-associated. Given a genetic map based on the occurrence of such SNPs, individuals can be grouped into genetic categories depending on a particular pattern of SNPs in their individual genome. In such a manner, treatment regimens can be tailored to groups of genetically similar individuals, taking into account traits that may be common among such genetically similar individuals.

Alternatively, a method termed the "candidate gene approach", can be utilized to identify genes that predict drug response. According to this method, if a gene that encodes a drug's target is known (e.g., 59914 and 59921 proteins of the present invention), all common variants of that gene can be fairly easily identified in the population and it can be determined if having one version of the gene versus another is associated with a particular drug response.

Alternatively, a method termed the "gene expression profiling", can be utilized to identify genes that predict drug response. For example, the gene expression of an animal dosed with a drug (e.g., a 59914 and 59921 molecule or 59914 and 59921 modulator of the present invention) can give an indication whether gene pathways related to toxicity have been turned on.

Information generated from more than one of the above pharmacogenomics approaches can be used to determine appropriate dosage and treatment regimens for prophylactic or therapeutic treatment of an individual. This knowledge, when applied to dosing or drug selection, can avoid adverse reactions or therapeutic failure and thus enhance therapeutic or prophylactic efficiency when treating a subject with a 59914 and 59921 molecule or 59914 and 59921 modulator, such as a modulator identified by one of the exemplary screening assays described herein.

The present invention further provides methods for identifying new agents, or combinations, that are based on identifying agents that modulate the activity of one or more of the gene products encoded by one or more of the 59914 and 59921 genes of the present invention, wherein these products may be associated with resistance of the cells to a therapeutic agent. Specifically, the activity of the proteins encoded by the 59914 and 59921 genes of the present invention can be used as a basis for identifying agents for overcoming agent resistance. By blocking the activity of one or more of the resistance proteins, target cells, e.g., human cells, will become sensitive to treatment with an agent that the unmodified target cells were resistant to.

Monitoring the influence of agents (e.g., drugs) on the expression or activity of 59914 and 59921 proteins can be applied in clinical trials. For example, the effectiveness of an agent determined by a screening assay as described herein to increase 59914 and 59921 gene expression or protein levels, or upregulate 59914 and 59921 activity, can be monitored in clinical trials of subjects exhibiting decreased 59914 and 59921 gene expression or protein levels, or downregulated 59914 and 59921 activity. Alternatively, the effectiveness of an agent determined by a screening assay to decrease 59914 and 59921 gene expression or protein levels, or downregulate 59914 and 59921 activity, can be monitored in clinical trials of subjects exhibiting increased 59914 and 59921 gene expression or protein levels, or upregulated 59914 and 59921 activity. In such clinical trials, the expression or activity of 59914 and 59921 genes, and preferably, other genes that have been implicated in, for example, a 59914 and 59921-associated disorder can be used as a "read out" or markers of the phenotype of a particular cell.

### 1.15 Other Embodiments

In another aspect, the invention features a method of analyzing a plurality of capture probes. The method is useful, e.g., to analyze gene expression. The method includes: providing a two dimensional array having a plurality of addresses, each address of the plurality being positionally distinguishable from each other address of the plurality, and each address of the plurality having a unique capture probe, e.g., a nucleic acid or peptide sequence, wherein the capture probes are from a cell or subject which expresses 59914 and 59921 or from a cell or subject in which a 59914 and 59921 mediated response has been elicited; contacting the array with a 59914 and 59921 nucleic acid (preferably purified), a 59914 and 59921 polypeptide (preferably purified), or an anti-59914 and 59921 antibody, and thereby evaluating the plurality of capture probes. Binding, e.g., in the case of a nucleic acid, hybridization with a capture probe at an address of the plurality, is detected, e.g., by a signal generated from a label attached to the 59914 and 59921 nucleic acid, polypeptide, or antibody.

The capture probes can be a set of nucleic acids from a selected sample, e.g., a sample of nucleic acids derived from a control or non-stimulated tissue or cell.

The method can include contacting the 59914 and 59921 nucleic acid, polypeptide, or antibody with a first array having a plurality of capture probes and a second array having a different plurality of capture probes. The results of each hybridization can be compared, e.g., to analyze differences in expression between a first and second sample. The first plurality of capture probes can be from a control sample, e.g., a wild type, normal, or non-diseased, non-stimulated, sample, e.g., a biological fluid, tissue, or cell sample. The second plurality of capture probes can be from an experimental sample, e.g., a mutant type, at risk, disease-state or disorder-state, or stimulated, sample, e.g., a biological fluid, tissue, or cell sample.

The plurality of capture probes can be a plurality of nucleic acid probes each of which specifically hybridizes, with an allele of 59914 and 59921. Such methods can be used to diagnose a subject, e.g., to evaluate risk for a disease or disorder, to evaluate suitability of a selected treatment for a subject, to evaluate whether a subject has a disease or disorder.

The method can be used to detect SNPs, as described above.

In another aspect, the invention features, a method of analyzing 59914 and 59921, e.g., analyzing structure, function, or relatedness to other nucleic acid or amino acid sequences. The method includes: providing a 59914 and 59921 nucleic acid or amino acid sequence; comparing the 59914 and 59921 sequence with one or more preferably a plurality of sequences from a collection of sequences, e.g., a nucleic acid or protein sequence database; to thereby analyze 59914 and 59921.

The method can include evaluating the sequence identity between a 59914 and 59921 sequence and a database sequence. The method can be performed by accessing the database at a second site, e.g., over the internet. Preferred databases include GenBank™ and SwissProt.

In another aspect, the invention features, a set of oligonucleotides, useful, e.g., for identifying SNP's, or identifying specific alleles of 59914 and 59921. The set includes a plurality of oligonucleotides, each of which has a different nucleotide at an interrogation position, e.g., an SNP or the site of a mutation. In a preferred embodiment, the oligonucleotides of the plurality identical in sequence with one another (except for differences in length). The oligonucleotides can be provided with differential labels, such that an oligonucleotides which hybridizes to one allele provides a signal that is distinguishable from an oligonucleotides which hybridizes to a second allele.

The sequence of a 59914 and 59921 molecules is provided in a variety of mediums to facilitate use thereof. A sequence can be provided as a manufacture, other than an isolated nucleic acid or amino acid molecule, which contains a 59914 and 59921 molecule. Such a manufacture can provide a nucleotide or amino acid sequence, e.g., an open reading frame, in a form which allows examination of the manufacture using means not directly applicable to examining the nucleotide or amino acid sequences, or a subset thereof, as they exists in nature or in purified form.

A 59914 and 59921 nucleotide or amino acid sequence can be recorded on computer readable media. As used herein, "computer readable media" refers to any medium that can be read and accessed directly by a computer. Such media include, but are not limited to: magnetic storage media, such as floppy discs, hard disc storage medium, and magnetic tape; optical storage media such as compact disc and CD-ROM; electrical storage media such as RAM, ROM, EPROM, EEPROM, and the like; and general hard disks and hybrids of these categories such as magnetic/optical storage media. The medium is adapted or configured for having thereon 59914 and 59921 sequence information of the present invention.

As used herein, the term "electronic apparatus" is intended to include any suitable computing or processing apparatus of other device configured or adapted for storing data or information. Examples of electronic apparatus suitable for use with the present invention include stand-alone computing apparatus; networks, including a local area network (LAN), a wide area network (WAN) Internet, Intranet, and Extranet; electronic appliances such as personal digital assistants (PDAs), cellular phones, pagers, and the like; and local and distributed processing systems.

As used herein, "recorded" refers to a process for storing or encoding information on the electronic apparatus readable medium. Those skilled in the art can readily adopt any of the presently known methods for recording information on known media to generate manufactures comprising the 59914 and 59921 sequence information.

A variety of data storage structures are available to a skilled artisan for creating a computer readable medium having recorded thereon a 59914 and 59921 nucleotide or amino acid sequence of the present invention. The choice of the data storage structure will generally be based on the means chosen to access the stored information. In addition, a variety of data processor programs and formats can be used to store the nucleotide sequence information of the present invention on computer readable medium. The sequence information can be represented in a word processing text file, formatted in commercially-available software such as WordPerfect and Microsoft Word, or represented in the form of an ASCII file, stored in a database application, such as DB2, Sybase, Oracle, or the like. The skilled artisan can readily adapt any number of data processor structuring formats (*e.g.*, text file or database) in order to obtain computer readable medium having recorded thereon the nucleotide sequence information of the present invention.

By providing the 59914 and 59921 nucleotide or amino acid sequences of the invention in computer readable form, the skilled artisan can routinely access the sequence information for a variety of purposes. For example, one skilled in the art can use the nucleotide or amino acid sequences of the invention in computer readable form to compare a target sequence or target structural motif with the sequence information stored within the data storage means. A search is used to identify fragments or regions of the sequences of the invention which match a particular target sequence or target motif.

The present invention therefore provides a medium for holding instructions for performing a method for determining whether a subject has a 59914 and 59921-associated disease or disorder or a pre-disposition to a 59914 and 59921-associated disease or disorder, wherein the method comprises the steps of determining 59914 and 59921 sequence information associated with the subject and based on the 59914 and 59921 sequence information, determining whether the subject has a 59914 and 59921-associated disease or disorder and/or recommending a particular treatment for the disease, disorder, or pre-disease condition.

The present invention further provides in an electronic system and/or in a network, a method for determining whether a subject has a 59914 and 59921-associated disease or disorder or a pre-disposition to a disease associated with 59914 and 59921, wherein the method comprises the steps of determining 59914 and 59921 sequence information associated with the subject, and based on the 59914 and 59921 sequence information, determining whether the subject has a 59914 and 59921-associated disease or disorder or a pre-disposition to a 59914 and 59921-associated disease or disorder, and/or recommending a particular treatment for the disease, disorder, or pre-disease condition. The method may further comprise the step of receiving phenotypic information associated with the subject and/or acquiring from a network phenotypic information associated with the subject.

The present invention also provides in a network, a method for determining whether a subject has a 59914 and 59921-associated disease or disorder or a pre-disposition to a 59914 and 59921-associated disease or disorder, said method comprising the steps of receiving 59914 and 59921 sequence information from the subject and/or information related thereto, receiving phenotypic information associated with the subject, acquiring information from the network corresponding to 59914 and 59921 and/or corresponding to a 59914 and 59921-associated disease or disorder, and based on one or more of the phenotypic information, the 59914 and 59921 information (e.g., sequence information and/or information related thereto), and the acquired information, determining whether the subject has a 59914 and 59921-associated disease or disorder or a pre-disposition to a 59914 and 59921-associated disease or disorder. The method may further comprise the step of recommending a particular treatment for the disease, disorder, or pre-disease condition.

The present invention also provides a business method for determining whether a subject has a 59914 and 59921-associated disease or disorder or a pre-disposition to a 59914 and 59921-associated disease or disorder, said method comprising the steps of receiving information related to 59914 and 59921 (e.g., sequence information and/or information related thereto), receiving phenotypic information associated with the subject, acquiring information from the network related to 59914 and 59921 and/or related to a 59914 and 59921-associated disease or disorder, and based on one or more of the phenotypic information, the 59914 and 59921 information, and the acquired information, determining whether the subject has a 59914 and 59921-associated disease or disorder or a pre-disposition to a 59914 and 59921-associated disease or disorder. The method may further comprise the step of recommending a particular treatment for the disease, disorder, or pre-disease condition.

The invention also includes an array comprising a 59914 and 59921 sequence of the present invention. The array can be used to assay expression of one or more genes in the array. In one embodiment, the array can be used to assay gene expression in a tissue to ascertain tissue specificity of genes in the array. In this manner, up to about 7600 genes can be simultaneously assayed for expression, one of which can be 59914 and 59921. This allows a profile to be developed showing a battery of genes specifically expressed in one or more tissues.

In addition to such qualitative information, the invention allows the quantitation of gene expression. Thus, not only tissue specificity, but also the level of expression of a battery of genes in the tissue if ascertainable. Thus, genes can be grouped on the basis of their tissue expression *per se* and level of expression in that tissue. This is useful, for example, in ascertaining the relationship of gene expression in that tissue. Thus, one tissue can be perturbed and the effect on gene expression in a second tissue can be determined. In this context, the effect of one cell type on another cell type in response to a biological stimulus can be determined. In this context, the effect of one cell type on another cell type in response to a biological stimulus can be determined. Such a determination is useful, for example, to know the effect of cell-cell interaction at the level of gene expression. If an agent is administered therapeutically to treat one cell type but has an undesirable effect on another cell type, the invention provides an assay to determine the molecular basis of the undesirable effect and thus provides the opportunity to coadminister a counteracting agent or otherwise treat the undesired effect. Similarly, even within a single cell type, undesirable biological effects can be determined at the molecular level. Thus, the effects of an agent on expression of other than the target gene can be ascertained and counteracted.

In another embodiment, the array can be used to monitor the time course of expression of one or more genes in the array. This can occur in various biological contexts, as disclosed herein, for example development of a 59914 and 59921-associated disease or disorder, progression of 59914 and 59921-associated disease or disorder, and processes, such a cellular transformation associated with the 59914 and 59921-associated disease or disorder.

The array is also useful for ascertaining the effect of the expression of a gene on the expression of other genes in the same cell or in different cells *(e.g*., ascertaining the effect of 59914 and 59921 expression on the expression of other genes). This provides, for example, for a selection of alternate molecular targets for therapeutic intervention if the ultimate or downstream target cannot be regulated.

The array is also useful for ascertaining differential expression patterns of one or more genes in normal and abnormal cells. This provides a battery of genes (*e.g.*, including 59914 and 59921) that could serve as a molecular target for diagnosis or therapeutic intervention.

As used herein, a "target sequence" can be any DNA or amino acid sequence of six or more nucleotides or two or more amino acids. A skilled artisan can readily recognize that the longer a target sequence is, the less likely a target sequence will be present as a random occurrence in the database. Typical sequence lengths of a target sequence are from about 10 to 100 amino acids or from about 30 to 300 nucleotide residues. However, it is well recognized that commercially important fragments, such as sequence fragments involved in gene expression and protein processing, may be of shorter length.

Computer software is publicly available which allows a skilled artisan to access sequence information provided in a computer readable medium for analysis and comparison to other sequences. A variety of known algorithms are disclosed publicly and a variety of commercially available software for conducting search means are and can be used in the computer-based systems of the present invention. Examples of such software include, but are not limited to, MacPattern (EMBL), BLASTN and BLASTX (NCBI).

Thus, the invention features a method of making a computer readable record of a sequence of a 59914 and 59921 sequence which includes recording the sequence on a computer readable matrix. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

In another aspect, the invention features, a method of analyzing a sequence. The method includes: providing a 59914 and 59921 sequence, or record, in computer readable form; comparing a second sequence to the 59914 and 59921 sequence; thereby analyzing a sequence. Comparison can include comparing to sequences for sequence identity or determining if one sequence is included within the other, e.g., determining if the 59914 and 59921 sequence includes a sequence being compared. In a preferred embodiment the 59914 and 59921 or second sequence is stored on a first computer, e.g., at a first site and the comparison is performed, read, or recorded on a second computer, e.g., at a second site. E.g., the 59914 and 59921 or second sequence can be stored in a public or proprietary database in one computer, and the results of the comparison performed, read, or recorded on a second computer. In a preferred embodiment the record includes one or more of the following: identification of an ORF; identification of a domain, region, or site; identification of the start of transcription; identification of the transcription terminator; the full length amino acid sequence of the protein, or a mature form thereof; the 5' end of the translated region.

This invention is further illustrated by the following exemplification, which should not be construed as limiting.

### EXEMPLIFICATION

### Gene Expression Analysis

Total RNA was prepared from various human tissues by a single step extraction method using RNA STAT-60 according to the manufacturer's instructions (TelTest, Inc). Each RNA preparation was treated with DNase I (Ambion) at 37°C for 1 hour. DNAse I treatment was determined to be complete if the sample required at least 38 PCR amplification cycles to reach a threshold level of fluorescence using β-2 microglobulin as an internal amplicon reference. The integrity of the RNA samples following DNase I treatment was confirmed by agarose gel electrophoresis and ethidium bromide staining. After phenol extraction cDNA was prepared from the sample using the SUPERSCRIPT™ Choice System following the manufacturer's instructions (GibcoBRL). A negative control of RNA without reverse transcriptase was mock reverse transcribed for each RNA sample.

Human 59914 and 59921 expression was measured by TaqMan® quantitative PCR (Perkin Elmer Applied Biosystems) in cDNA prepared from a variety of normal and diseased (e.g., cancerous) human tissues or cell lines.

Probes were designed by PrimerExpress software (PE Biosystems) based on the sequence of the human 59914 and 59921 genes. Each human 59914 and 59921 gene probe was labeled using FAM (6-carboxyfluorescein), and the β2-microglobulin reference probe was labeled with a different fluorescent dye, VIC. The differential labeling of the target gene and internal reference gene thus enabled measurement in same well. Forward and reverse primers and the probes for both β2-microglobulin and target gene were added to the TaqMan® Universal PCR Master Mix (PE Applied Biosystems). Although the final concentration of primer and probe could vary, each was internally consistent within a given experiment. A typical experiment contained 200nM of forward and reverse primers plus 100nM probe for β-2 microglobulin and 600 nM forward and reverse primers plus 200 nM probe for the target gene. TaqMan matrix experiments were carried out on an ABI PRISM 7700 Sequence Detection System (PE Applied Biosystems). The thermal cycler conditions were as follows: hold for 2 min at 50°C and 10 min at 95°C, followed by two-step PCR for 40 cycles of 95°C for 15 sec followed by 60°C for 1 min.

The following method was used to quantitatively calculate human 59914 and 59921 gene expression in the various tissues relative to β-2 microglobulin expression in the same tissue. The threshold cycle (Ct) value is defined as the cycle at which a statistically significant increase in fluorescence is detected. A lower Ct value is indicative of a higher mRNA concentration. The Ct value of the human 59914 and 59921 genes is normalized by subtracting the Ct value of the β-2 microglobulin gene to obtain a _{Δ}Ct value using the following formula: _{Δ}Ct=Ctₕᵤₘₐₙ _{59914 and 59921} - Ct _{β-2 microglobulin}. Expression is then calibrated against a cDNA sample showing a comparatively low level of expression of the human 59914 and 59921 gene. The _{Δ}Ct value for the calibrator sample is then subtracted from _{Δ}Ct for each tissue sample according to the following formula: _{ΔΔ}Ct=_{Δ}Ct-ₛₐₘₚₗₑ - _{Δ}Ct-_{calibrator}. Relative expression is then calculated using the arithmetic formula given by 2^{-ΔΔCt}. Expression of the target human 59914 and 59921 genes in each of the tissues tested is then graphically represented as discussed in more detail below.

The results indicate significant 59914 expression in normal brain cortex; moderate 59914 expression in human umbilical vein endothelial cells (HUVEC), prostate tumor and lung tumor; low levels of 59914 expression in colon tumor, kidney, and hypothalamus; significant 59921 expression in kidney, pancreas, and colon tumor; and low to moderate 59921 levels of expression in spinal cord, hypothalamus, nerve, dorsal root ganglia, prostate tumor, lung tumor, salivary glands, and liver fibrosis

The contents of all references, patents and published patent applications cited throughout this application are incorporated herein by reference.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. An isolated nucleic acid molecule selected from the group consisting of:
a) a nucleic acid molecule comprising a nucleotide sequence which is at least 80% identical to the nucleotide sequence of SEQ ID NO:1, SEQ ID NO:3, SEQ ID NO:4, or SEQ ID NO:6;
b) a nucleic acid molecule comprising a fragment of at least 600 nucleotides of the nucleotide sequence of SEQ ID NO:1 or SEQ ID NO:3;
c) a nucleic acid molecule comprising a fragment of at least 1660 nucleotides of the nucleotide sequence of SEQ ID NO:4 or SEQ ID NO:6;
d) a nucleic acid molecule which encodes a polypeptide comprising the amino acid sequence of SEQ ID NO:2 or SEQ ID NO:5;
e) a nucleic acid molecule which encodes a fragment of a polypeptide comprising the amino acid sequence of SEQ ID NO:2, wherein the fragment comprises at least 100 contiguous amino acids of SEQ ID NO:2
f) a nucleic acid molecule which encodes a fragment of a polypeptide comprising the amino acid sequence of SEQ ID NO:5, wherein the fragment comprises at least 300 contiguous amino acids of SEQ ID NO:5; and
g) a nucleic acid molecule which encodes a naturally occurring allelic variant of a polypeptide comprising the amino acid sequence of SEQ ID NO:2 or SEQ ID NO:5, wherein the nucleic acid molecule hybridizes to a nucleic acid molecule comprising SEQ ID NO:1, 3, 4, 6, or a complement thereof, under stringent conditions.

2. The isolated nucleic acid molecule of claim 1, further comprising a fragment of at least 1200 nucleotides of the nucleotide sequence of SEQ ID NO:1 or SEQ ID NO:3.

3. The isolated nucleic acid molecule of claim 1, further comprising a fragment of at least 2000 nucleotides of the nucleotide sequence of SEQ ID NO:1 or SEQ ID NO:3.

4. The isolated nucleic acid molecule of claim 1, further comprising a fragment of at least 1900 nucleotides of the nucleotide sequence of SEQ ID NO:4 or SEQ ID NO:6.

5. The isolated nucleic acid molecule of claim 1, which encodes a fragment of a polypeptide comprising the amino acid sequence of SEQ ID NO:2, wherein the fragment comprises at least 300 contiguous amino acids of SEQ ID NO:2.

6. The isolated nucleic acid molecule of claim 1, which encodes a fragment of a polypeptide comprising the amino acid sequence of SEQ ID NO:5, wherein the fragment comprises at least 400 contiguous amino acids of SEQ ID NO:5.

7. The isolated nucleic acid molecule of claim 1, which is selected from the group consisting of:
a. a nucleic acid comprising the nucleotide sequence of SEQ ID NO:1 or SEQ ID NO:3; and
b. a nucleic acid molecule which encodes a polypeptide comprising the amino acid sequence of SEQ ID NO:2 or SEQ ID NO:5.

8. The nucleic acid molecule of any of claims 1 to 7, further comprising vector nucleic acid sequences.

9. The nucleic acid molecule any of claims 1 to 7, further comprising nucleic acid sequences encoding a heterologous polypeptide.

10. A host cell which contains the nucleic acid molecule of any of claims 1 to 9.

11. The host cell of claim 10 which is a mammalian host cell.

12. A non-human mammalian host cell containing the nucleic acid molecule of any of claims 1 to 9.

13. An isolated polypeptide selected from the group consisting of:
a) a polypeptide which is encoded by a nucleic acid molecule comprising a nucleotide sequence which is at least 80% identical to a nucleic acid comprising the nucleotide sequence of SEQ ID NO:1, SEQ ID NO:3, SEQ ID NO:4, SEQ ID NO:6, or a complement thereof;
b) a naturally occurring allelic variant of a polypeptide comprising the amino acid sequence of SEQ ID NO:2 or SEQ ID NO:5, wherein the polypeptide is encoded by a nucleic acid molecule which hybridizes to a nucleic acid molecule comprising SEQ ID NO:1, SEQ ID NO:3, SEQ ID NO:4, or SEQ ID NO:6;
c) a fragment of a polypeptide comprising the amino acid sequence of SEQ ID NO:2, wherein the fragment comprises at least 100 contiguous amino acids of SEQ ID NO:2; and
d) a fragment of a polypeptide comprising the amino acid sequence of SEQ ID NO:5, wherein the fragment comprises at least 300 contiguous amino acids of SEQ ID NO:5.

14. The isolated polypeptide of claim 13, comprising a fragment which comprises at least 200 contiguous amino acids of SEQ ID NO:2.

15. The isolated polypeptide of claim 13, comprising a fragment which comprises at least 300 contiguous amino acids of SEQ ID NO:2.

16. The isolated polypeptide of claim 13, comprising a fragment which comprises at least 400 contiguous amino acids of SEQ ID NO:5.

17. The isolated polypeptide of claim 13, comprising a fragment which comprises at least 500 contiguous amino acids of SEQ ID NO:5.

18. The isolated polypeptide of claim 13, comprising a fragment which is at least 90% homologous to the amino acid sequence of SEQ ID NO:2 or SEQ ID NO:5.

19. The isolated polypeptide of claim 13, comprising a fragment which is at least 95% homologous to the amino acid sequence of SEQ ID NO:2 or SEQ ID NO:5.

20. The isolated polypeptide of claim 13, comprising the amino acid sequence of SEQ ID NO:2 or SEQ ID NO:5.

21. The polypeptide of any of claims 13 to 20 further comprising heterologous amino acid sequences.

22. An antibody which selectively binds to a polypeptide of any of claims 13 to 20.

23. The antibody of claim 22, which is a monoclonal antibody.

24. The antibody of claim 23, comprising an immunologically active portion selected from the group consisting of:
a. an scFV fragment;
b. a dcFV fragment;
c. an Fab fragment; and
d. an F(ab')₂ fragment.

25. The antibody of claim 22 or 23, wherein the antibody is selected from the group consisting of:
a. a chimeric antibody;
b. a humanized antibody;
c. a human antibody;
d. a non-human antibody; and
e. a single chain antibody.

26. A method for producing a polypeptide selected from the group consisting of:
a) a polypeptide comprising the amino acid sequence of SEQ ID NO:2 or SEQ ID NO:5;
b) a polypeptide comprising a fragment of the amino acid sequence of SEQ ID NO:2, wherein the fragment comprises at least 100 contiguous amino acids of SEQ ID NO:2;
c) a polypeptide comprising a fragment of the amino acid sequence of SEQ ID NO:5, wherein the fragment comprises at least 300 contiguous amino acids of SEQ ID NO:5; and
d) a naturally occurring allelic variant of a polypeptide comprising the amino acid sequence of SEQ ID NO:2 or SEQ ID NO:5, wherein the polypeptide is encoded by a nucleic acid molecule which hybridizes to a nucleic acid molecule comprising SEQ ID NO:1, SEQ ID NO:3, SEQ ID NO:4, SEQ ID NO:6, or a complement thereof under stringent conditions;
comprising culturing the host cell of any of claims 10 to 12 under conditions in which the nucleic acid molecule is expressed.

27. A method for detecting the presence of a polypeptide of any of claims 13 to 20 in a sample, comprising:
contacting the sample with a compound which selectively binds to a polypeptide of any of claims 13 to 20, as the case may be; and
determining whether the compound binds to the polypeptide in the sample.

28. The method of claim 27, wherein the compound which binds to the polypeptide is an antibody.

29. A kit comprising a compound which selectively binds to a polypeptide of any of claims 13 to 21 and instructions for use.

30. A method for detecting the presence of a nucleic acid molecule of any of claims 1 to 7 in a sample, comprising the steps of:
contacting the sample with a nucleic acid probe or primer which selectively hybridizes to the nucleic acid molecule; and
determining whether the nucleic acid probe or primer binds to a nucleic acid molecule in the sample.

31. The method of claim 30, wherein the sample comprises mRNA molecules and is contacted with a nucleic acid probe.

32. A kit comprising a compound which selectively hybridizes to a nucleic acid molecule of any of claims 1 to 7 and instructions for use.

33. A method for identifying a compound which binds to a polypeptide of any of claims 13 to 20 comprising the steps of:
contacting a polypeptide, or a cell expressing a polypeptide of any of claims 13 to 20, as the case may be, with a test compound; and
determining whether the polypeptide binds to the test compound.

34. The method of claim 33, wherein the binding of the test compound to the polypeptide is detected by a method selected from the group consisting of:
a) detection of binding by direct detecting of test compound/polypeptide binding;
b) detection of binding using a competition binding assay; and
c) detection of binding using an assay for 59914 and 59921-mediated signal transduction.

35. A method for modulating the activity of a polypeptide of any of claims 13 to 20 comprising contacting a polypeptide or a cell expressing a polypeptide of any of claims 13 to 20, as the case may be, with a compound which binds to the polypeptide in a sufficient concentration to modulate the activity of the polypeptide.

36. A method for identifying a compound which modulates the activity of a polypeptide of any of claims 13 to 20, comprising:
contacting a polypeptide of any of claim 13 to 20, as the case may be, with a test compound; and
determining the effect of the test compound on the activity of the polypeptide to thereby identify a compound which modulates the activity of the polypeptide.
